# EUROPEAN PATENT APPLICATION

(11) **EP 2 743 346 A1**
(43) Date of publication of application: **18.06.2014**
(21) Application number: 12196601.4
(22) Date of filing: 11.12.2012
(51) Int. Cl.: C12N 9/10, G06F 19/16

(54) **Crystal structure of blood coagulation factor XIIIa**

(71) Applicant: Zedira GmbH, 64293 Darmstadt (DE)
(72) Inventor: Hils, Martin Dr., 64295 Darmstadt (DE); Pasternack, Ralf Dr., 64347 Griesheim (DE); Büchold, Christian Dr., 61118 Bad Vilbel (DE); Weber, Johannes, 64295 Darmstadt (DE); Heine, Andreas Dr., 35091 Cölbe (DE); Klebe, Gerhard Prof. Dr., 35039 Marburg (DE); Stieler, Martin, 35085 Ebsdorfergrund (DE)
(74) Representative: Arth, Hans-Lothar

(57) **Abstract**

The present invention relates to crystal forms of blood coagulation factor XIIIa, crystal structure information obtained from them, methods of preparing such crystal forms, their use for the identification and / or design of inhibitors of blood coagulation factor XIIIa activity and methods for identifying, optimizing and designing compounds which should have the ability to interact with or inhibit blood coagulation factor XIIIa.

## Description

The present invention relates to crystal forms of blood coagulation factor Xllla, crystal structure information obtained from them, methods of preparing such crystal forms, their use for the identification and / or design of inhibitors of blood coagulation factor Xllla activity and methods for identifying, optimizing and designing compounds which should have the ability to interact with or inhibit blood coagulation factor Xllla.

### Background of the invention

Thrombotic events are a major cause of morbidity and mortality. Accordingly, remarkable efforts have been undertaken to develop drugs targeting coagulation factors or platelet activation to prevent or to treat thromboembolic events. The new direct acting oral anticoagulants address discrete, central proteases in the clotting cascade like factor IIa (thrombin) or factor Xa providing new therapeutic options.

In contrast, coagulation factor XIII (EC 2.3.2.13), also called plasma transglutaminase or fibrin stabilizing factor (FSF), has attracted only minor interest until yet. The human transglutaminase family consists of eight members catalyzing the unique formation of "cross-links" or isopeptide bonds between distinct substrate proteins. Since coagulation factor XIII is the major factor influencing clot maturation and accretion the enzyme is considered a suitable target to potentially achieve a safer and more efficient thrombolysis at even lower dosage of clot dissolving agents and also even for thrombus prevention.

Specific inhibitors may be of benefit for patients with a high risk of thrombotic events or to prevent atherosclerosis. Such a therapeutic approach would obviously not interfere with the up-stream factors like thrombin or with the platelet activation and activity.

Plasma coagulation factor XIII (pFXIII), is a heterotetrameric proenzyme consisting of two A-subunits providing the transglutaminase activity and two carrier/regulatory B-subunits. Factor XIII derived from platelets or megakaryocytes, also called cellular factor XIII (cFXIII), consists only of the homodimeric A-subunits. Recombinant cellular FXIII A-subunit was the first member of the transglutaminase family which has been crystallized as inactive proenzyme and its structure has been subsequently solved being a dimer.

Even if the structure of inactive coagulation factor XIII was the first member of the transglutaminase family solved by x-ray crystallography lots of questions remained. The active site is buried and the access to the catalytic site is obscured by the activation peptide. All experimental attempts failed to illuminate the active conformation so far. Neither proteolytic activation by thrombin nor the addition of calcium led to significant conformational changes being expected essential for catalysis [Weiss et al., FEBS Letters (1998), 423, 291-296; Fox et al. J. Biol. Chem (1999), 274(8), 4917-4923].

FXllla describes any catalytically active form of FXIII, e.g. activated by high calcium-concentrations or by proteolytical cleavage of the propeptide.

Very few inhibitors of factor XIIIa have been described so far. For example, a 66 amino acid peptide derived from the salivary gland of the giant Amazon leech Haementeria ghilianii is reported [Finney et al. Biochem. J. 1997, 324, pp. 797-805]. The peptide was named Tridegin, according to the Welsh word "triarddeg", meaning thirteen. Further, the pharmaceutical company Merck Sharp and Dohme developed a set of small molecule thioimidazole blockers targeting Factor XIII [Freund et al., Biochemistry (1994), 33, 10109-10119]. However in order to successfully develop a drug which inhibits Factor XIIIa in a selective manner (i.e. addressing the other transglutaminases to a significantly lesser extent), an *in silico* screening method based on the crystal structure is desired.

Thus, it is the objective of the present invention to provide crystal strucure coordinates of blood coagulation factor XIIIa, methods to obtain the crystal form of blood coagulation factor XIIIa as well as methods for designing, identifying and optimizing compounds as inhibitors of blood coagulation factor XIIIa based on the crystal strucure and crystal strucure coordinates.

The objective of the present invention is solved by the teachings of the independent claims. Further advantageous features, aspects and details of the invention are evident from the dependent claims, the description, the figures, and the examples of the present application.

### Brief description of the invention

The present invention provides methods for identifying inhibitors of factor XIIIa as well as methods for preparing crystal forms of blood coagulation factor XIIIa and their crystal structure information. The present invention relates also to the first x-ray crystal structure of FXllla in the active conformation. This is achived by inhibiting FXIII with an inhibitor trapping the active conformation and revealing the binding mode. The active conformation is further characterized by a structural rearrangement of the beta barrel domains rending the active site accessible. The active site provides data enabeling rational drug design based on the use of such structural data.

Blood coagulation factor XIII, also called plasma transglutaminase, has a unique function stabilizing the fibrin clot. The introduction of covalent cross-links between the γ chains and subsequently the α chains of fibrin provides mechanical stability and modulates the visco-elastic properties. Further the plasma transglutaminase decorates the clot with anti-fibrinolytic factors, especially with α2-antiplasmin. For decades factor XIIIa is considered a suitable target for anti-coagulation in certain risk patients due to the unique mode of action. Targeting factor XIIIa with a direct acting blocker would not impair the thrombin level or the platelet activity avoiding critical bleeding episodes.

The present invention provides the possibility to identify and / or design inhibitiors of blood coagulation factor XIIIa and relates to the crystal form of blood coagulation factor XIIIa, the crystal structure information obtained from the crystal form, methods of preparing such a crystal form, its use for the identification and/or design of inhibitors of blood coagulation factor XIIIa activity and the diagnostic and/or pharmaceutical use of those inhibitors of blood coagulation factor XIIIa identified by these methods.

### Detailed description of the invention

The present invention relates to the active conformation of blood coagulation factor XIIIa also named herein blood coagulation FXllla or simply FXllla.

### Crystal forms of Blood Coagulation Factor XIIIa

The present invention is directed to the crystal forms of blood coagulation factor XIIIa characterized as having a space group of P1, two monomers of coagulation factor XIIIa in the asymmetric unit and unit cell dimension of a₁ = 56 ± 3 Å, b₁ = 80 ± 3 Å, C₁ = 103 ± 3 Å, α₁ = 88 ± 3°, β₁ = 77 ± 3° and γ₁ = 82 ± 3°; or
having a space group of P1, two monomers of coagulation factor XIIIa in the asymmetric unit and unit cell dimension of a₂ = 57 ± 3 Å, b₂ = 64 ± 3 Å, c₂ = 67 ± 3 Å, β₂ = 76 ± 3°, β₂ = 72 ± 3° and γ₂ = 83 ± 3°; or
having a space group of C2, two monomers of coagulation factor XIIIa in the asymmetric unit and unit cell dimension of a₃ = 90 ± 3 Å, b₃ = 65 ± 3 Å, c₃ = 70 ± 3 Å, α₃ = 90 ± 3°, β₃ = 96 ± 3° and γ₃ = 90 ± 3°.

Thus so far three crystal structures of blood coagulation factor XIIIa could be obtained with three different unit cells while always two monomers of coagulation factor XIIIa are present in one unit cell.

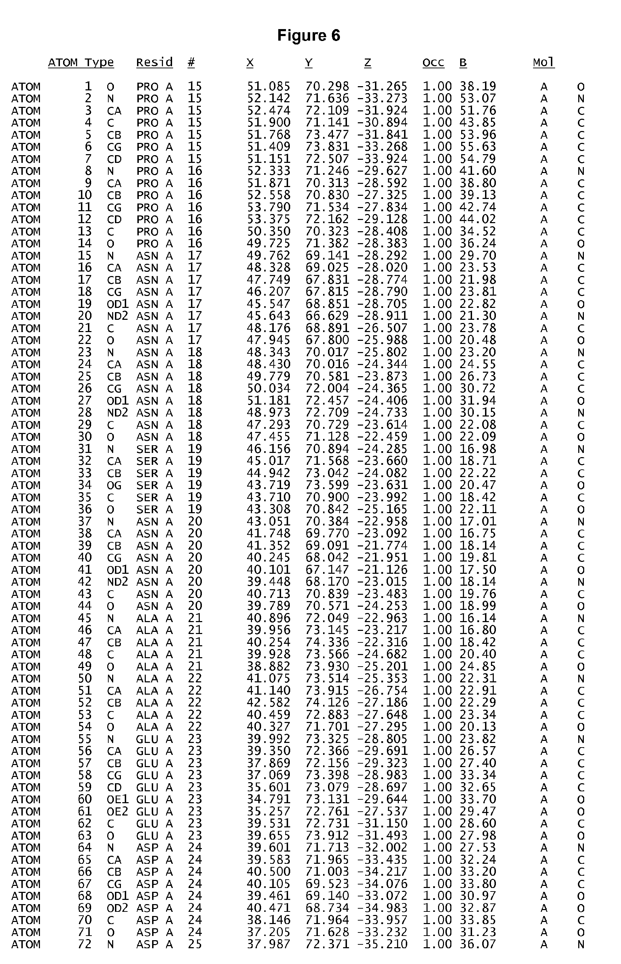

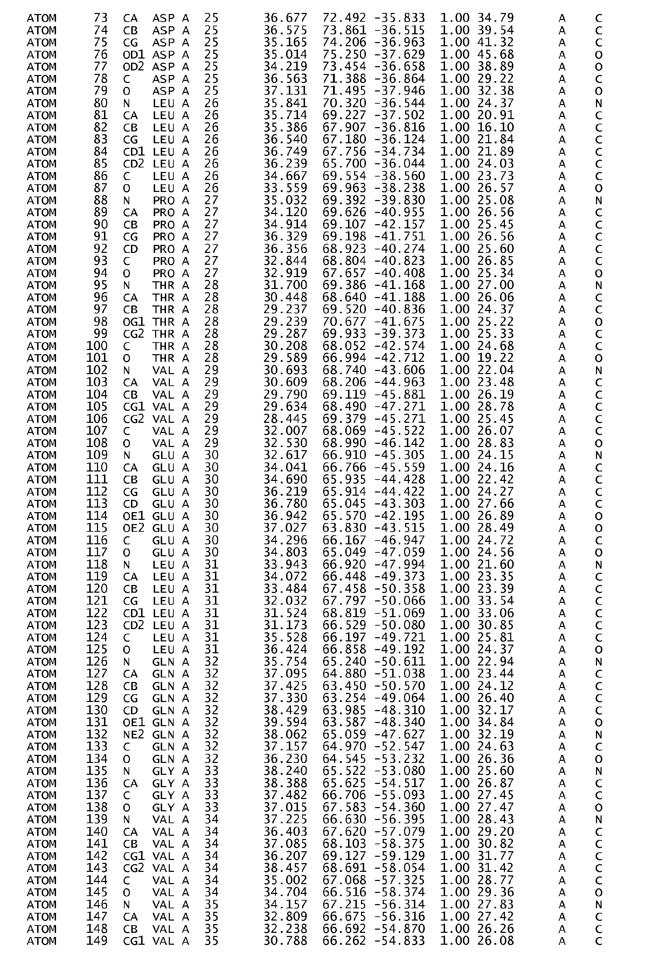

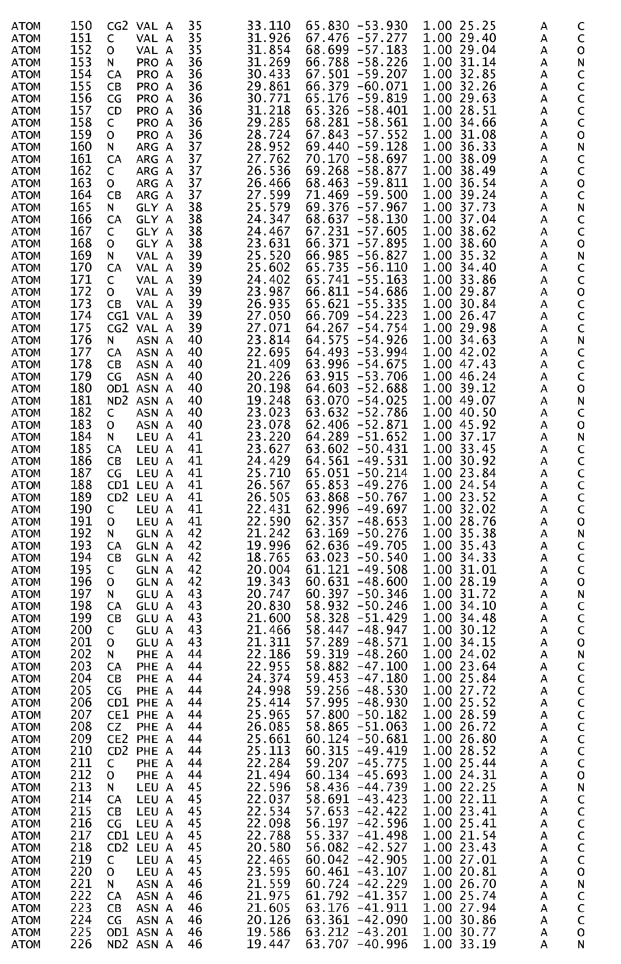

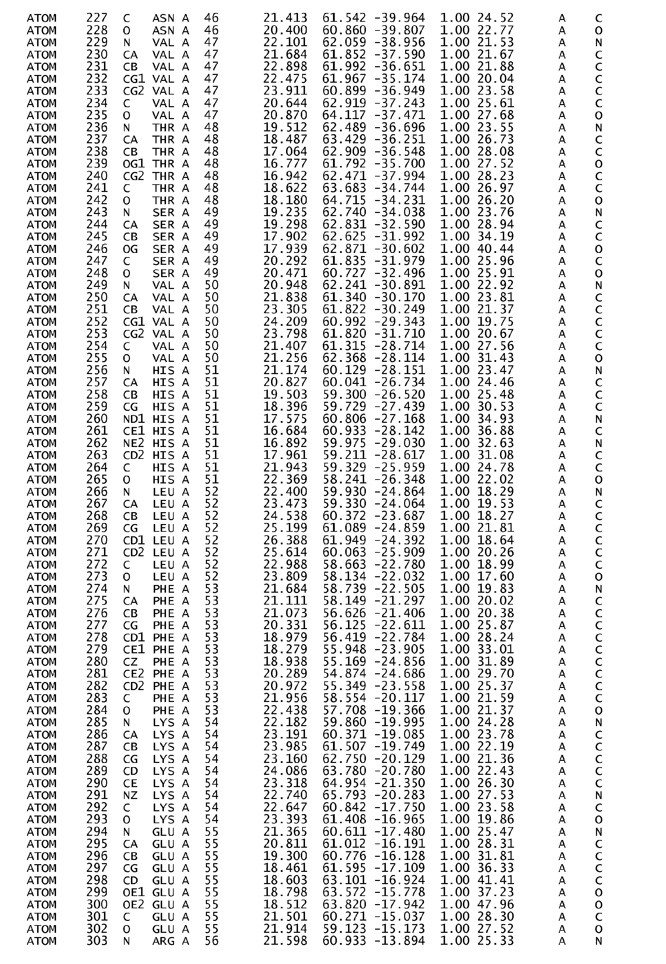

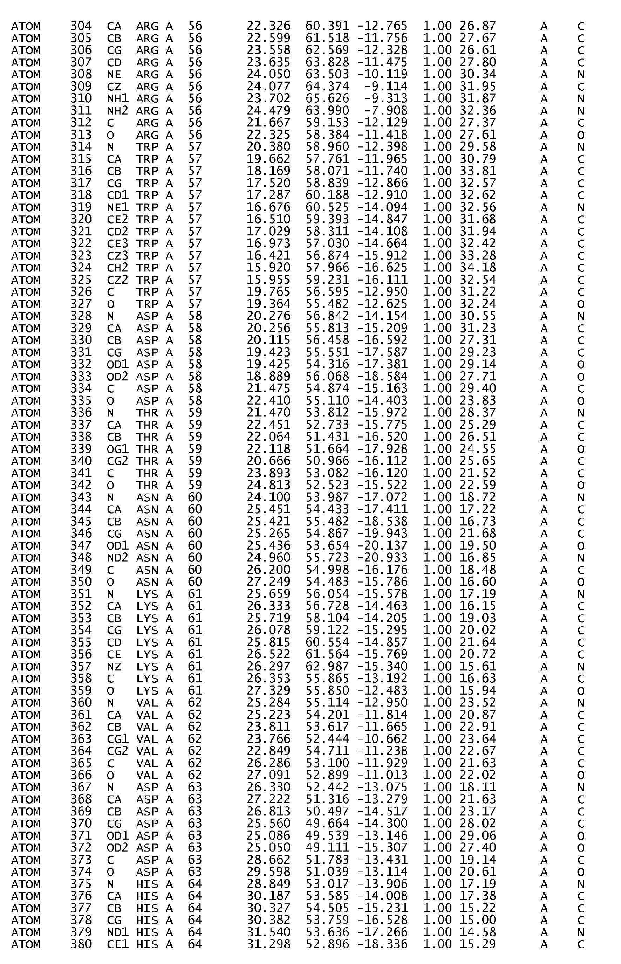

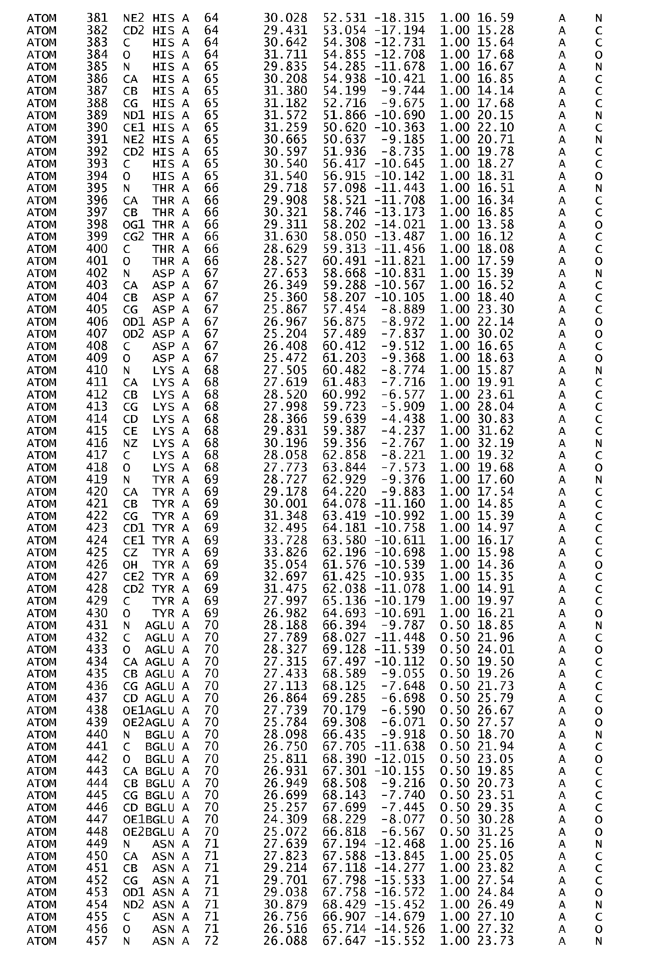

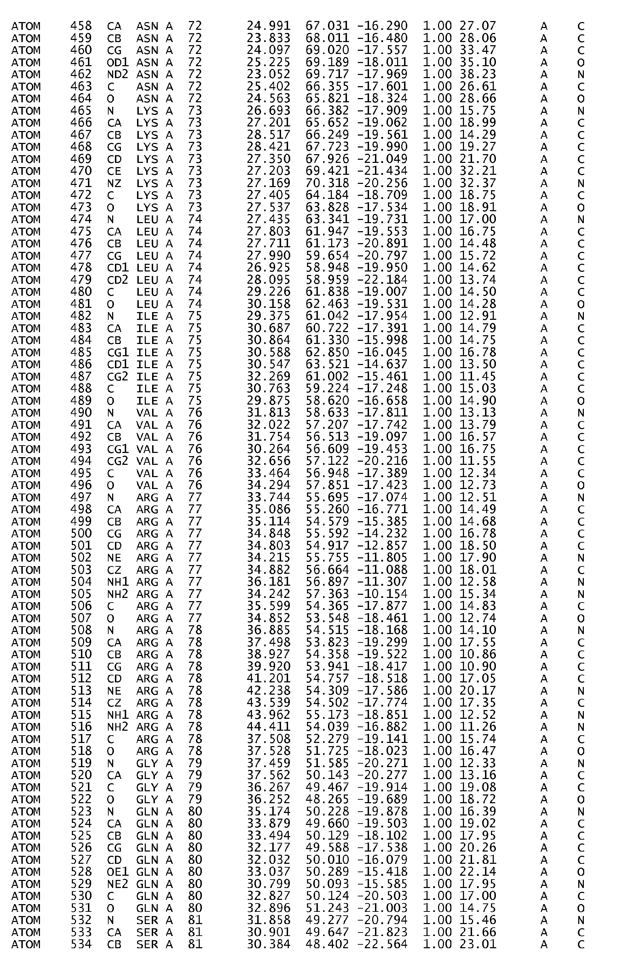

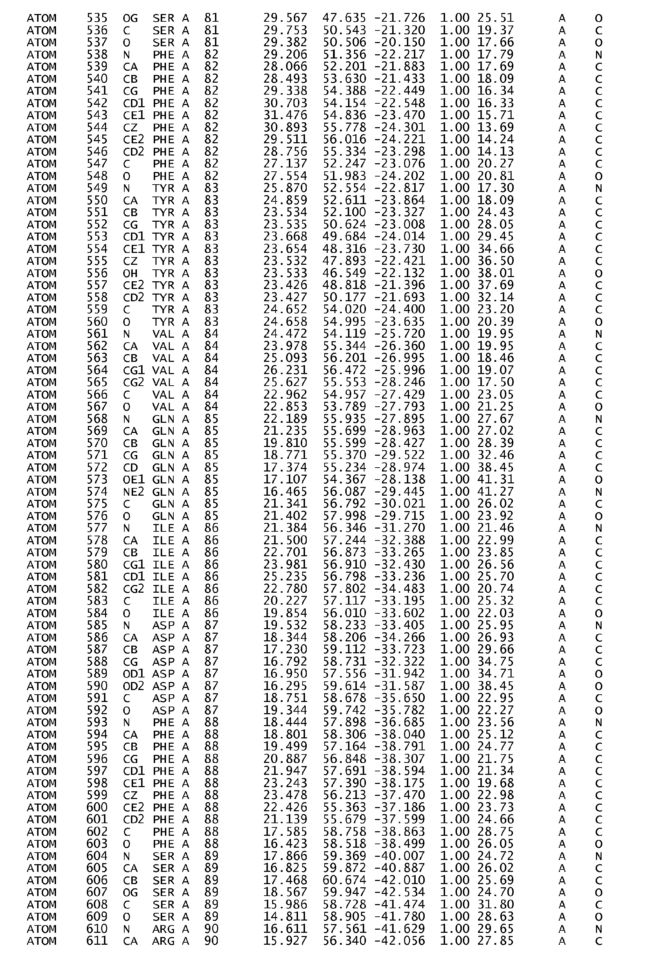

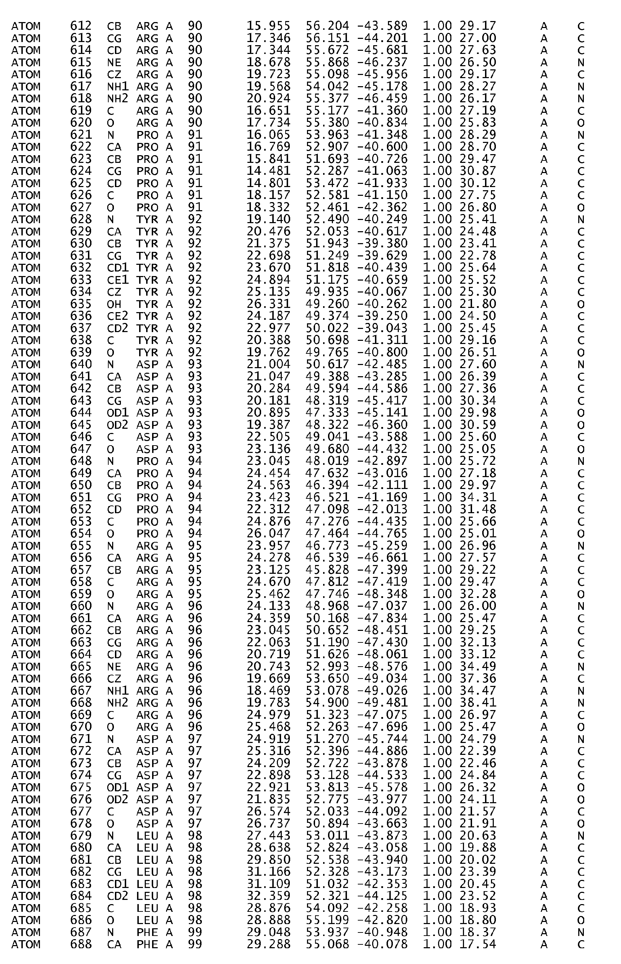

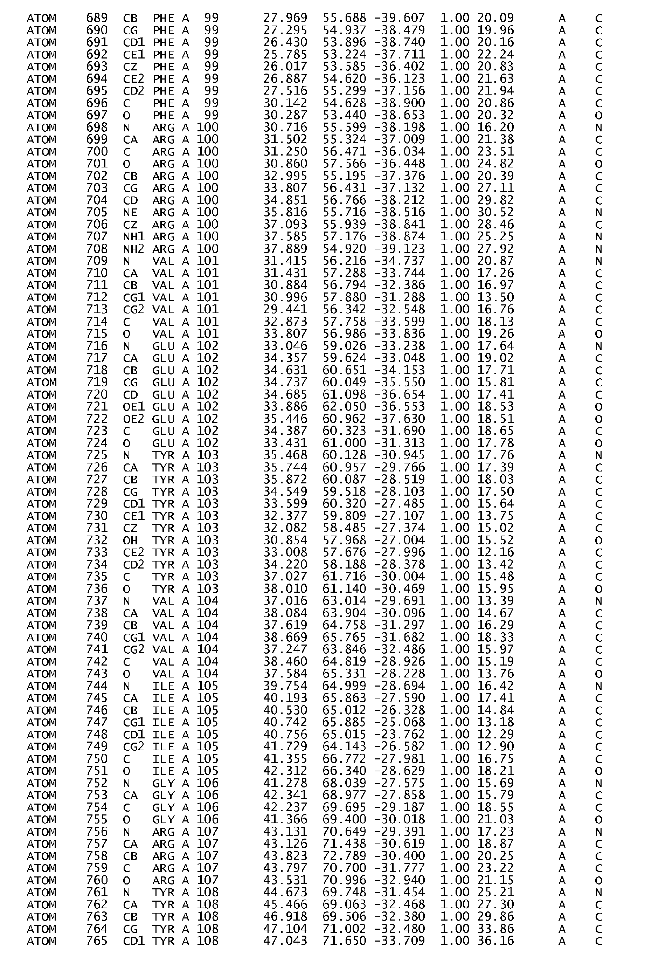

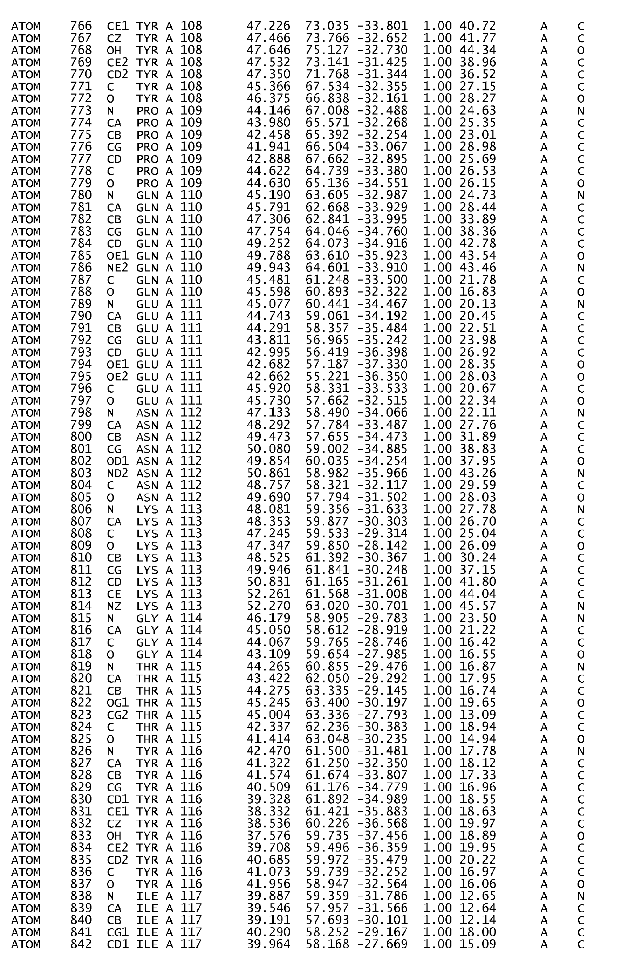

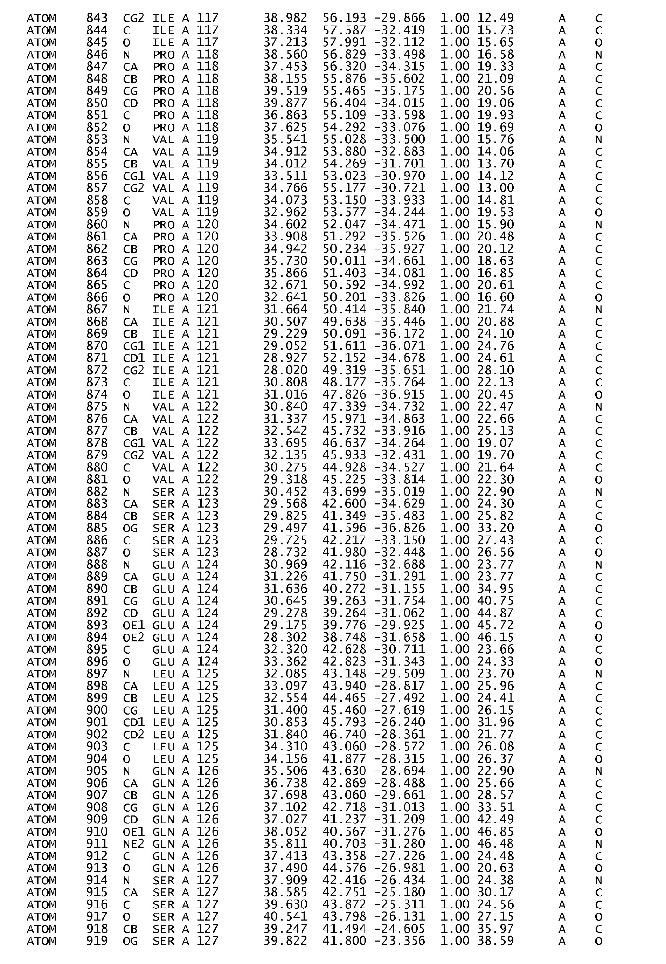

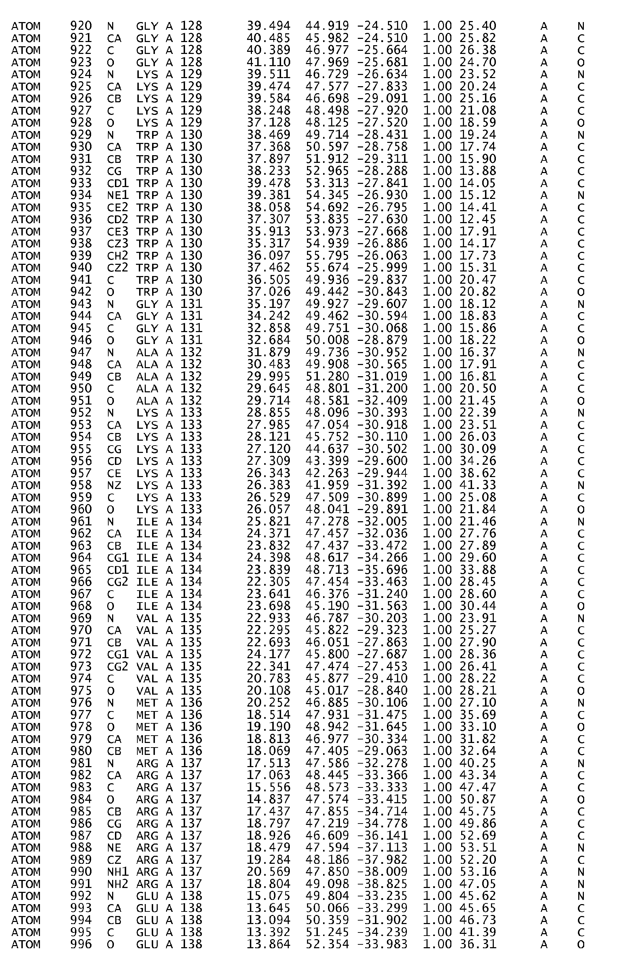

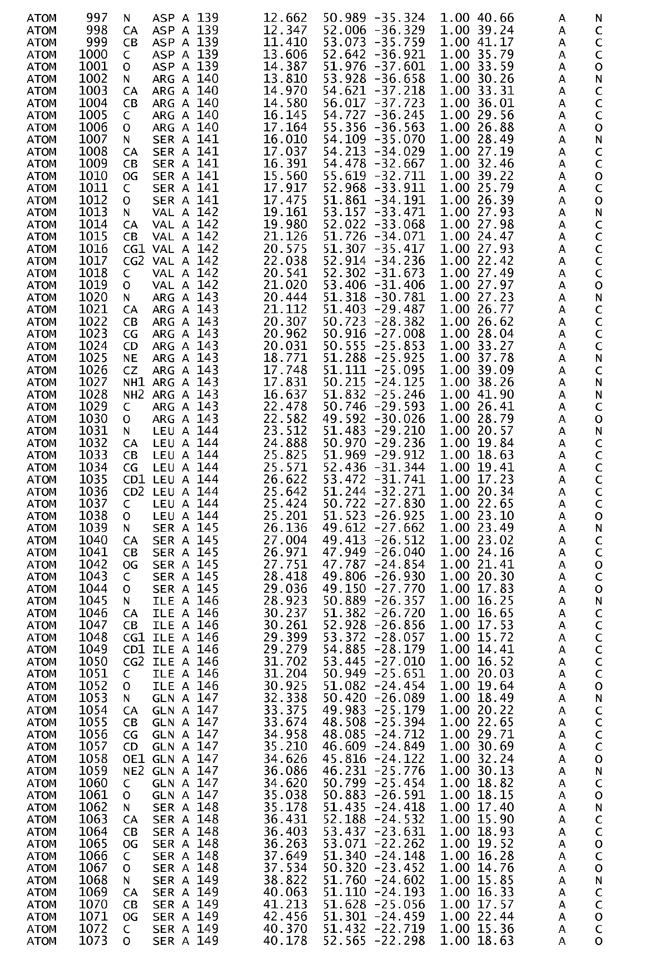

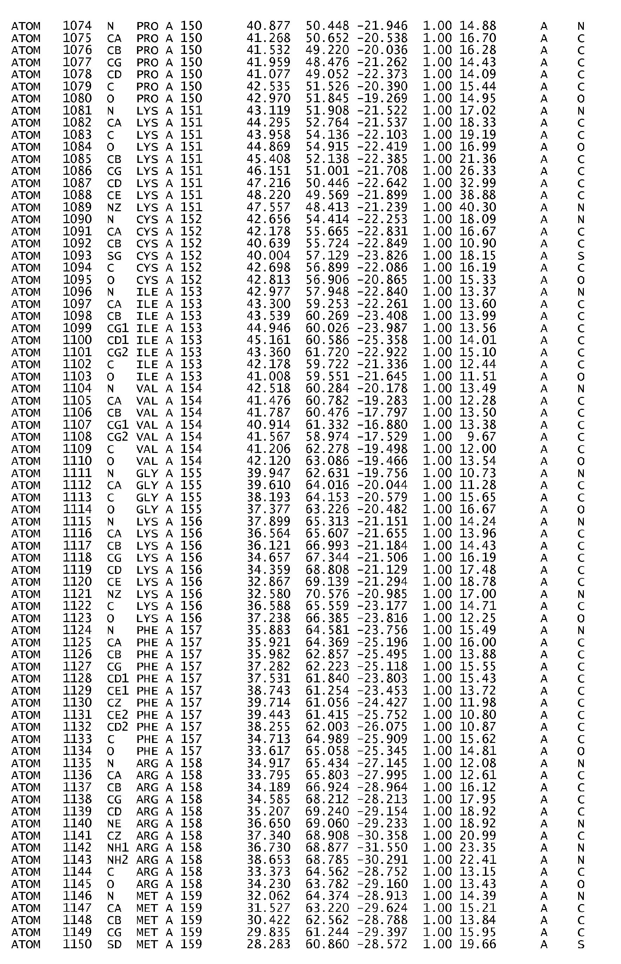

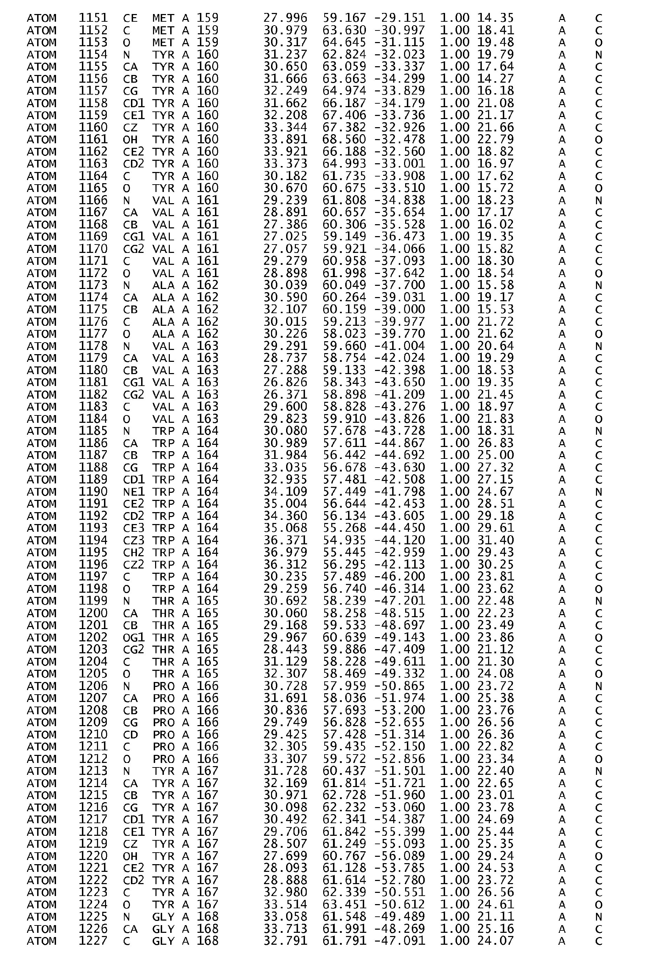

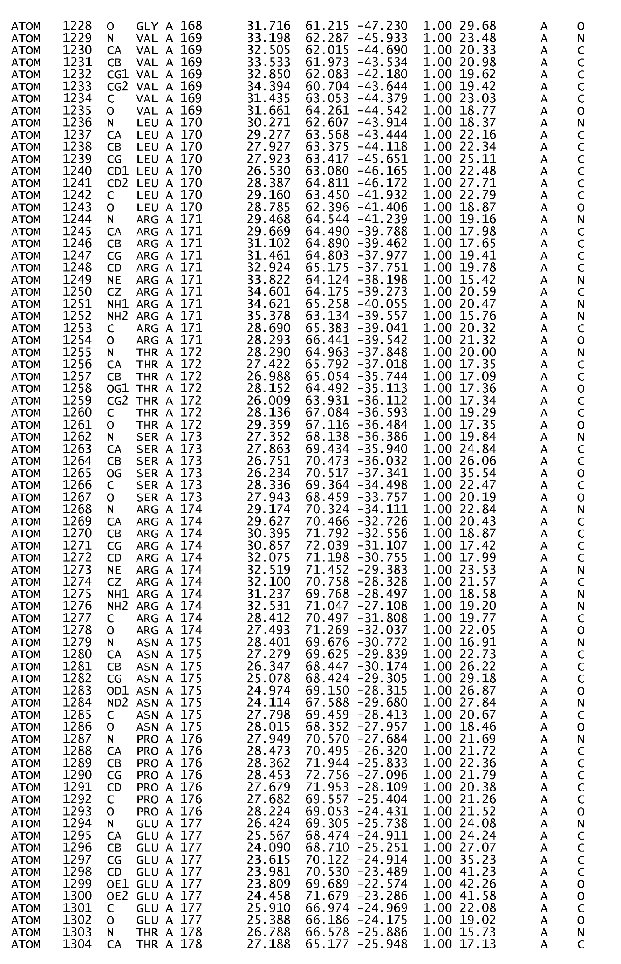

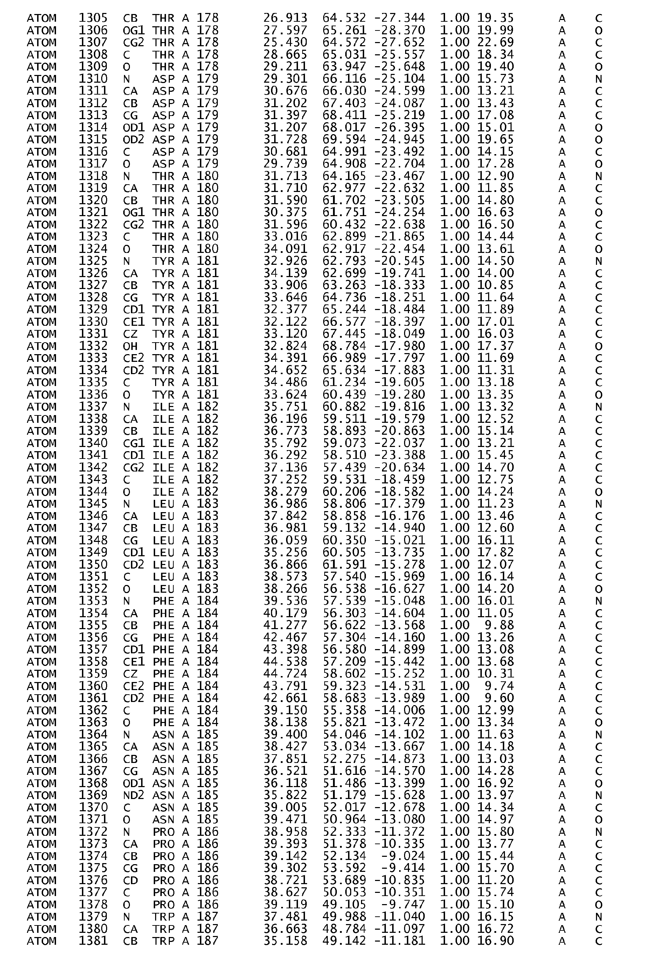

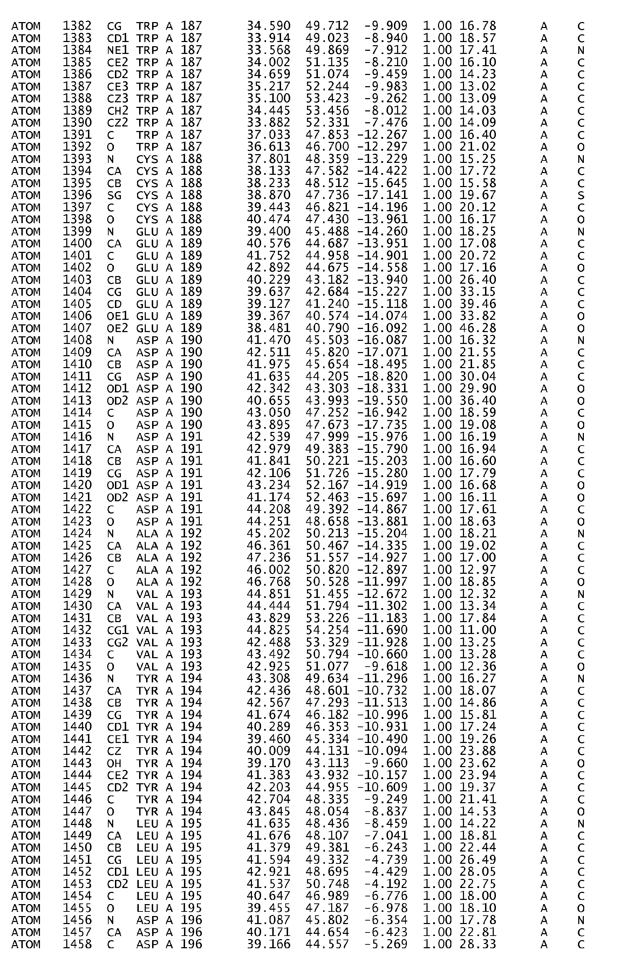

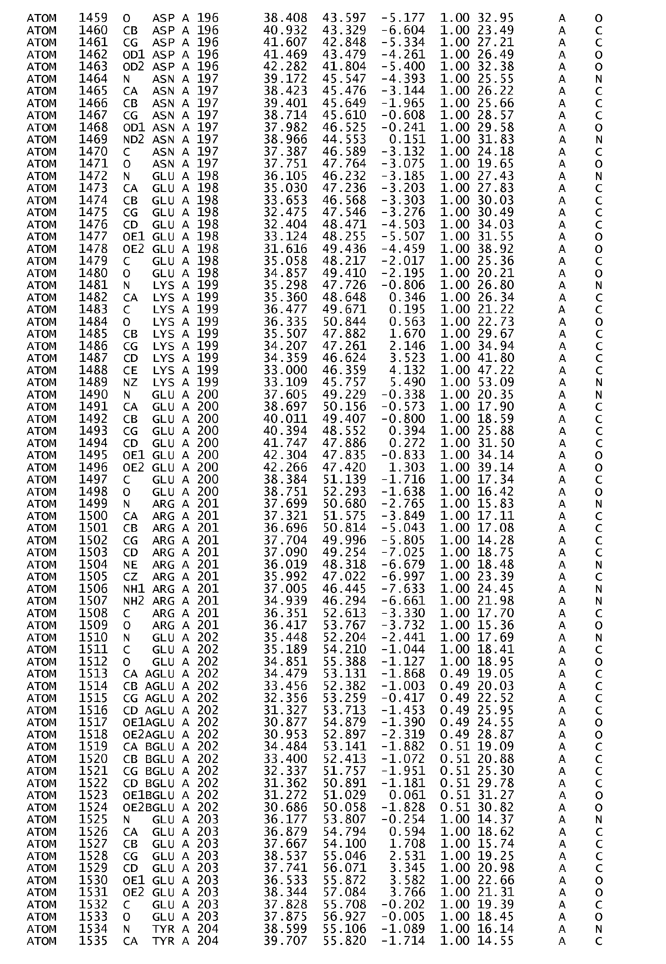

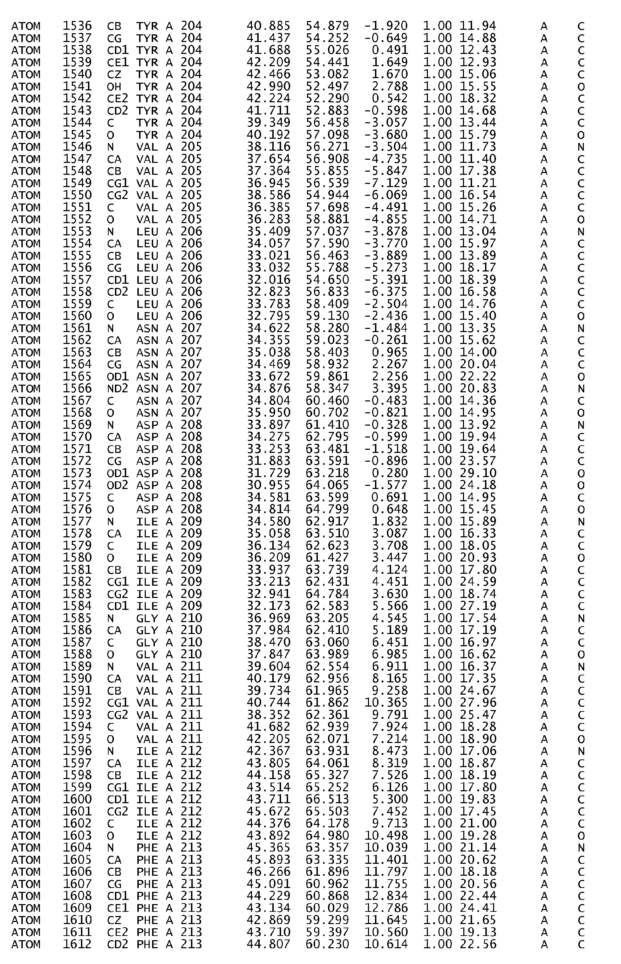

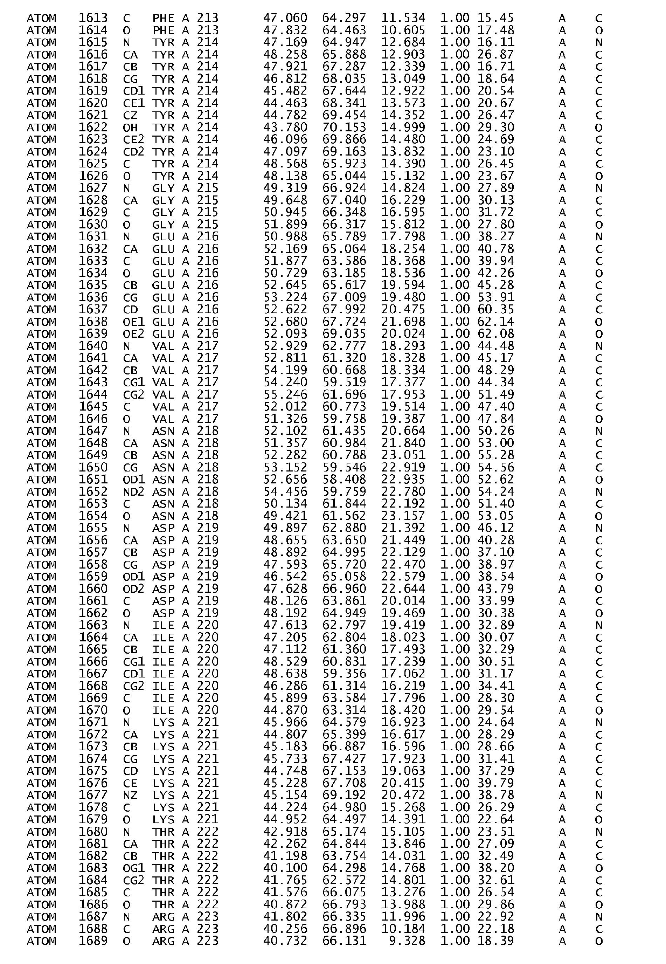

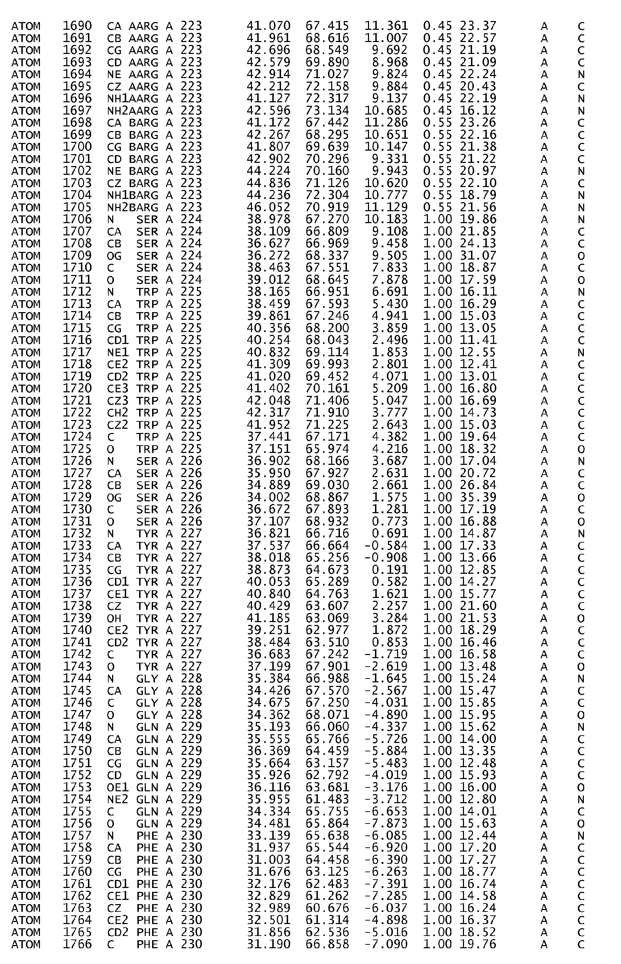

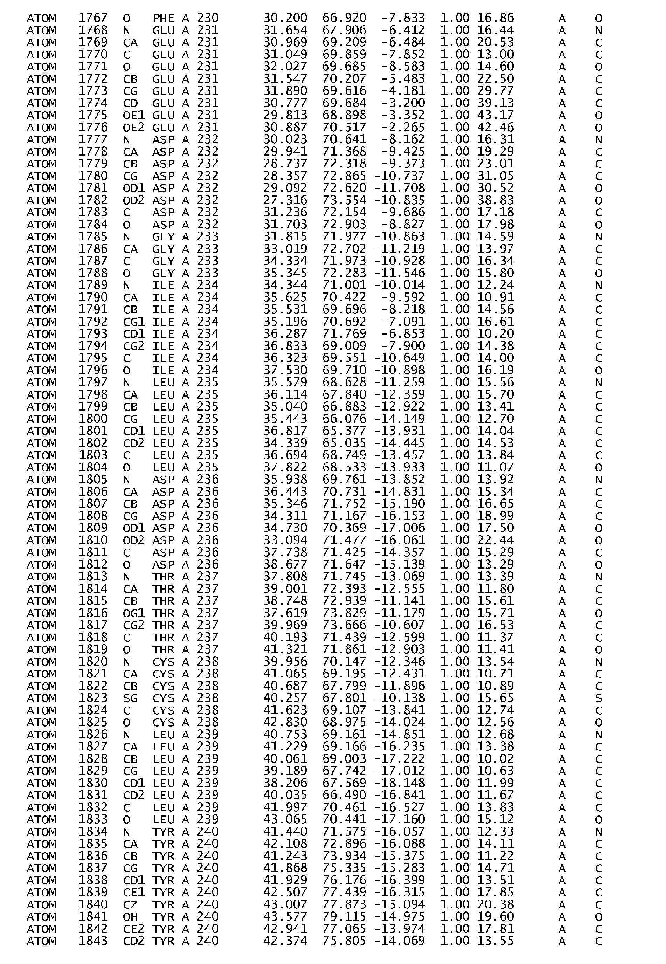

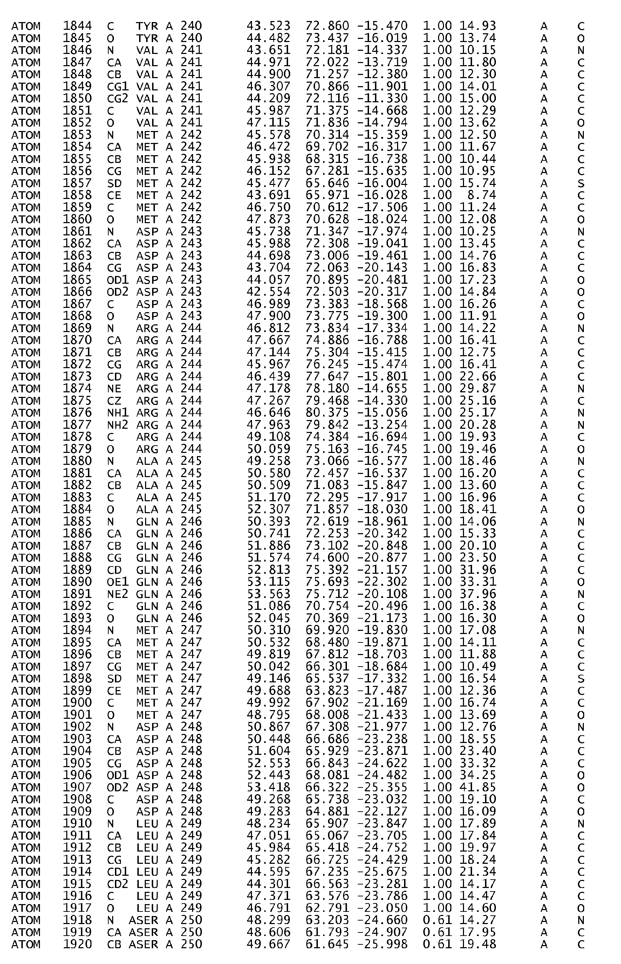

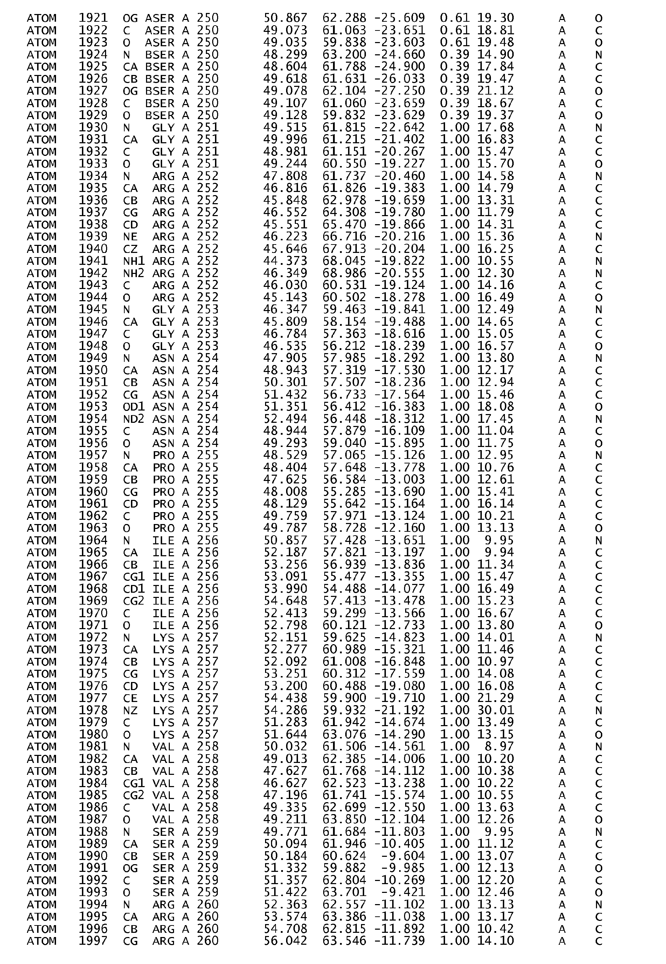

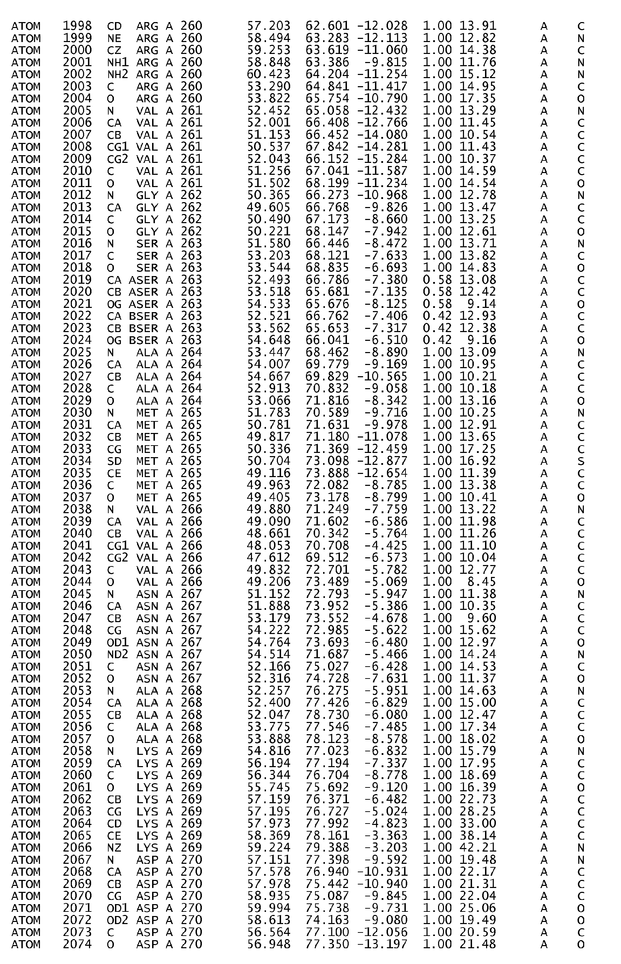

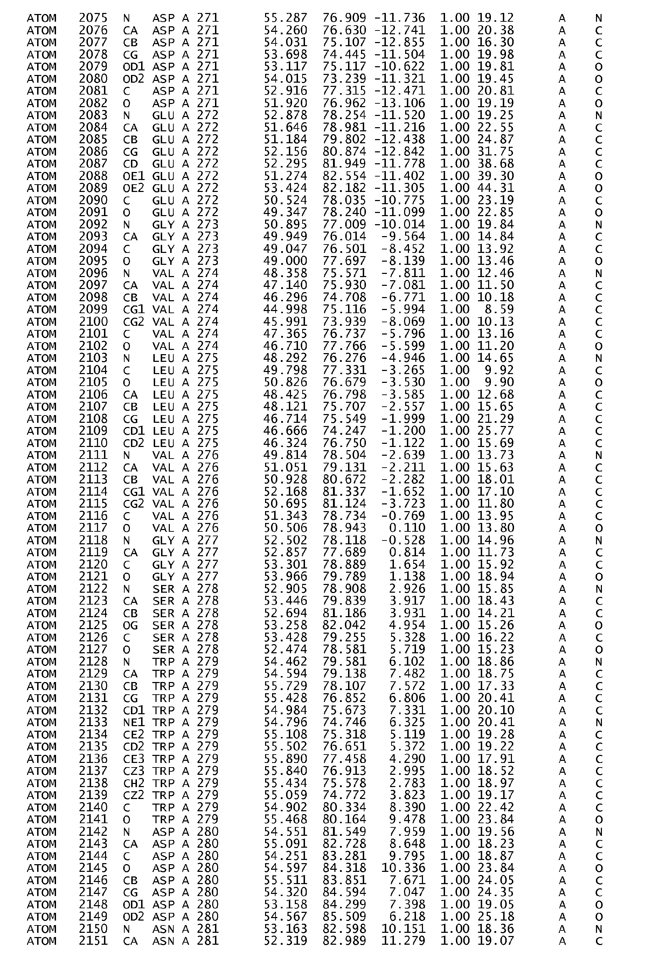

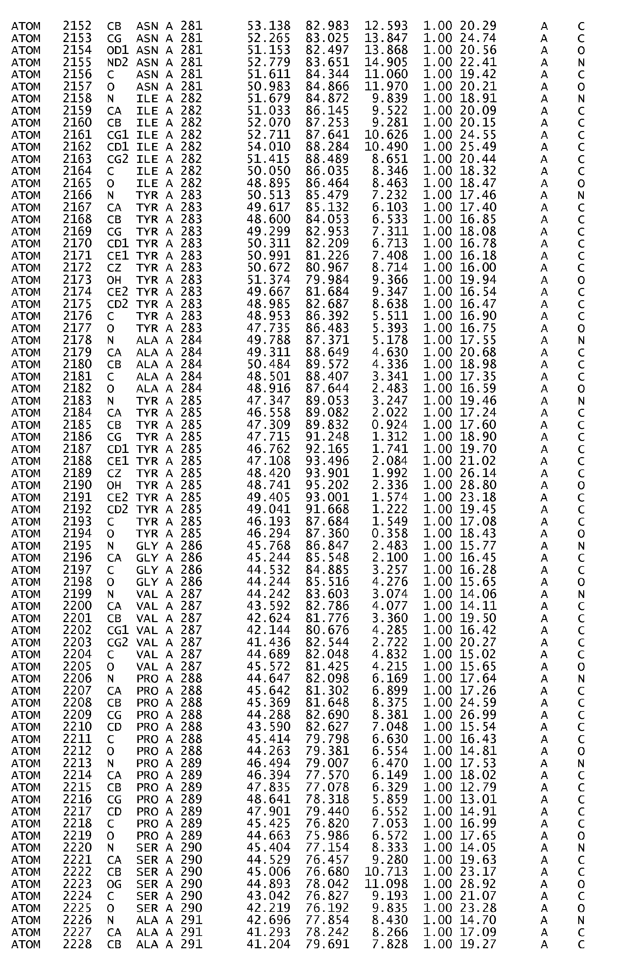

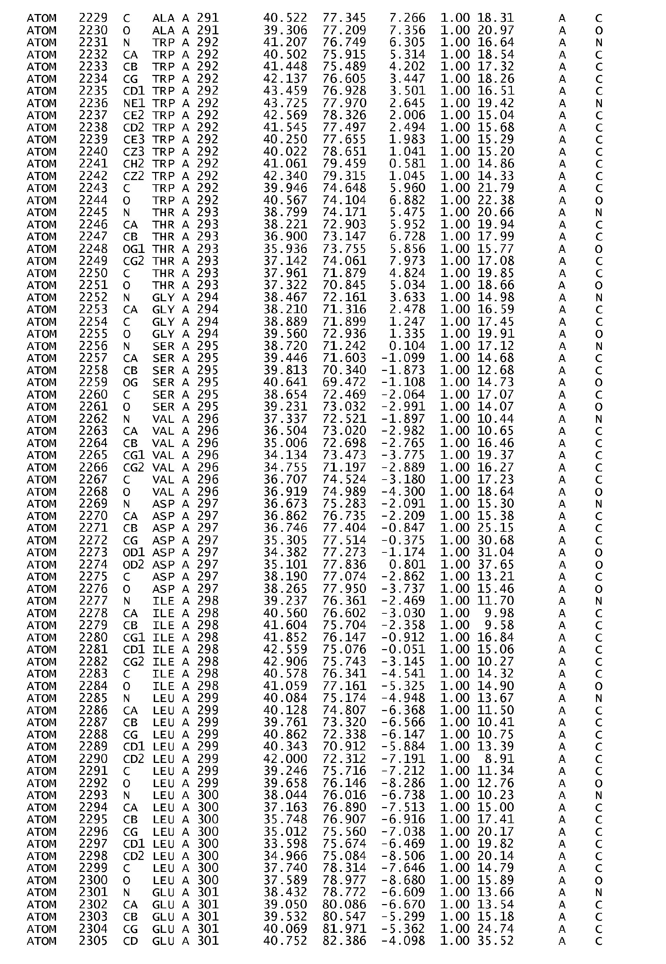

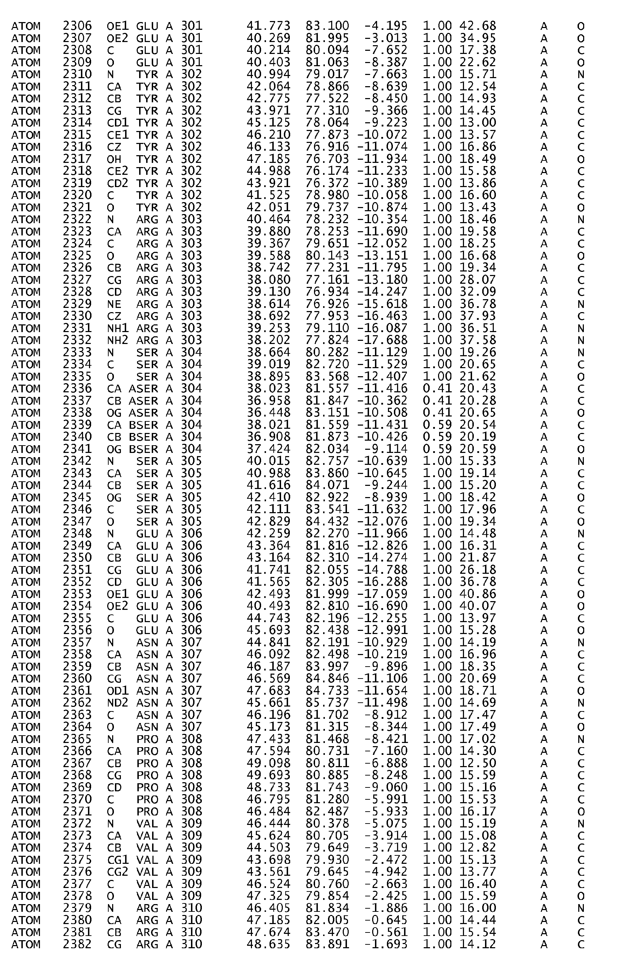

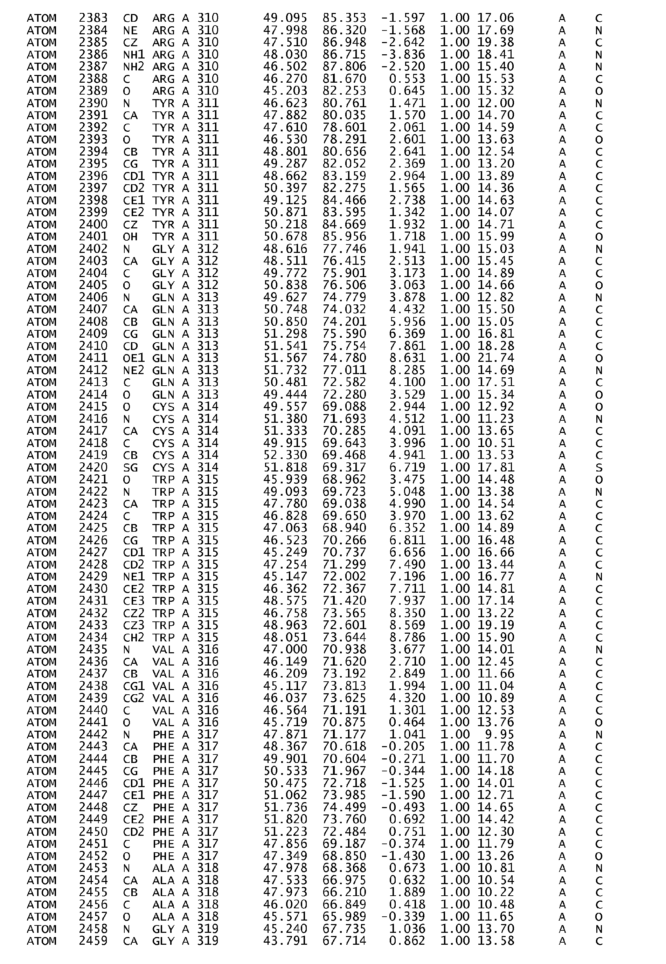

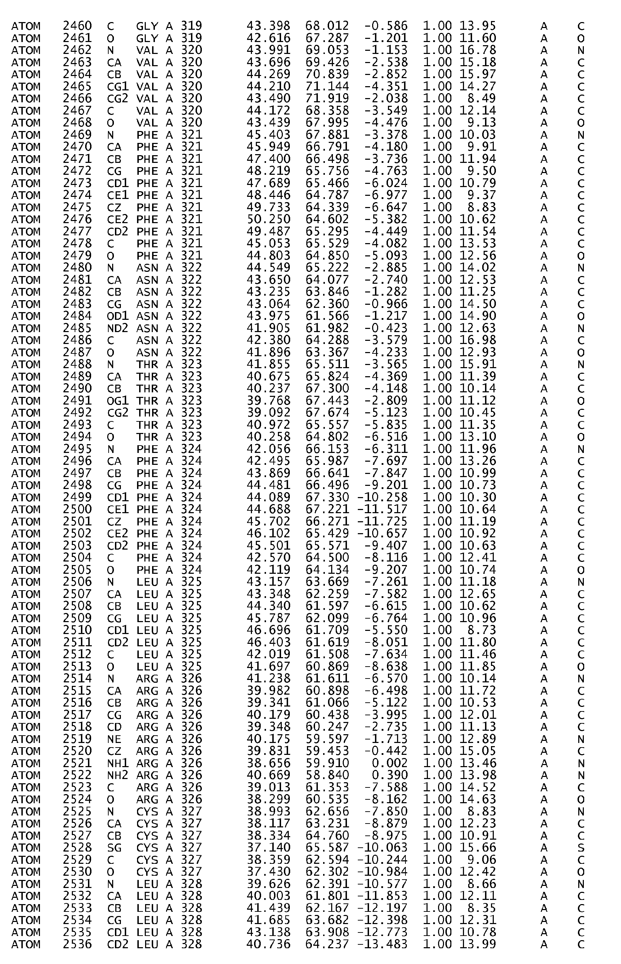

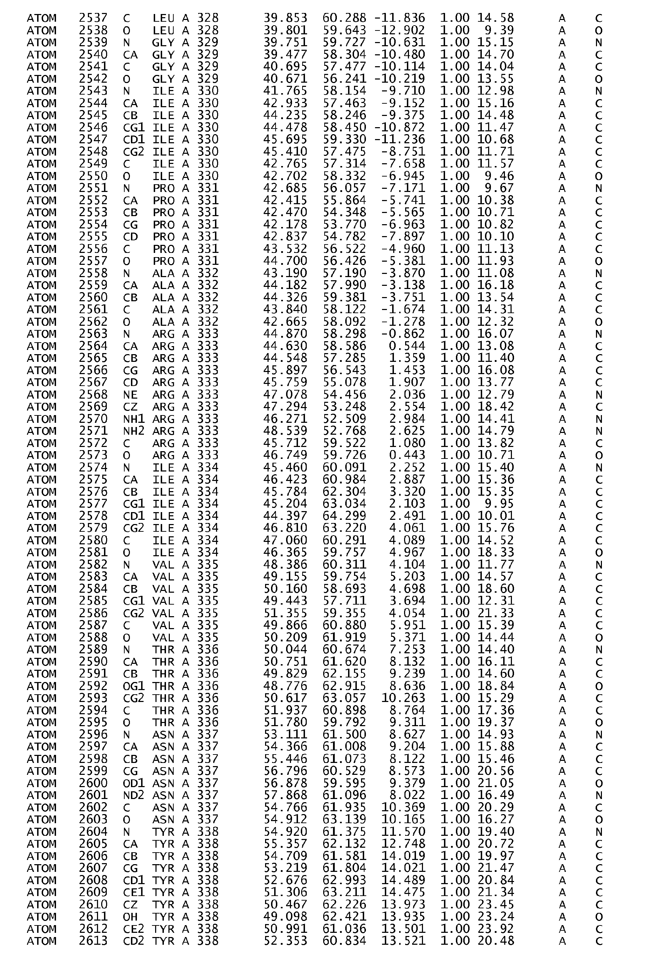

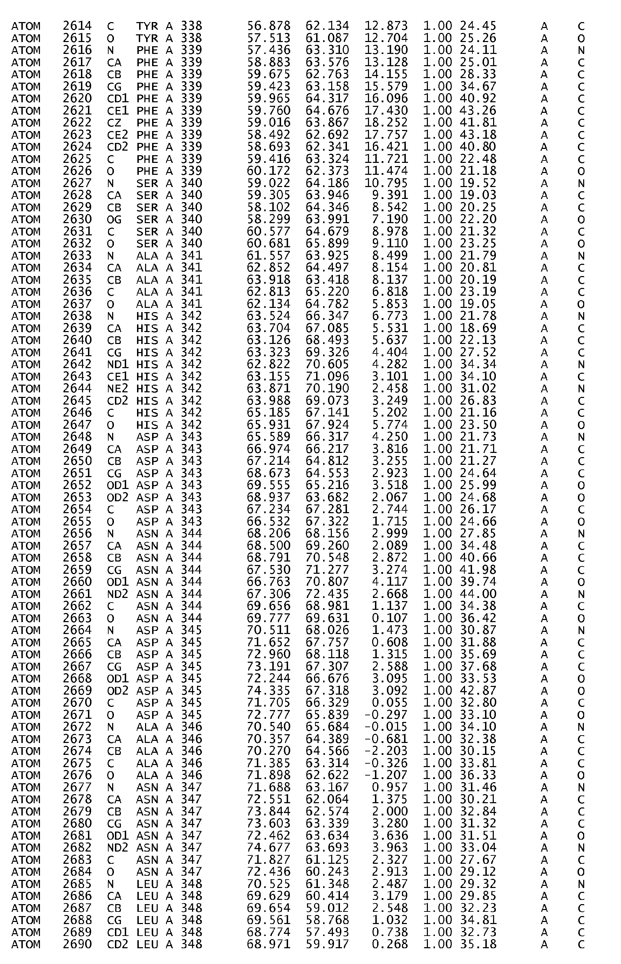

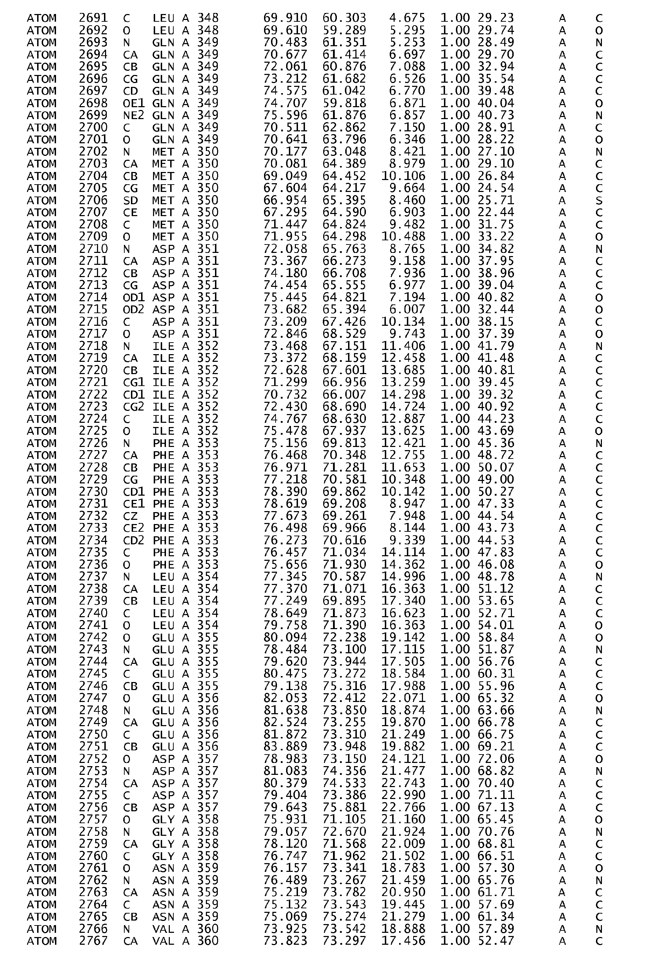

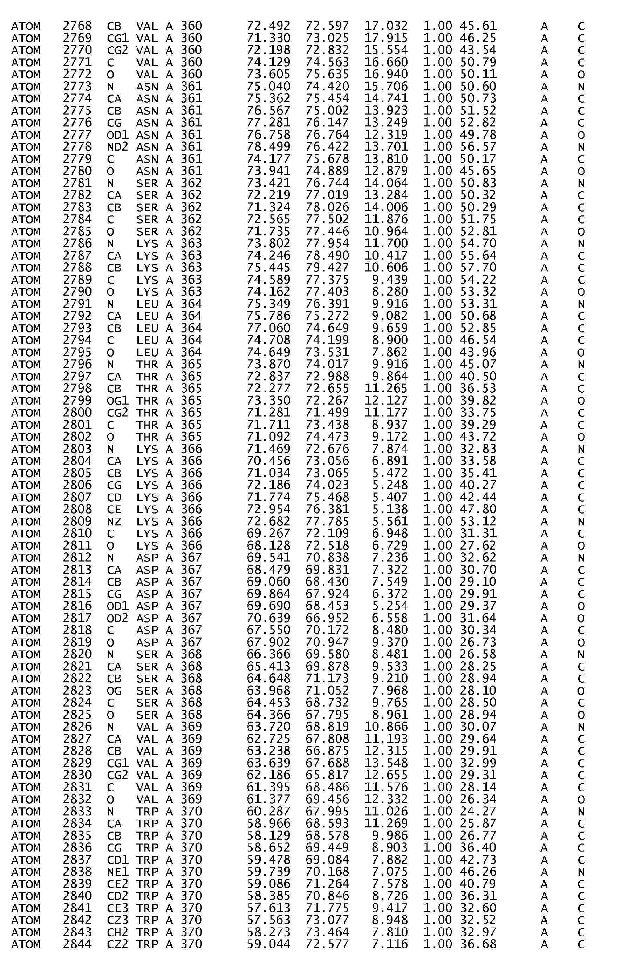

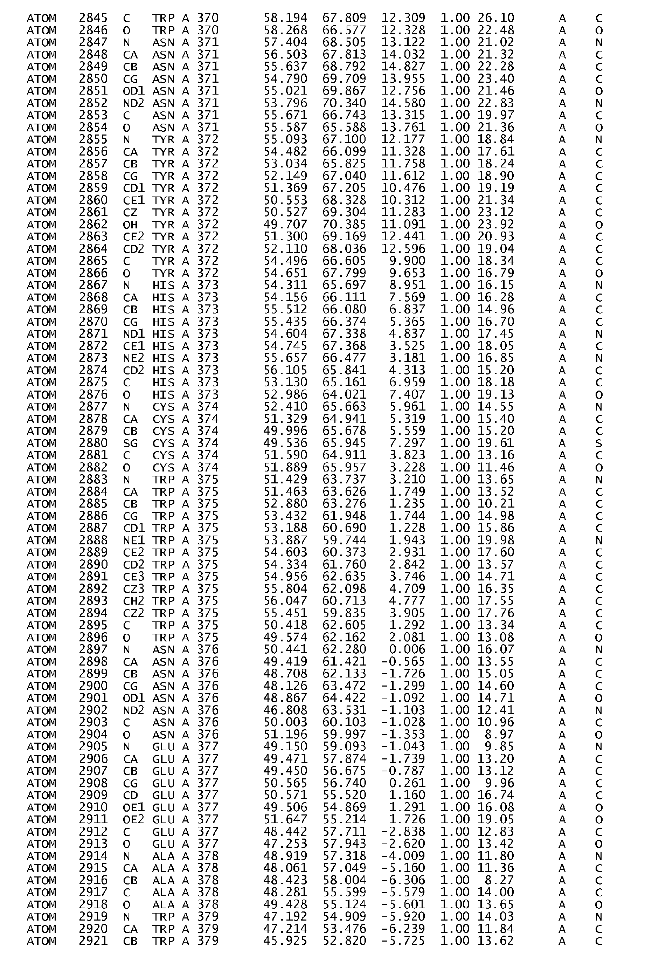

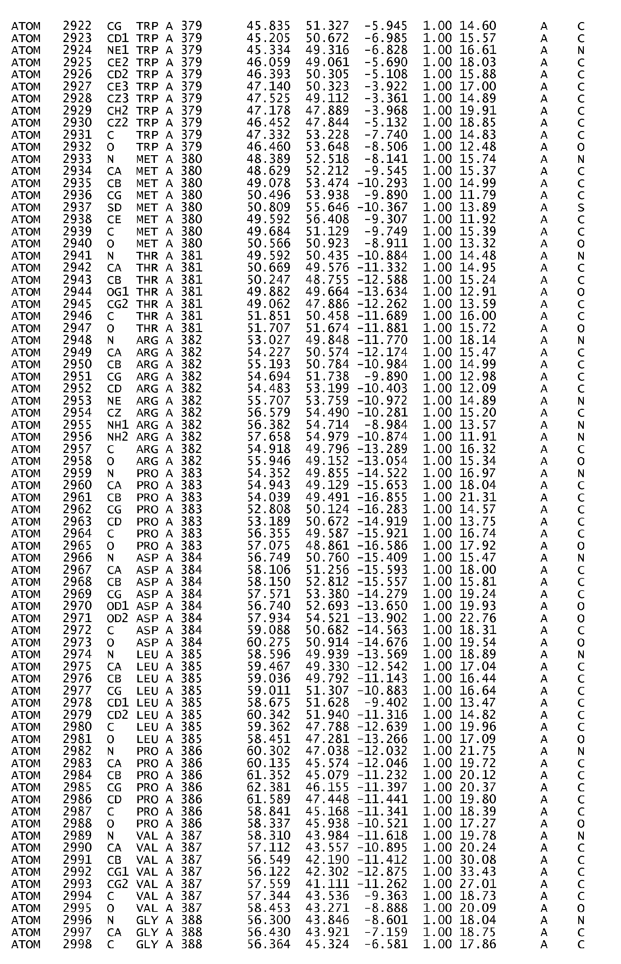

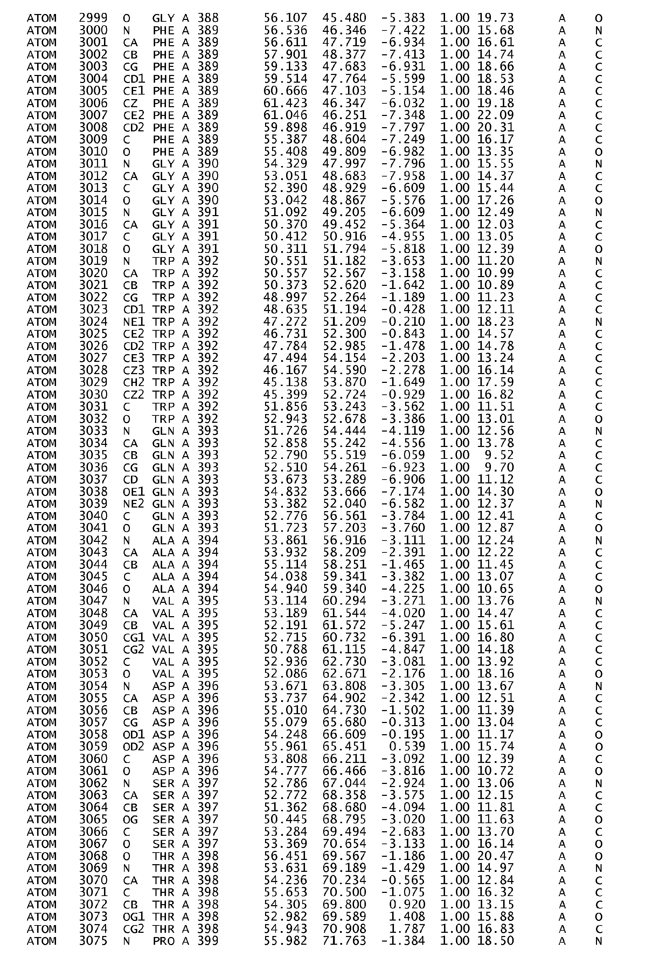

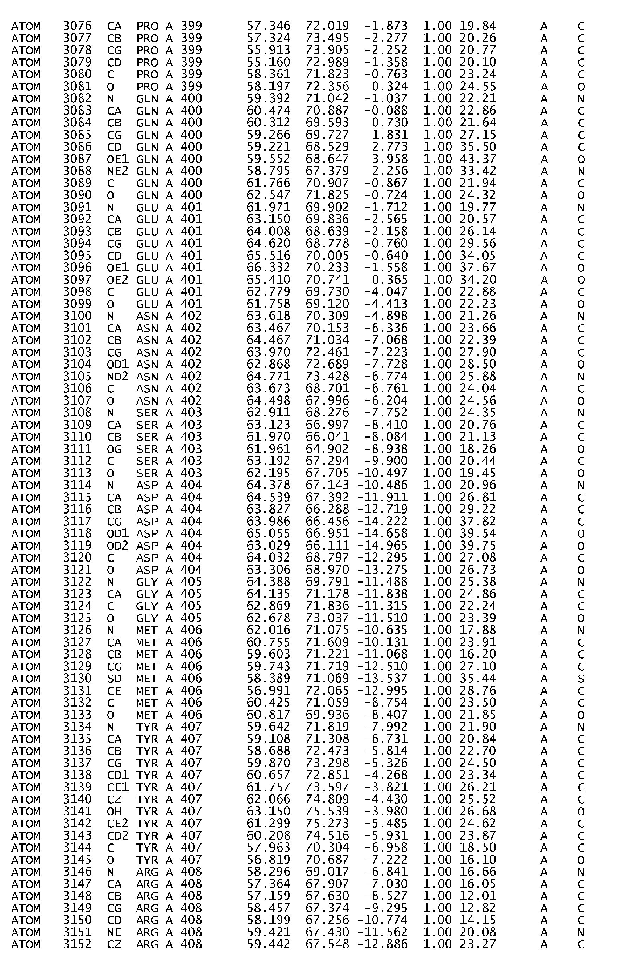

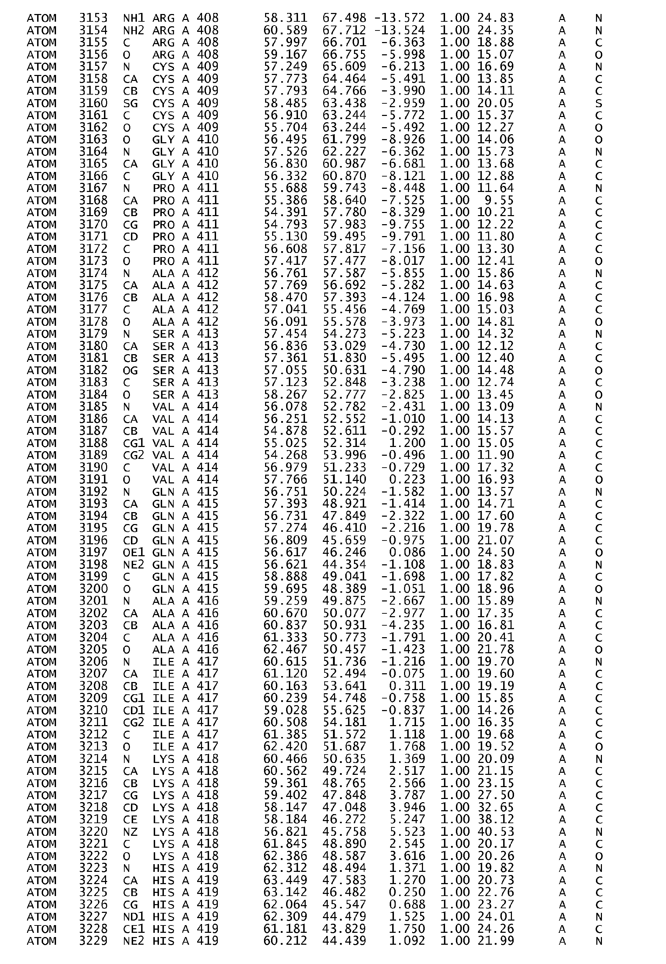

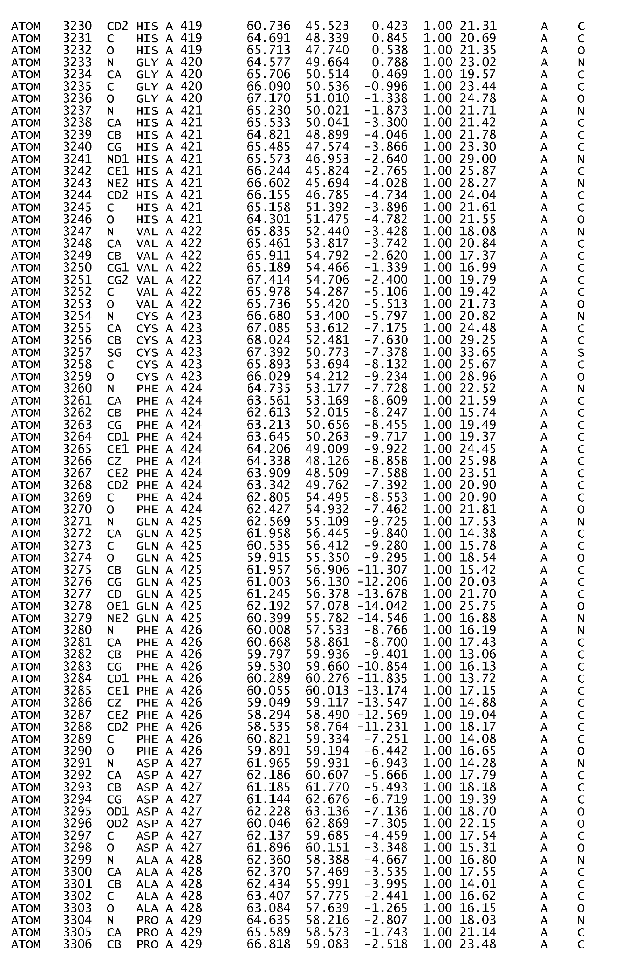

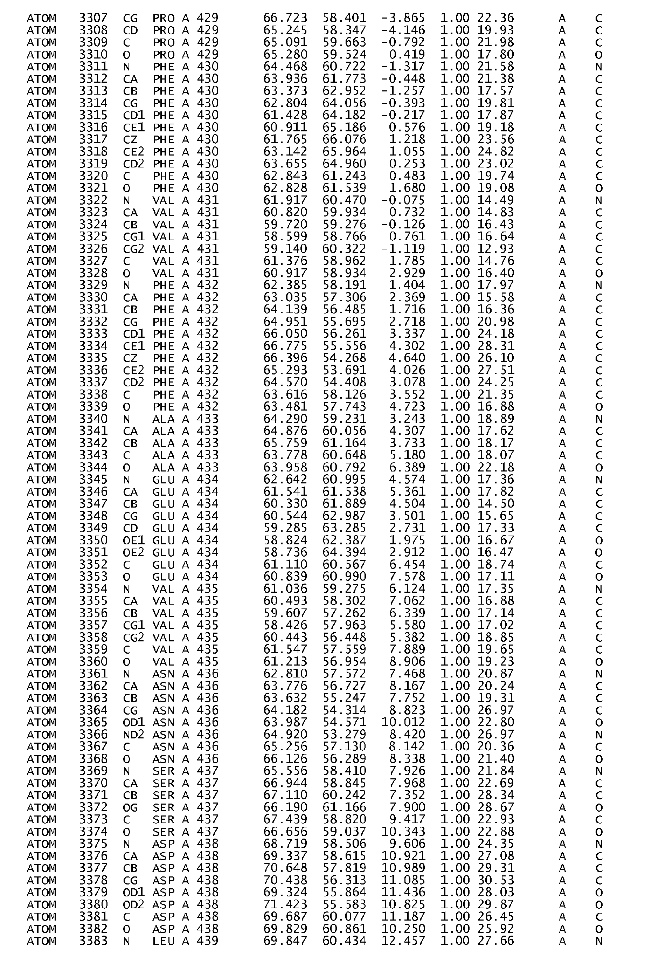

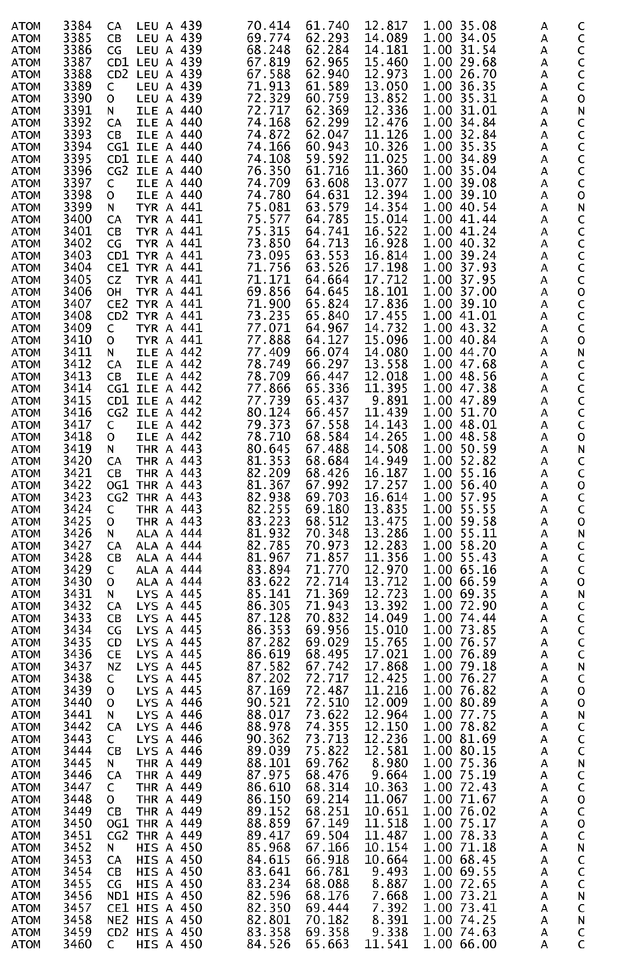

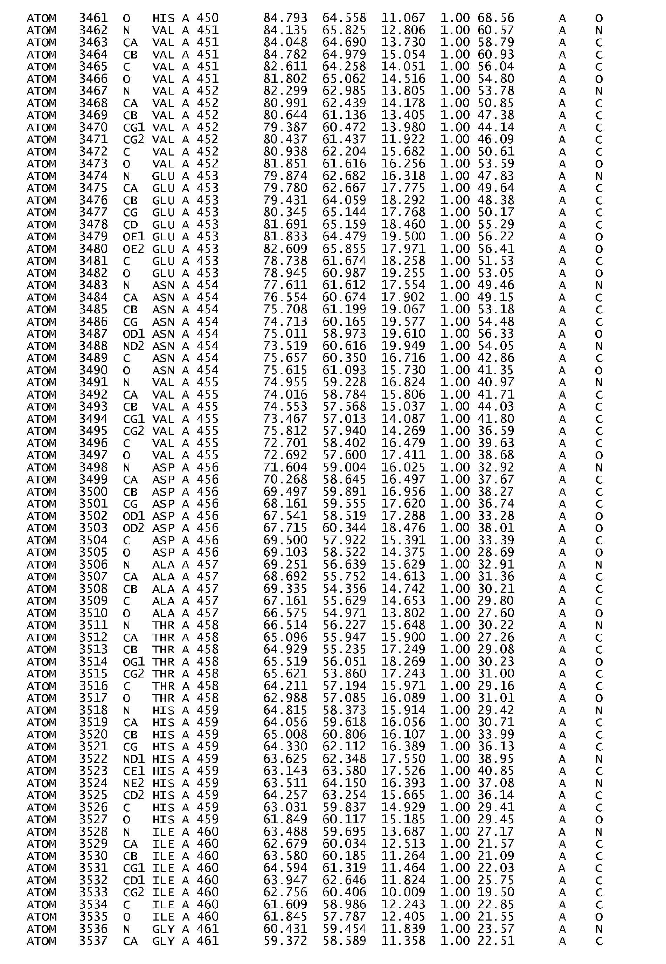

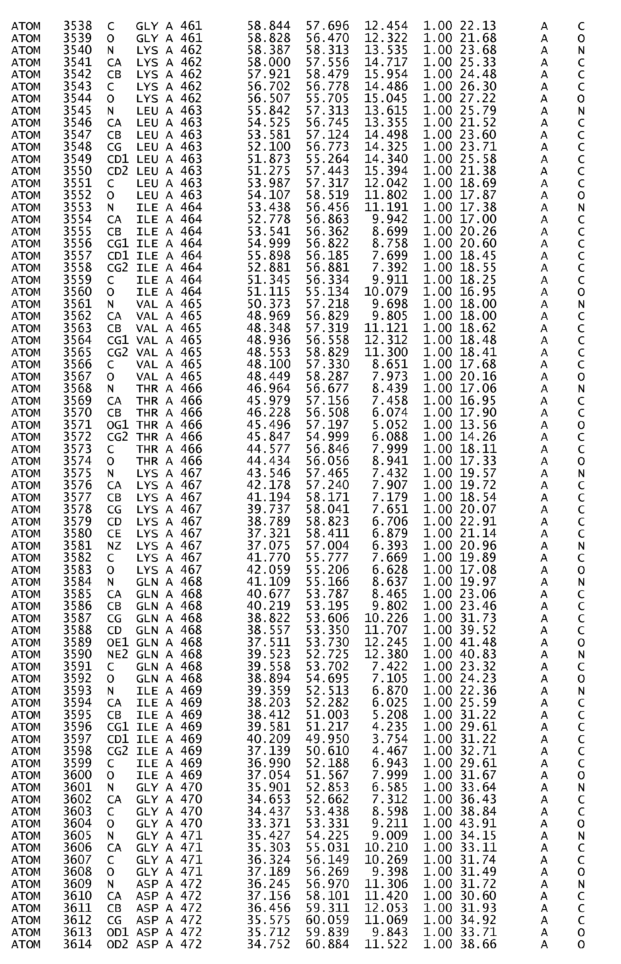

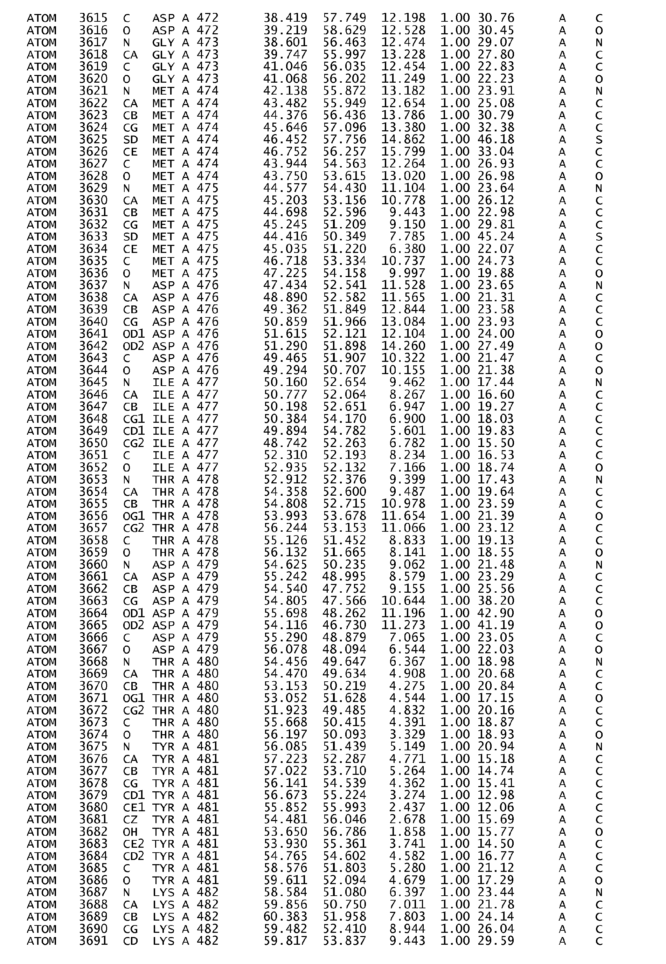

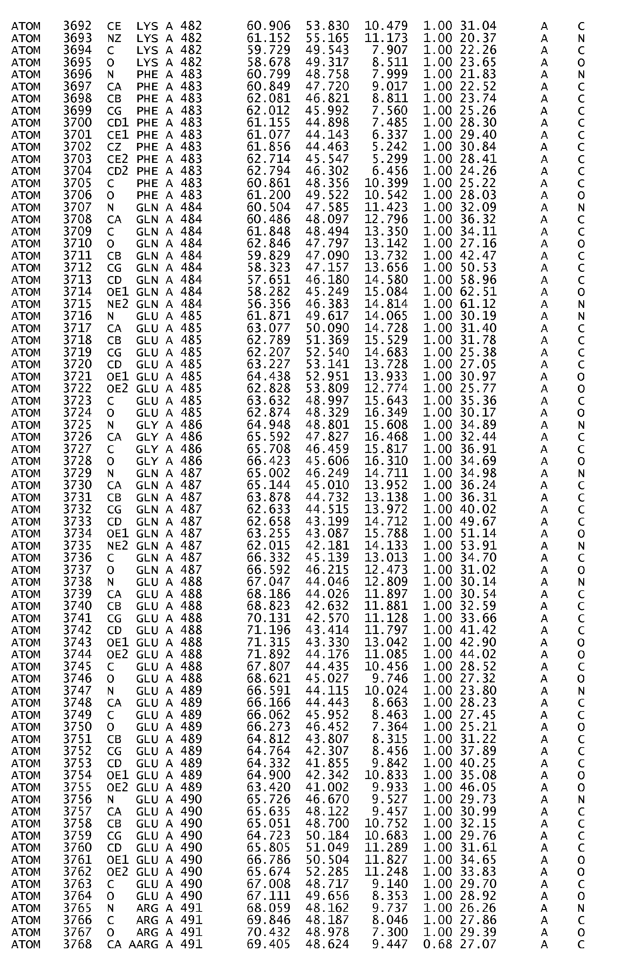

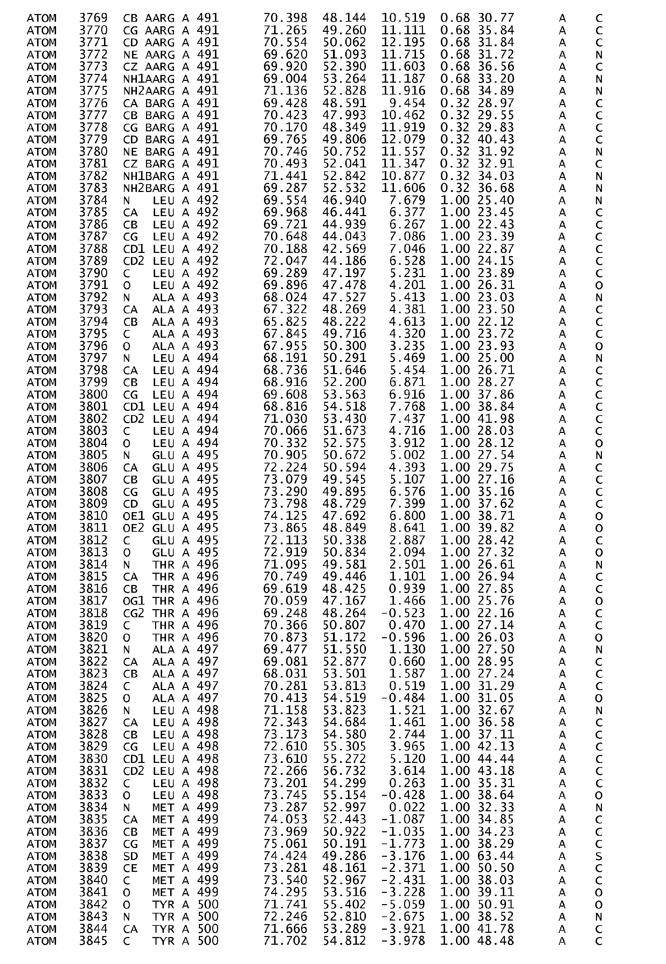

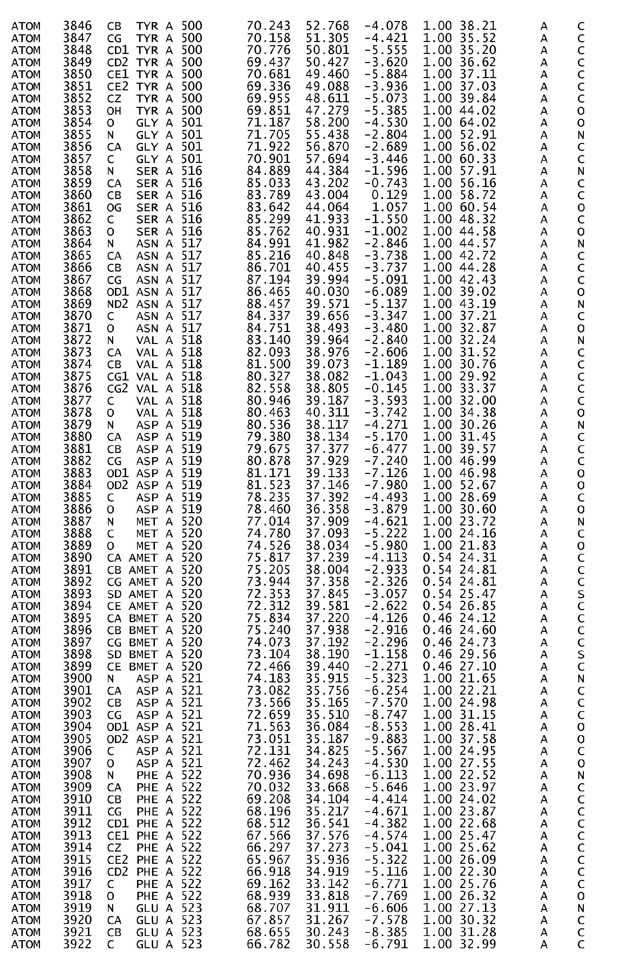

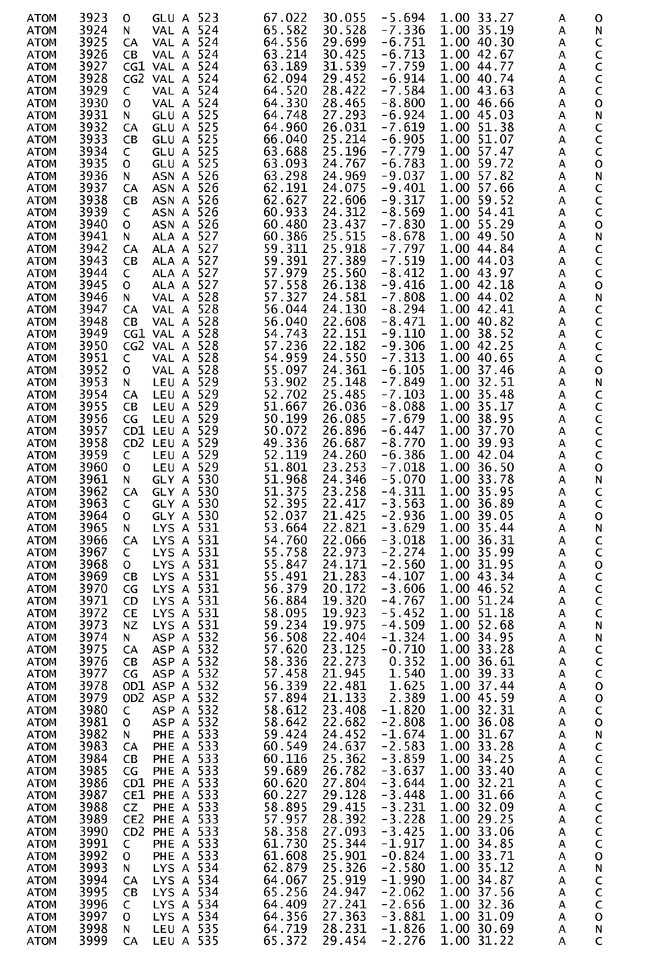

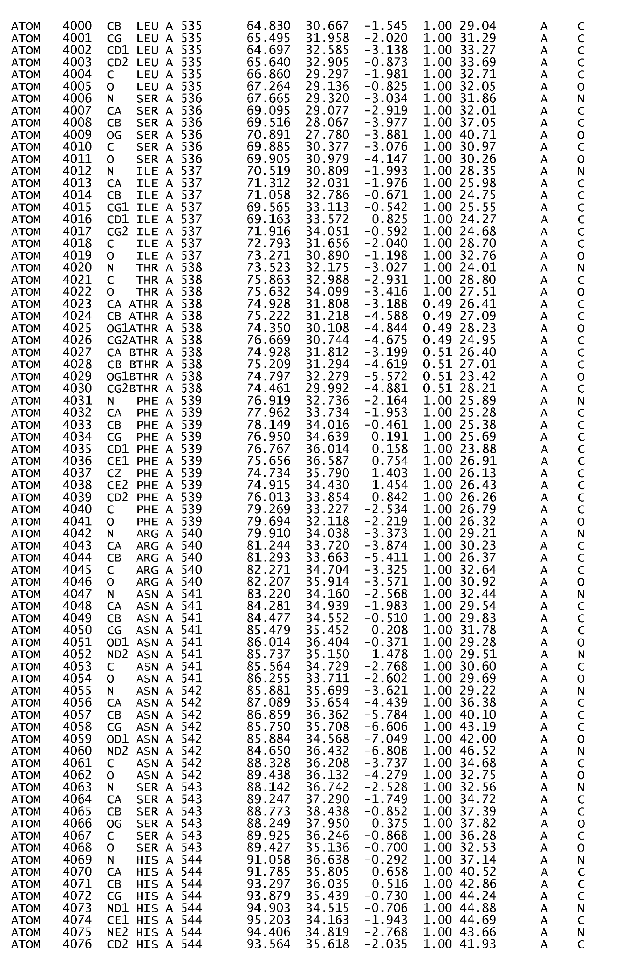

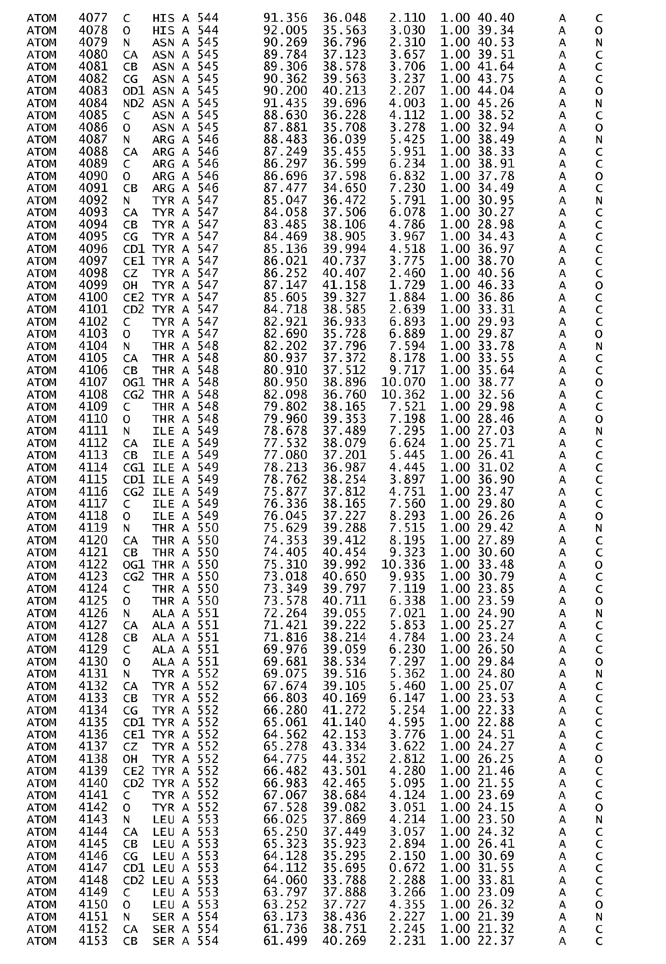

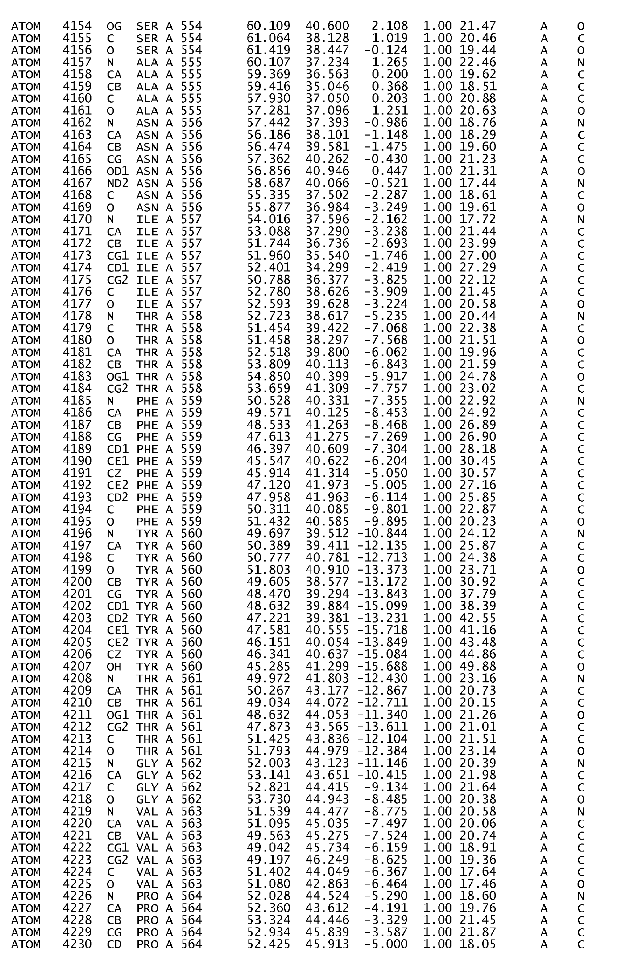

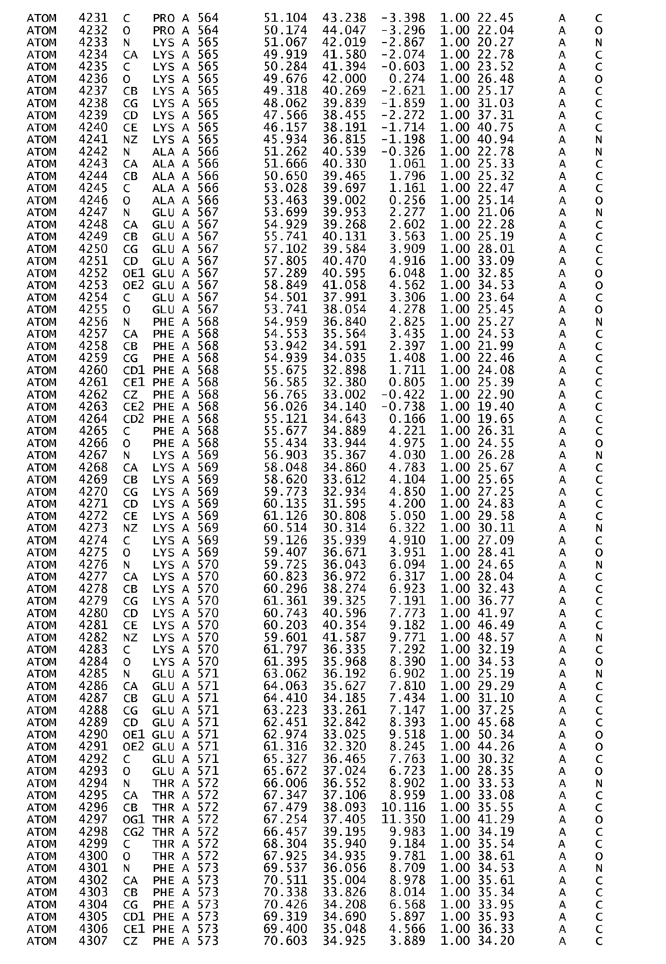

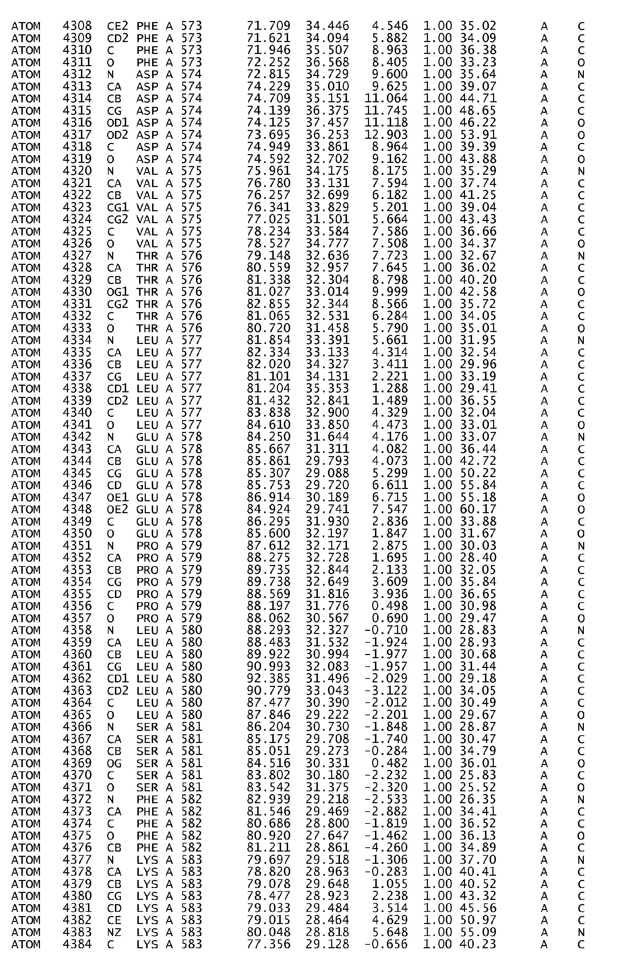

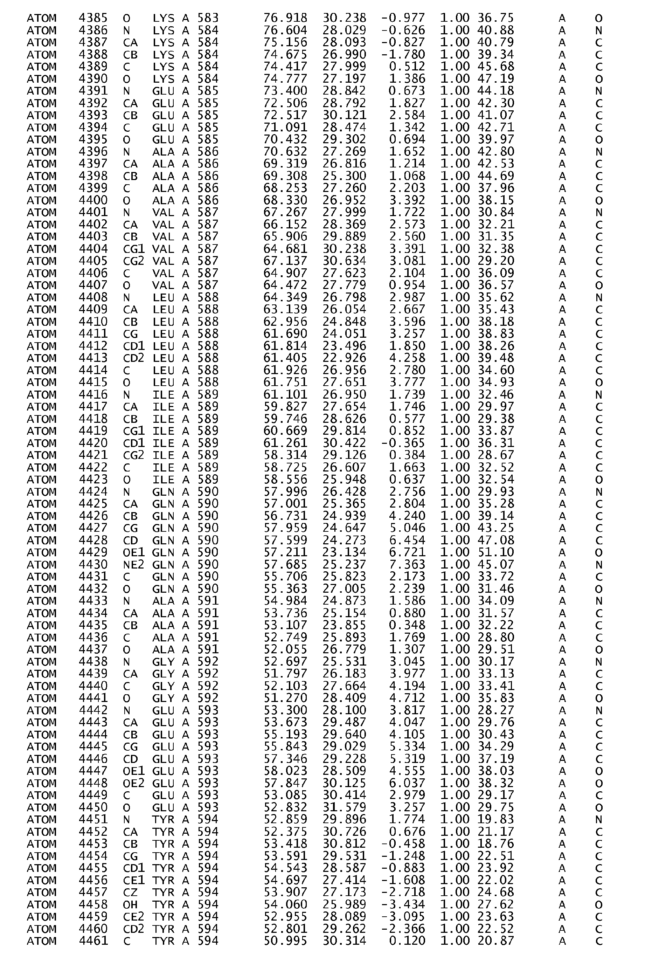

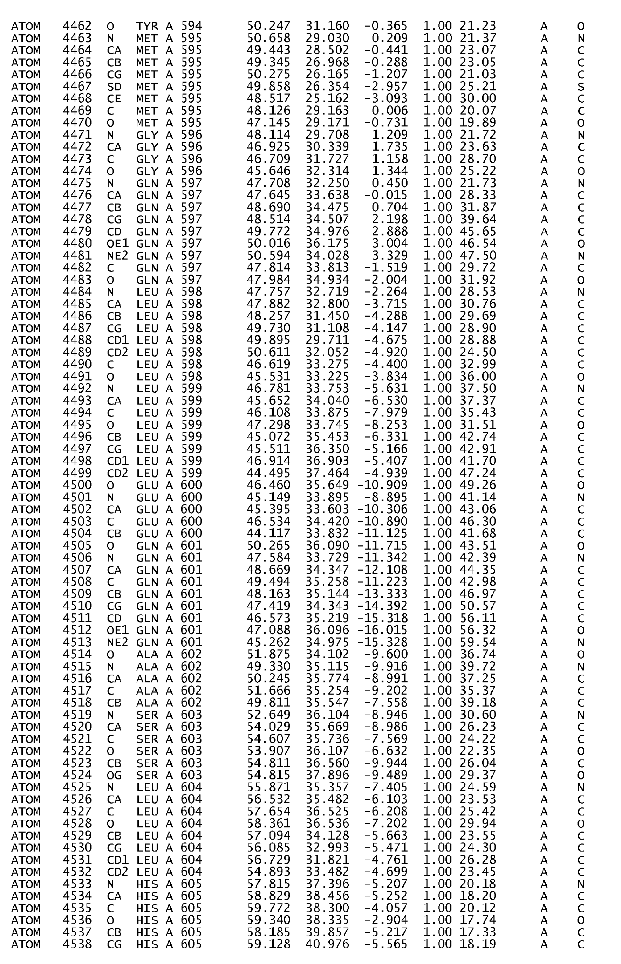

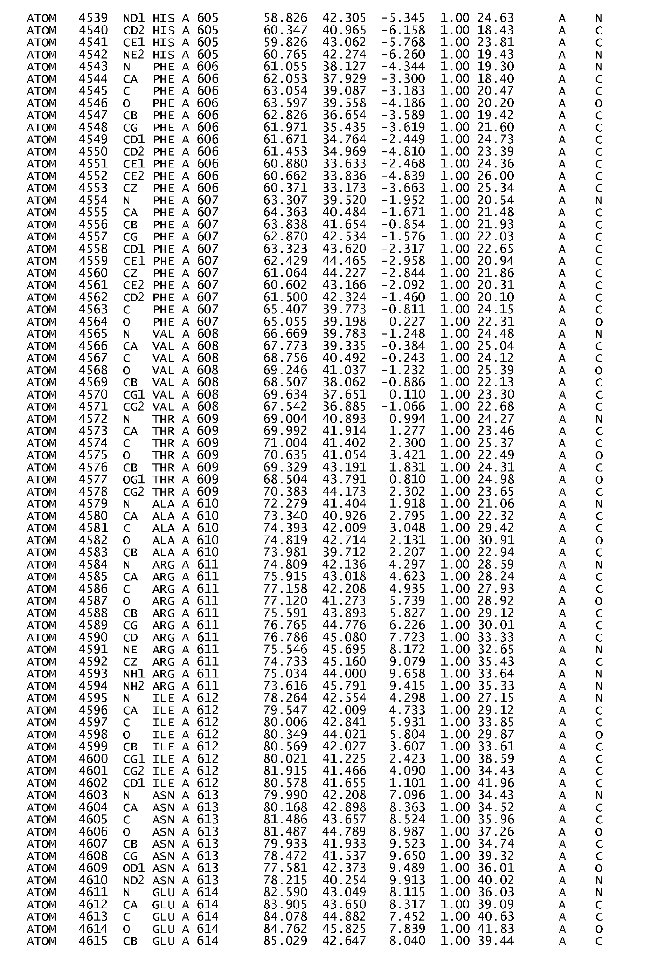

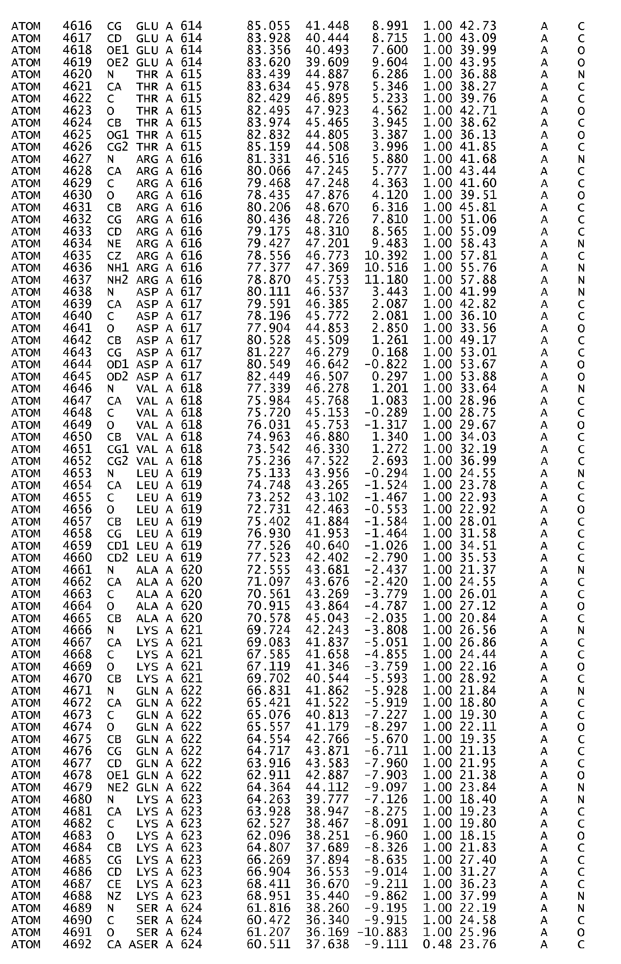

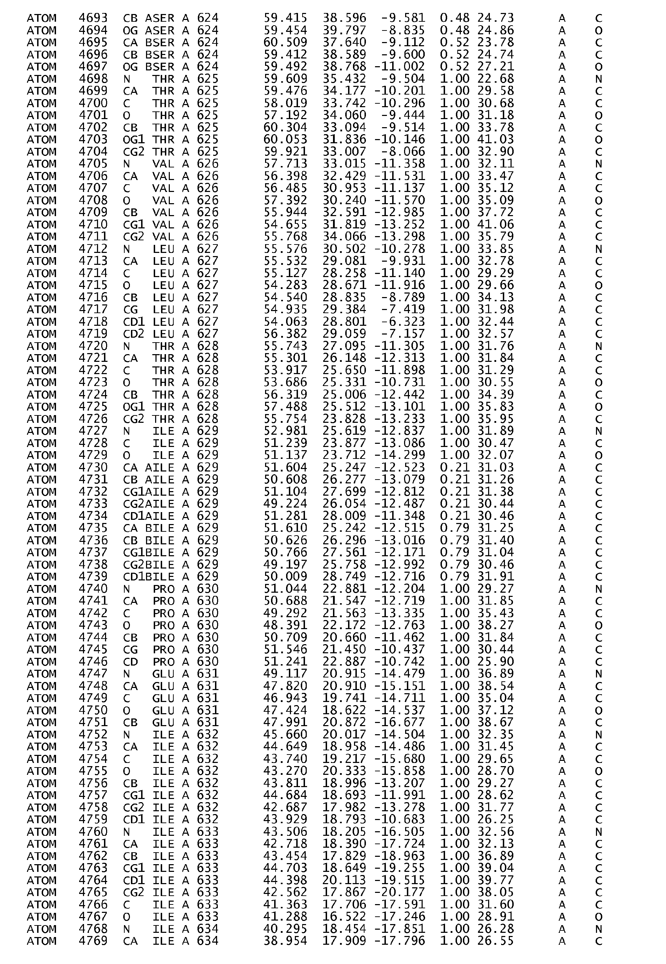

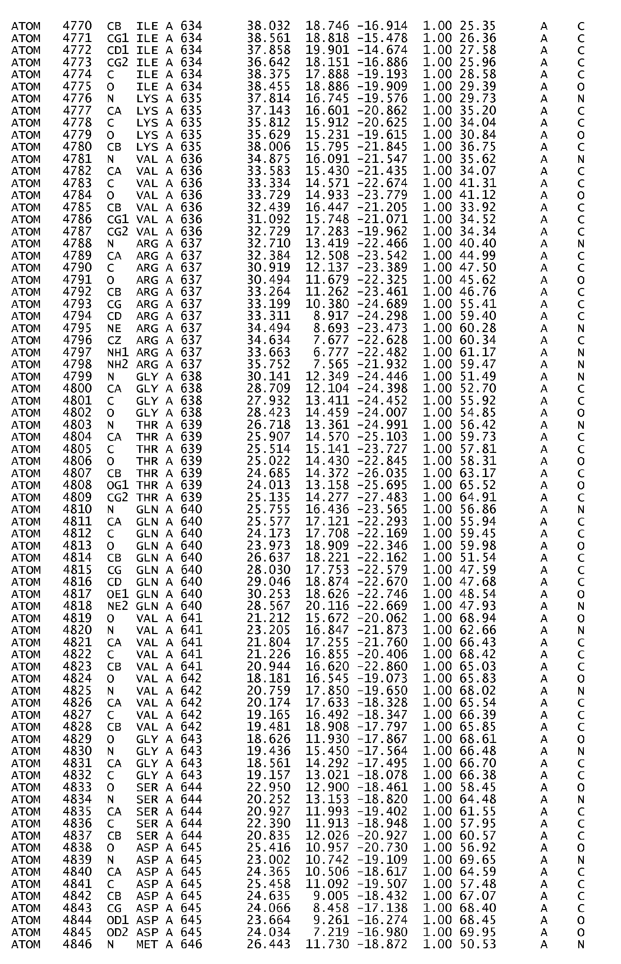

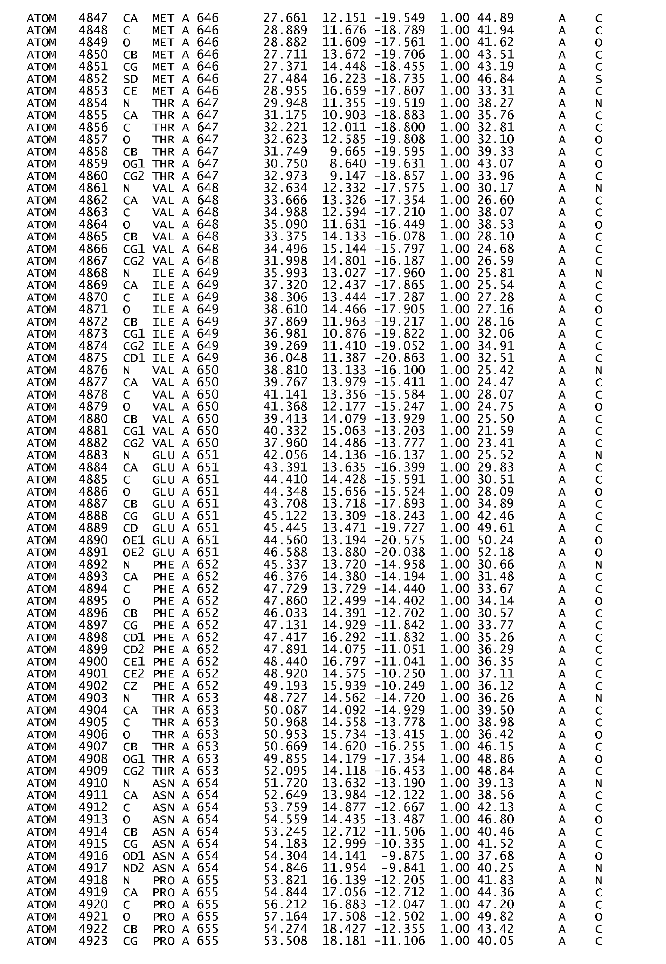

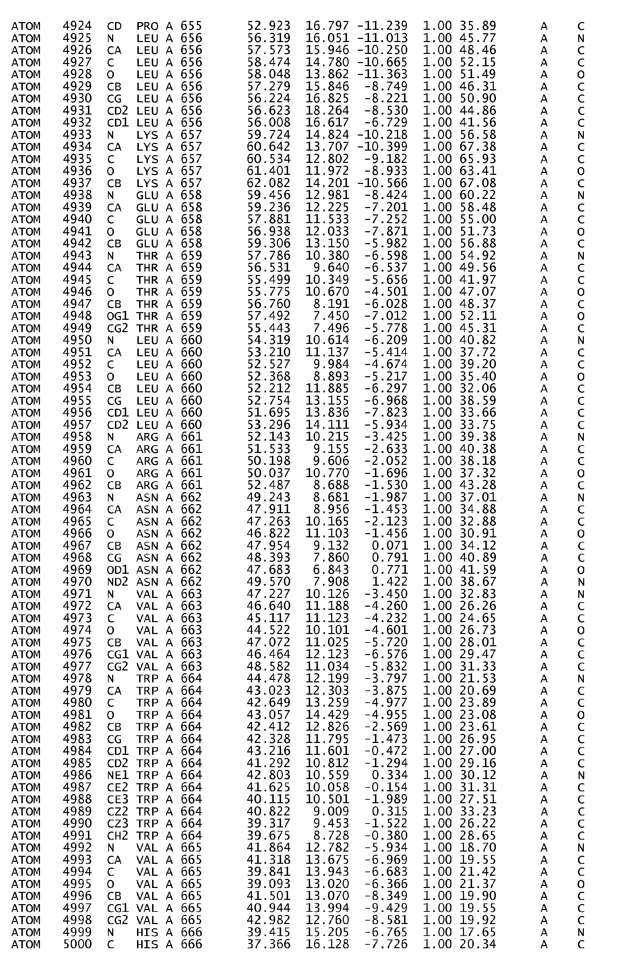

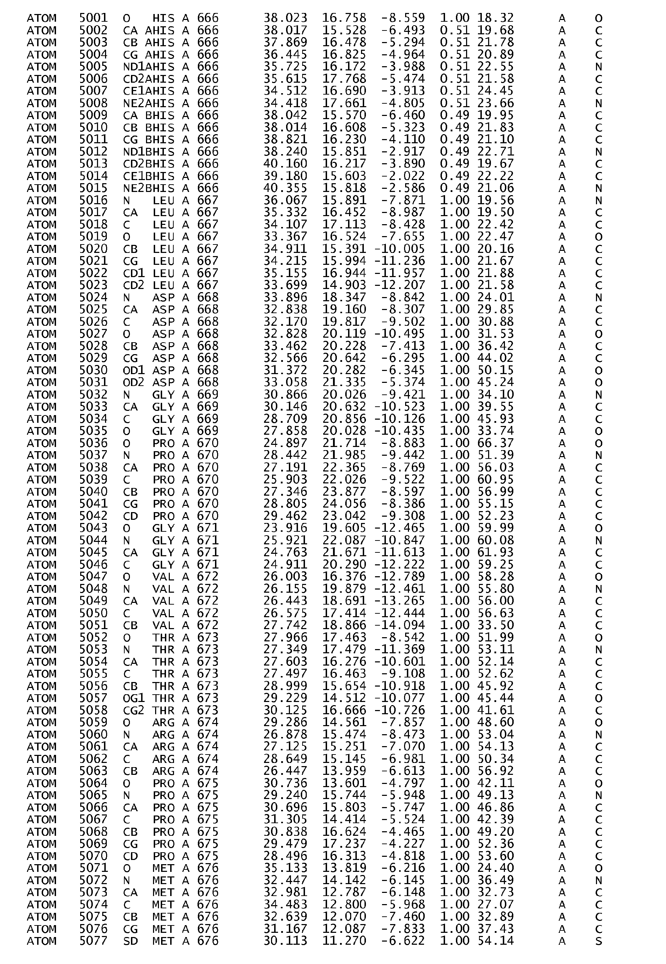

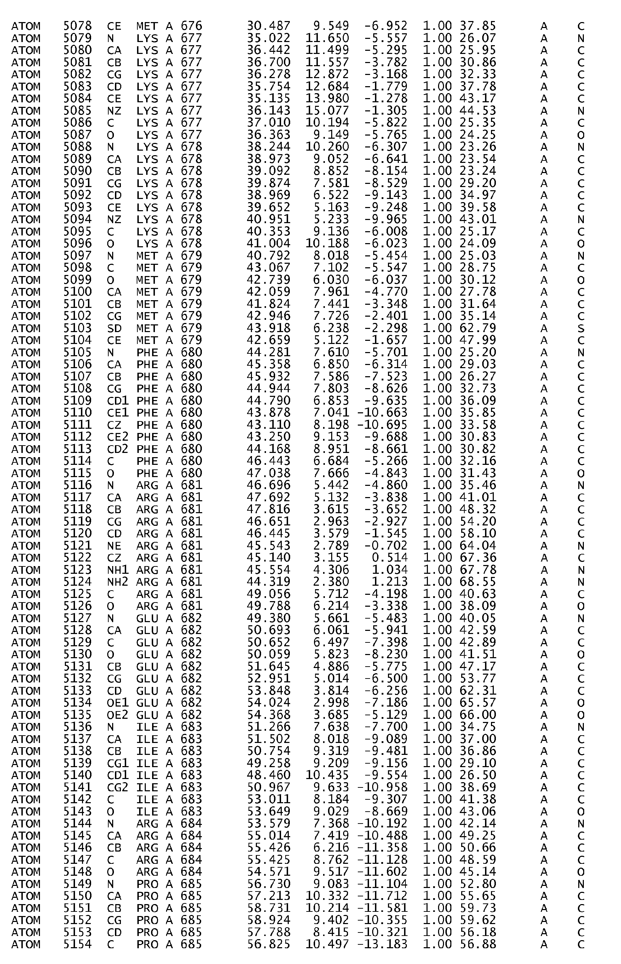

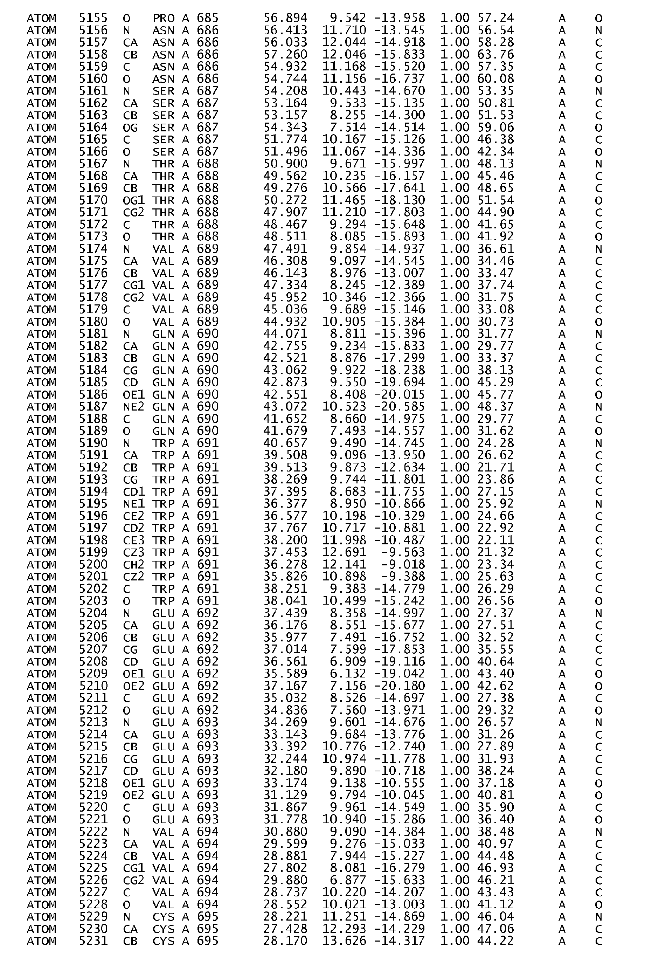

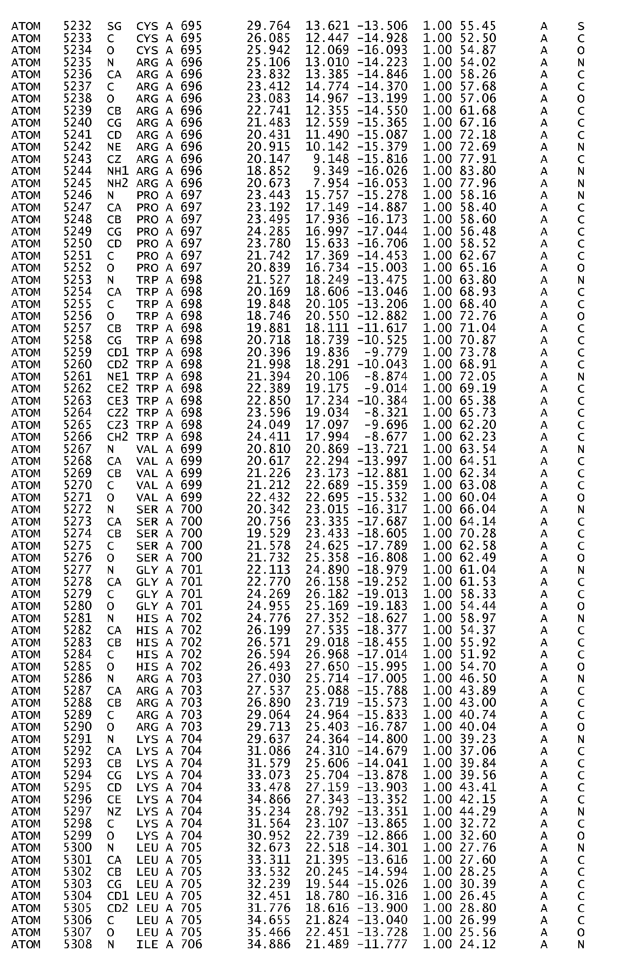

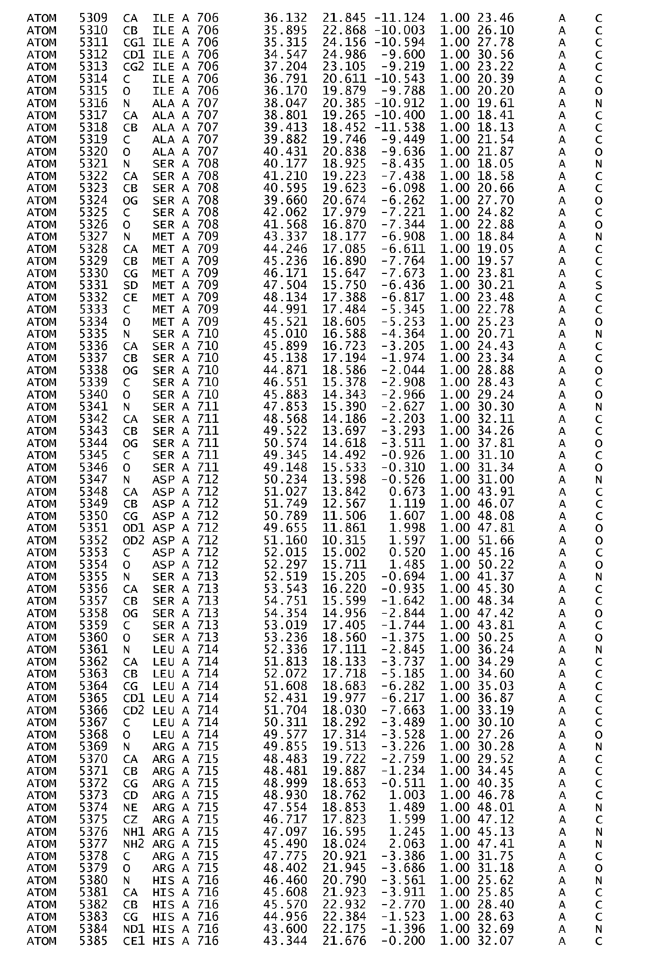

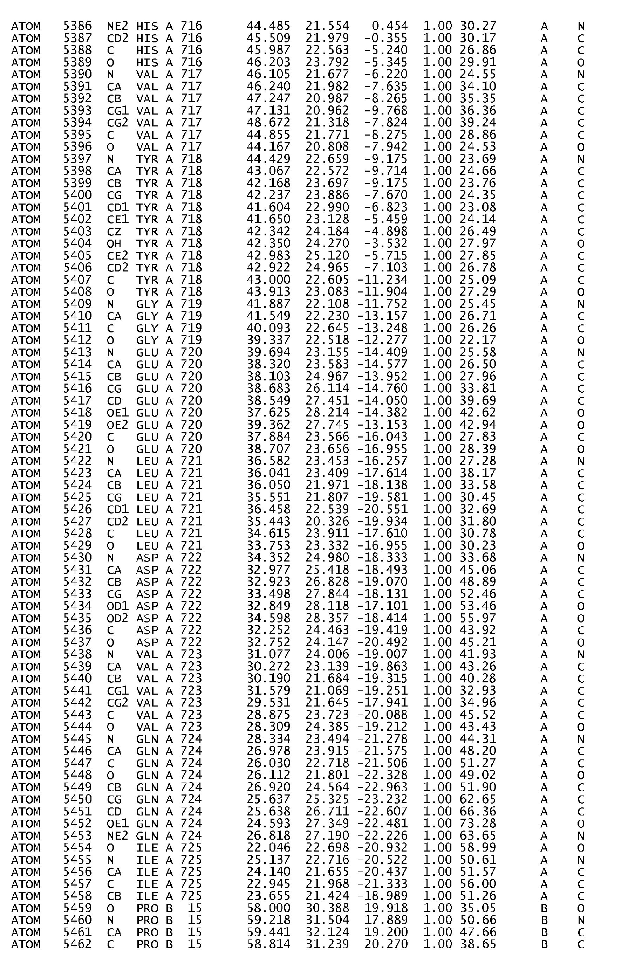

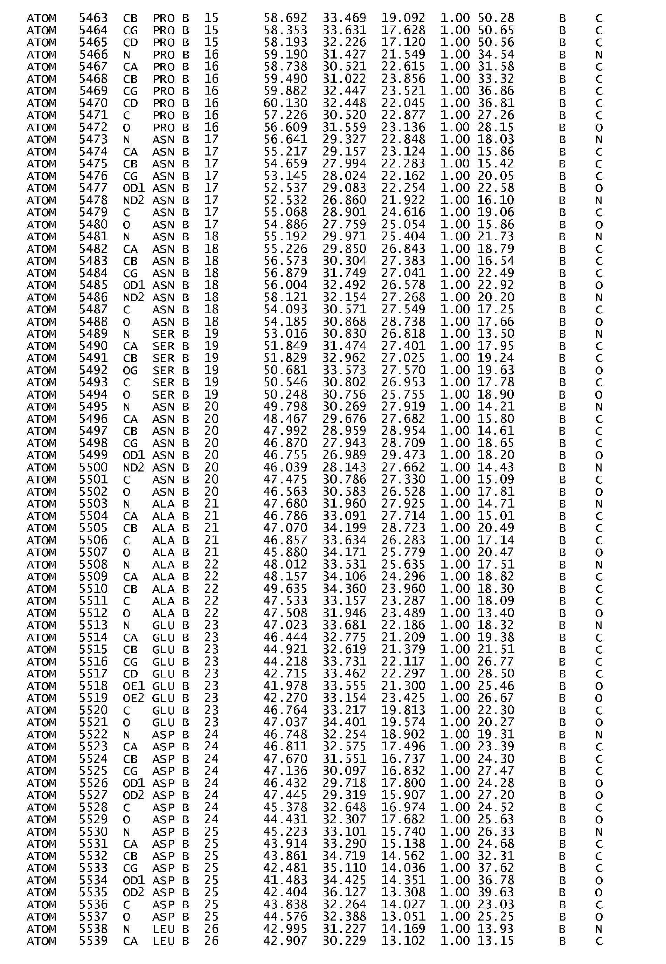

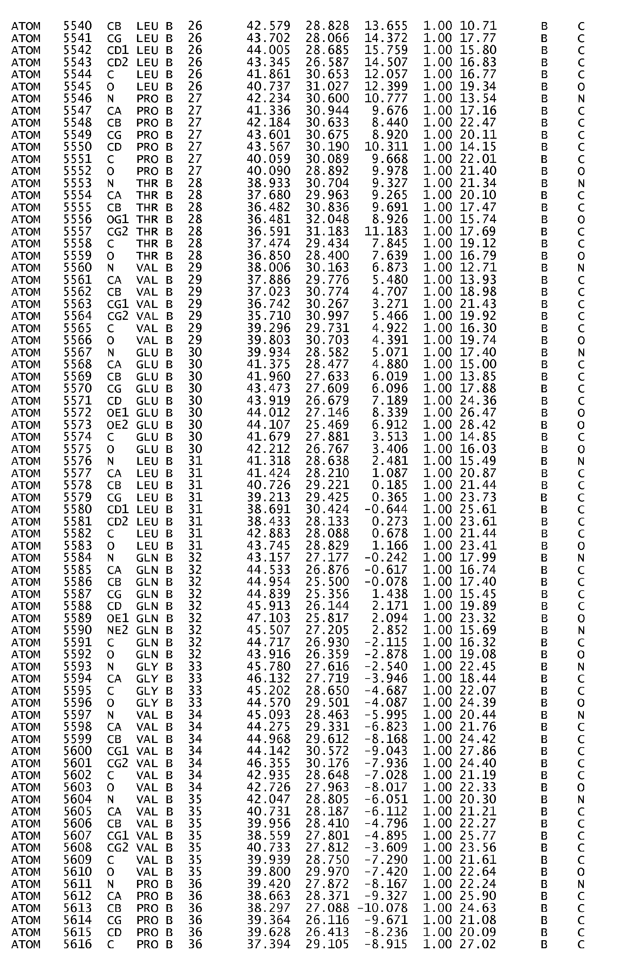

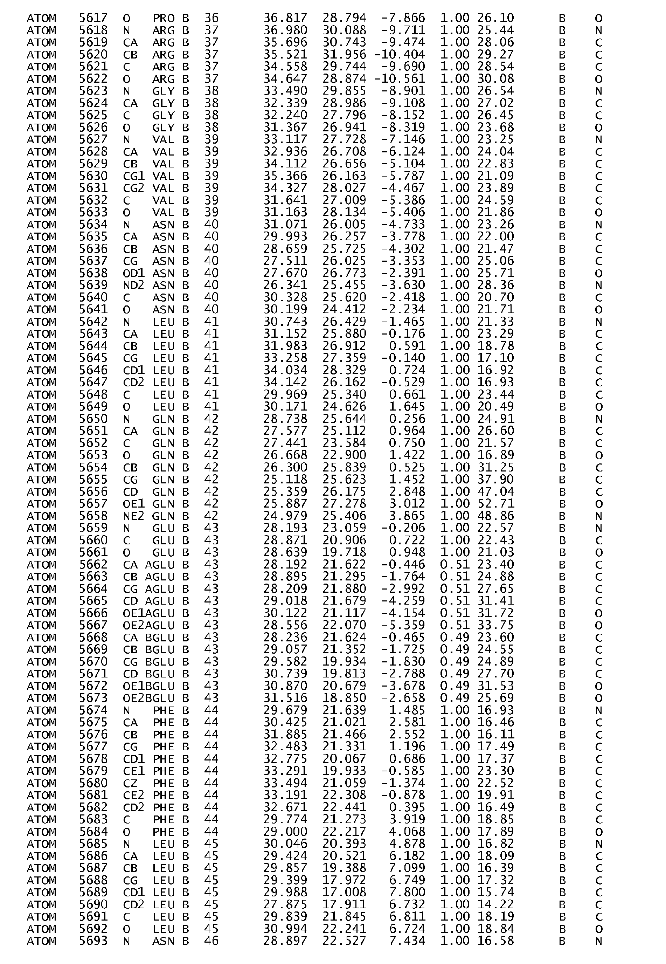

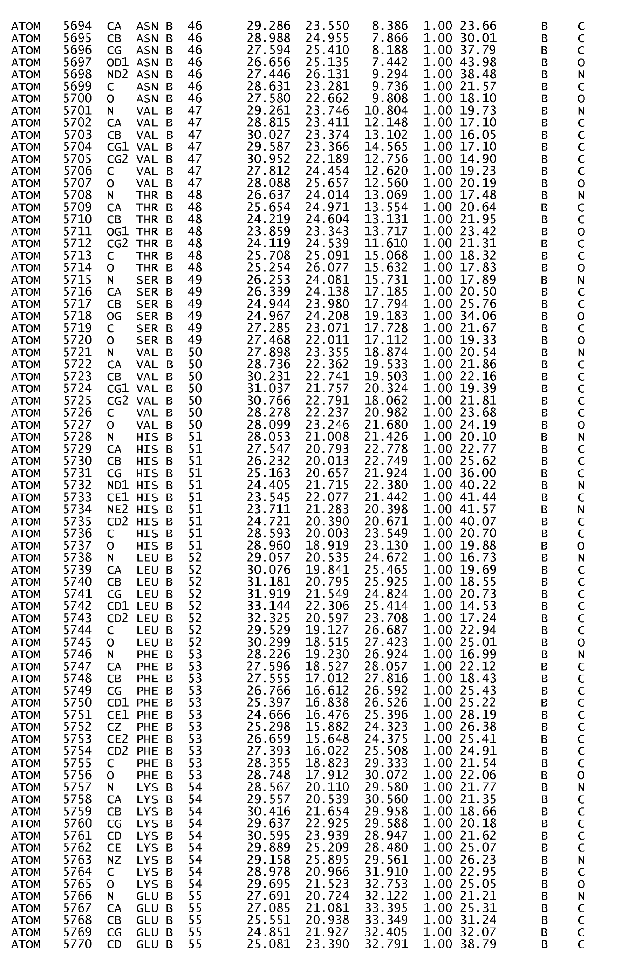

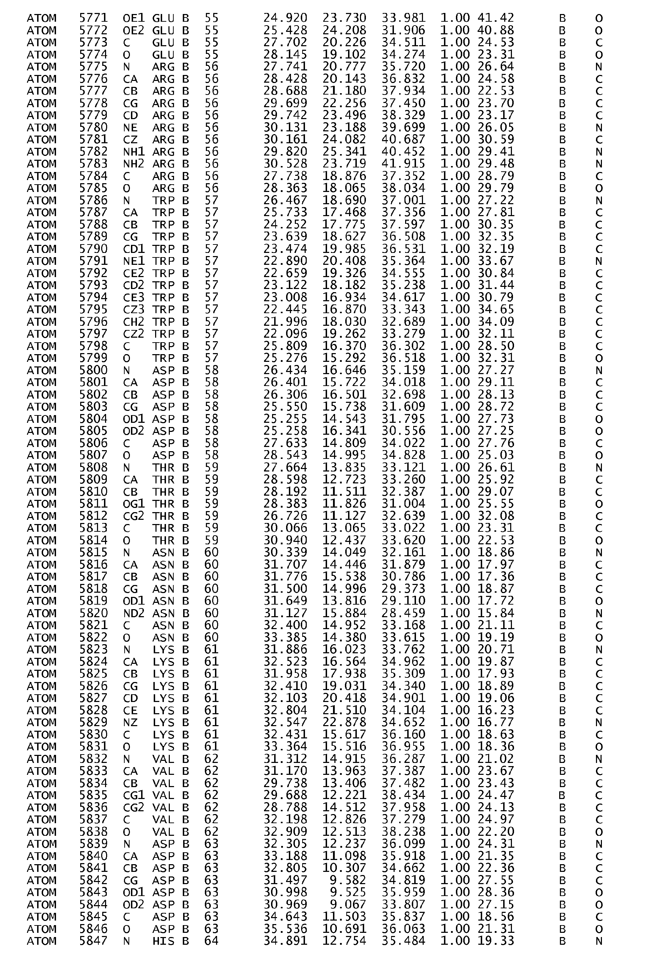

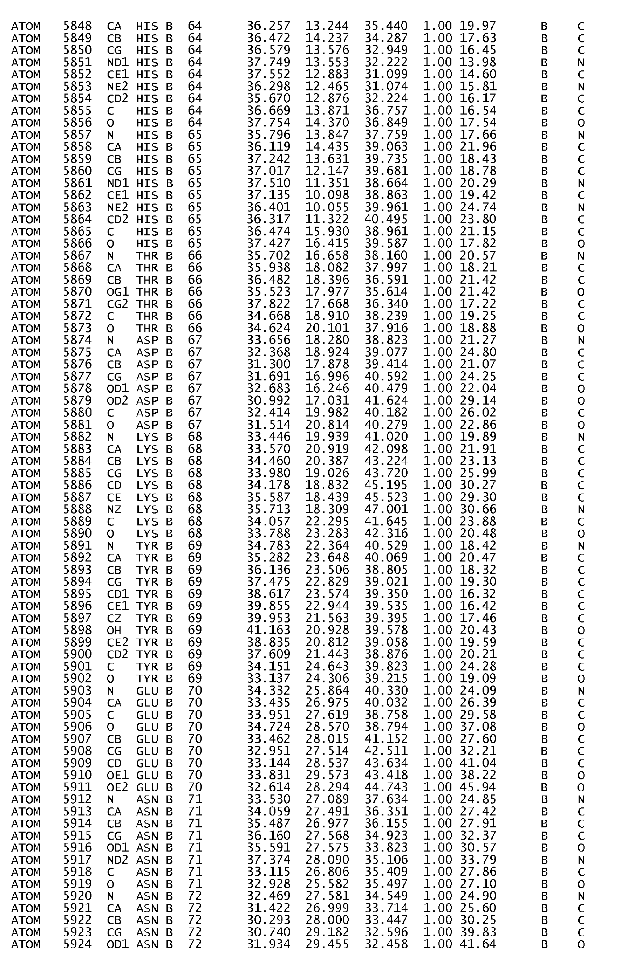

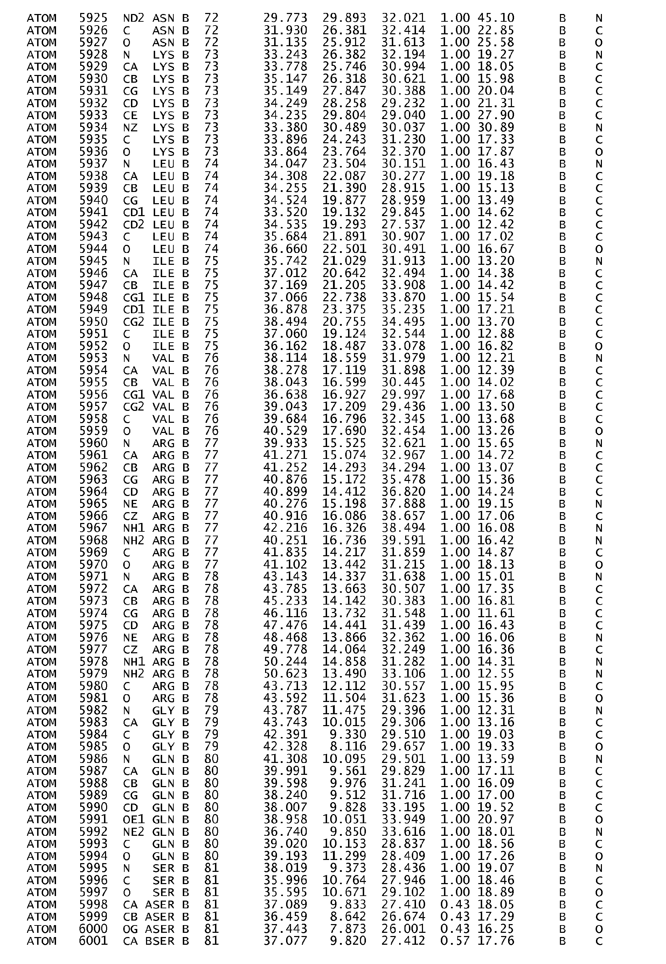

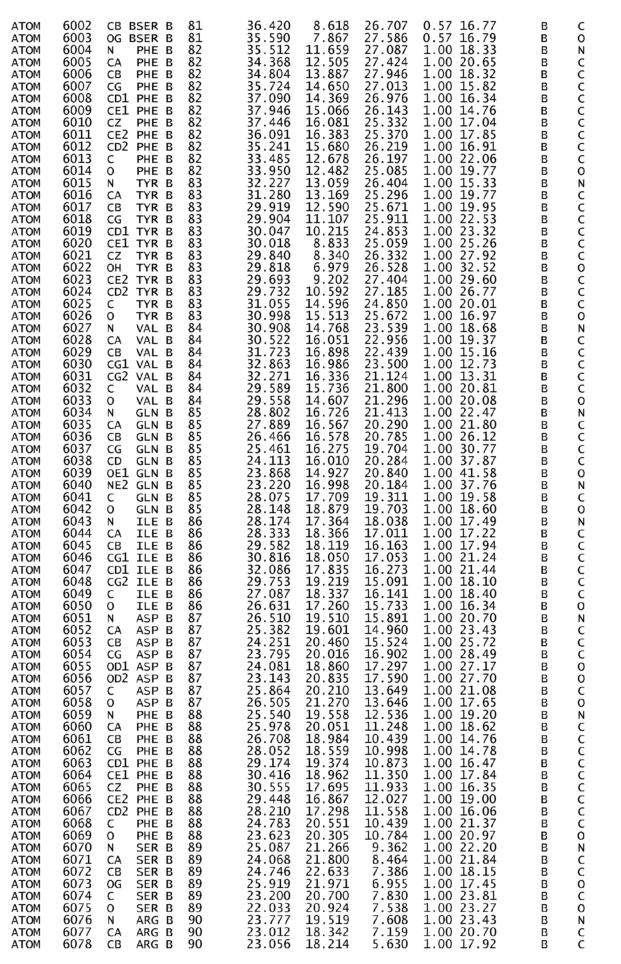

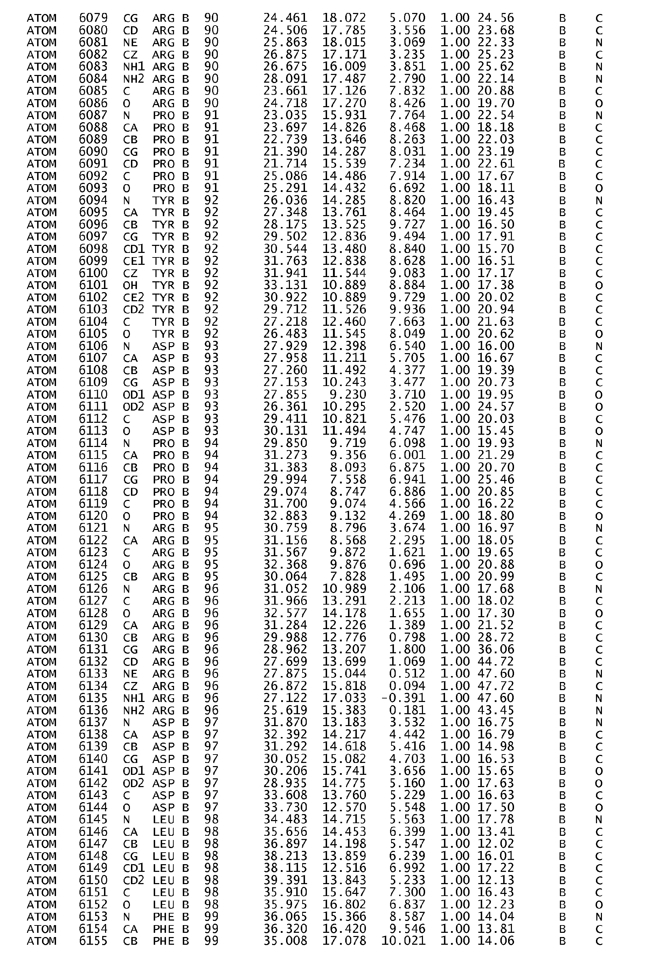

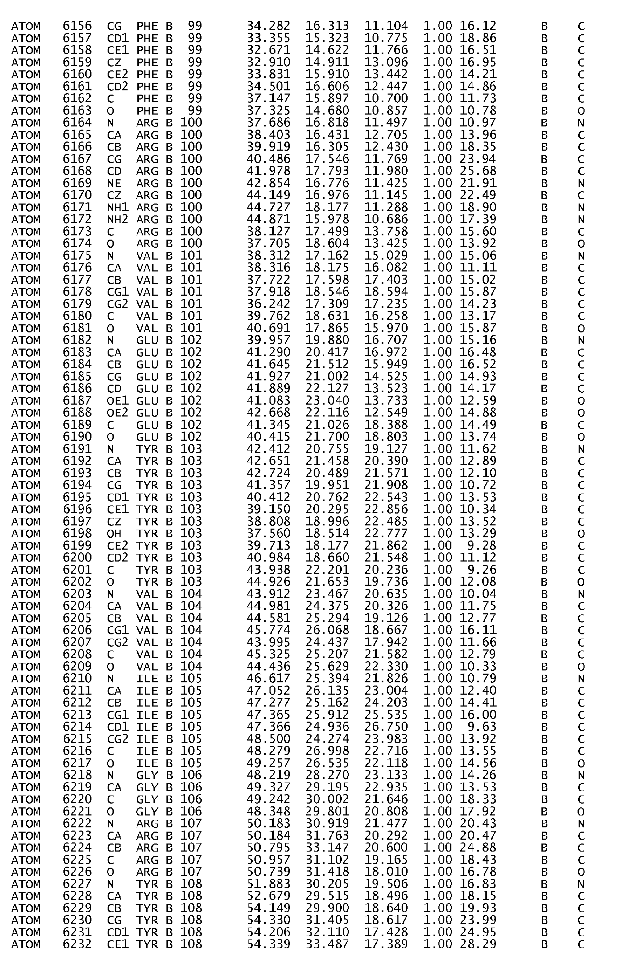

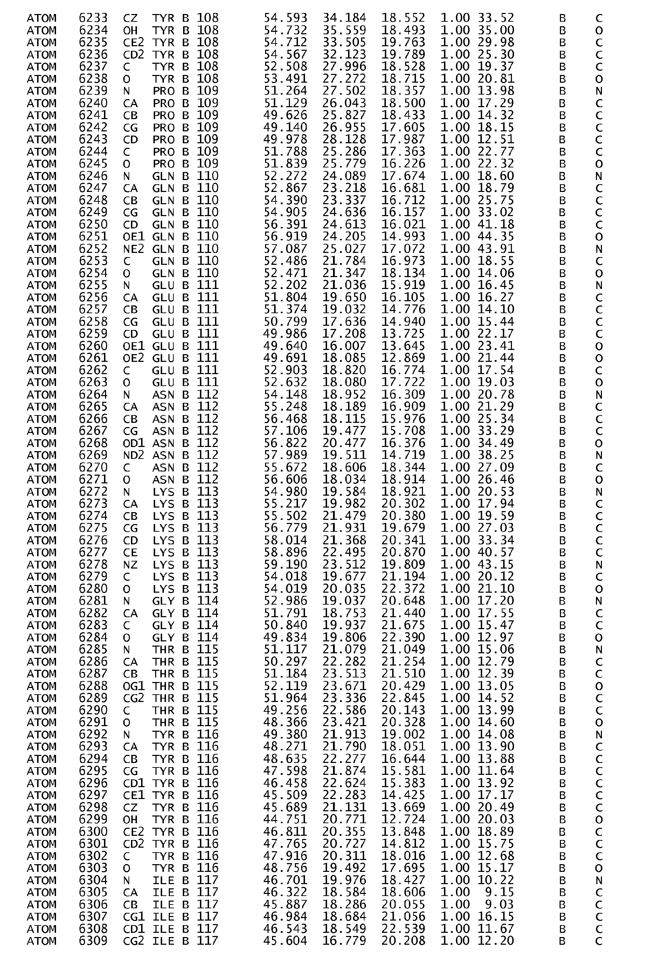

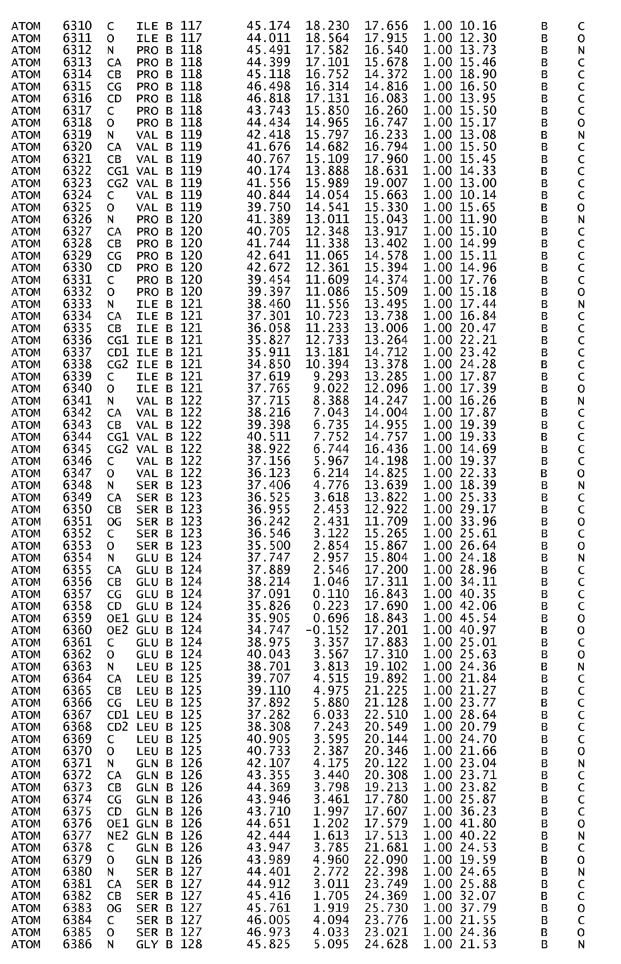

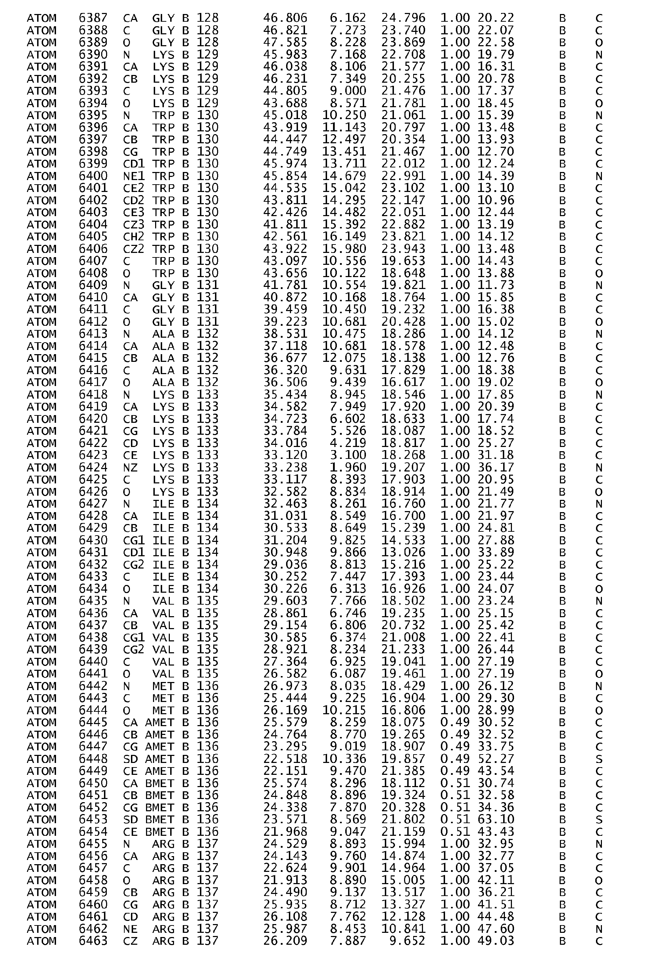

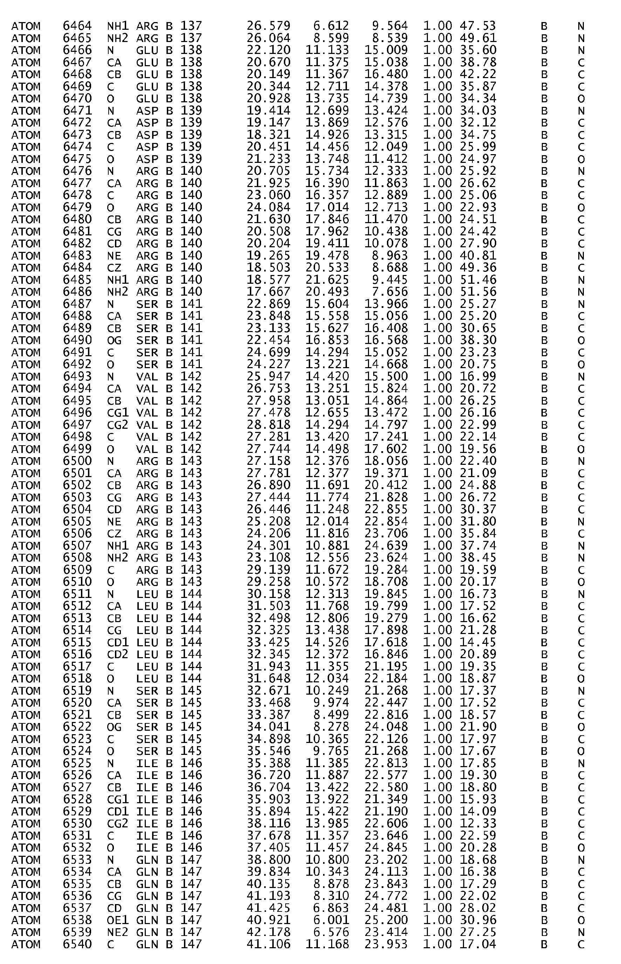

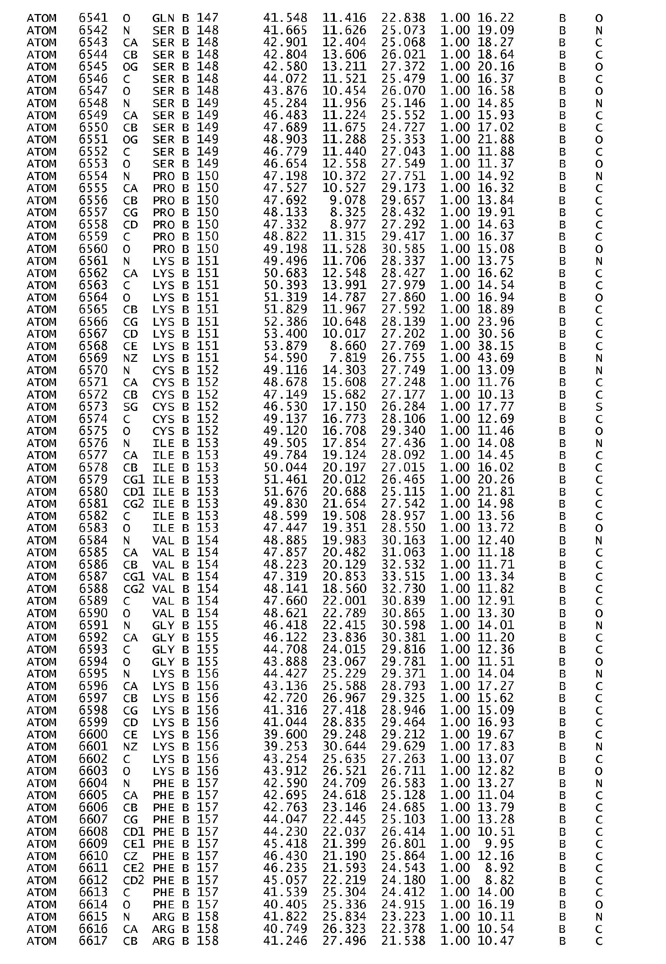

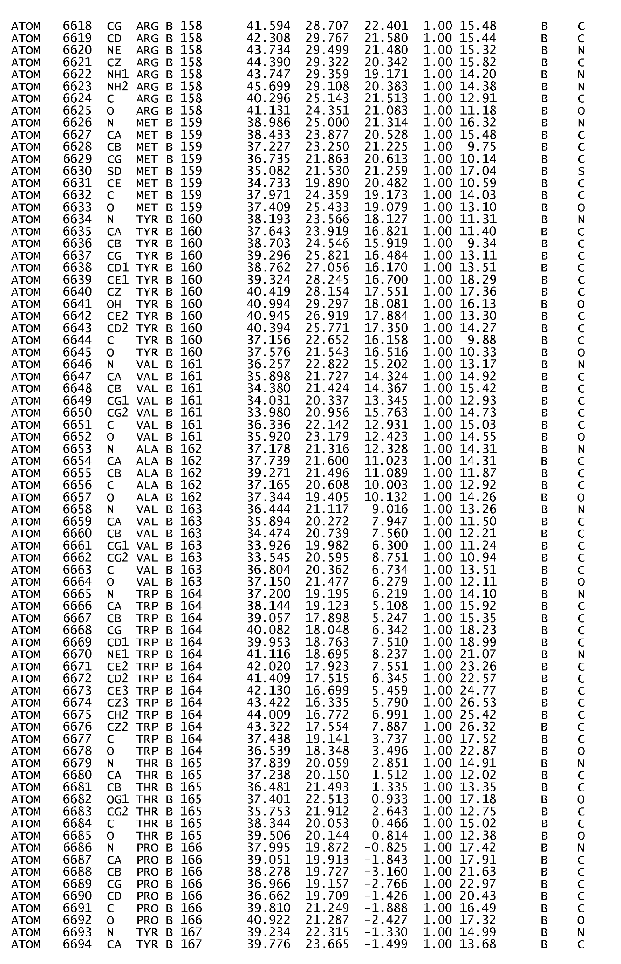

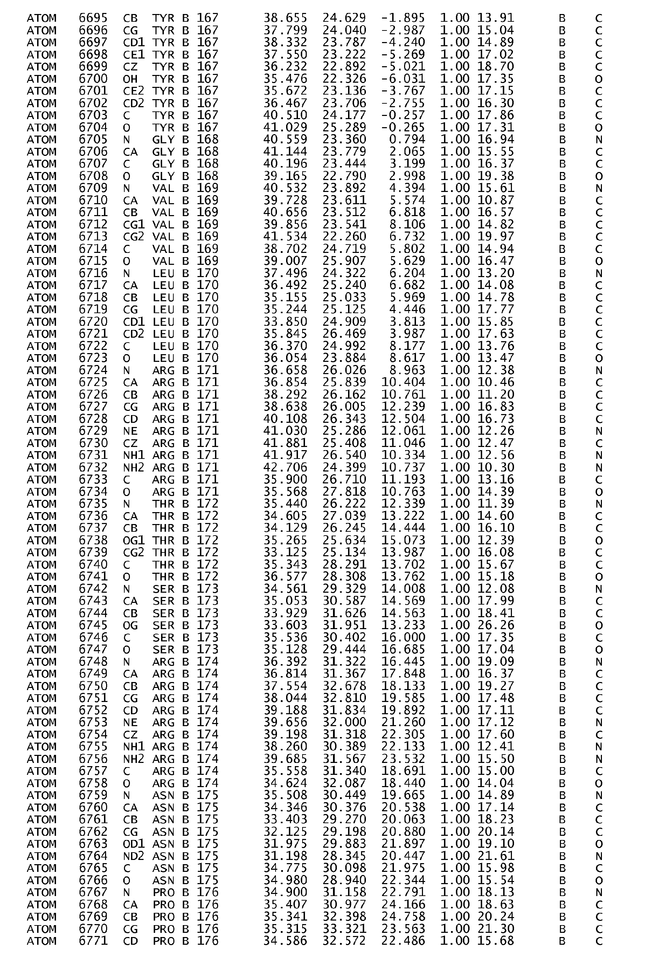

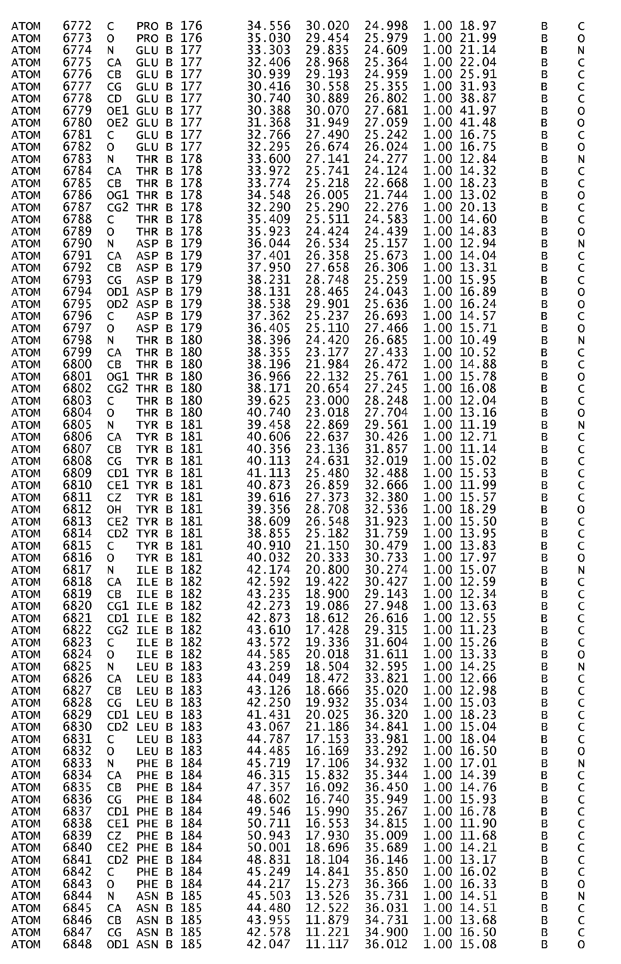

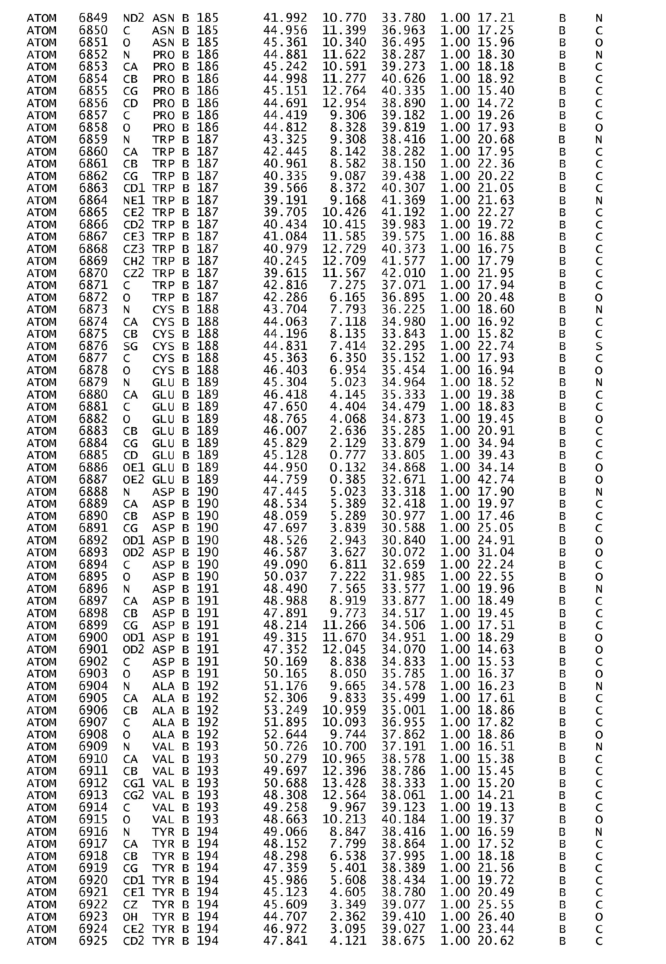

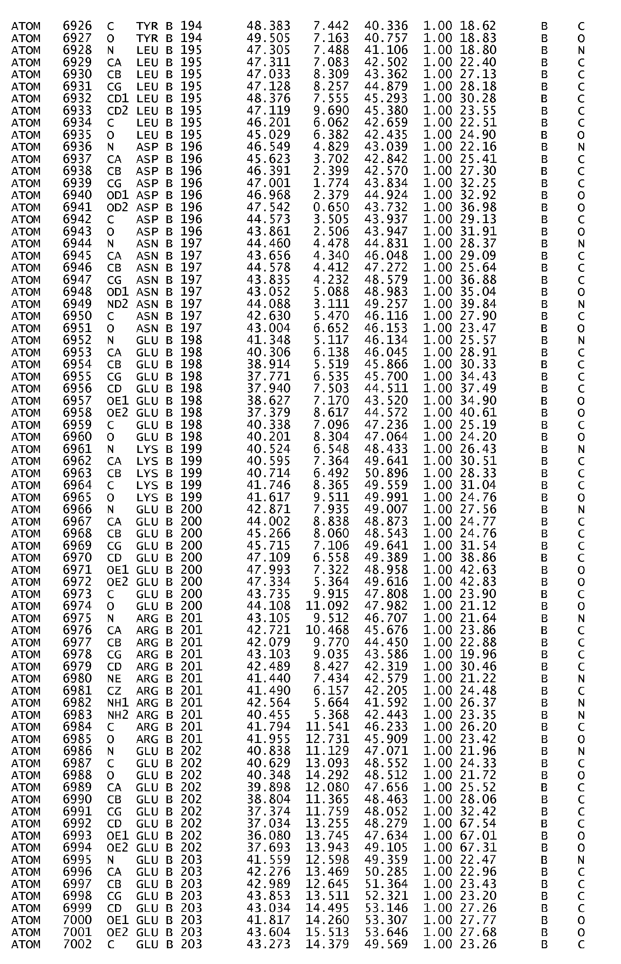

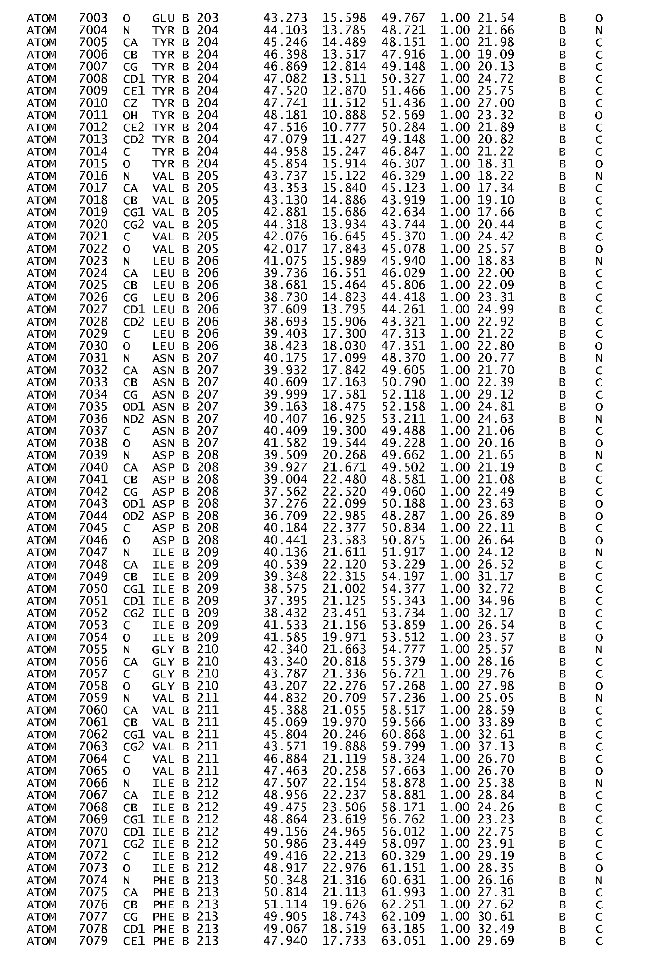

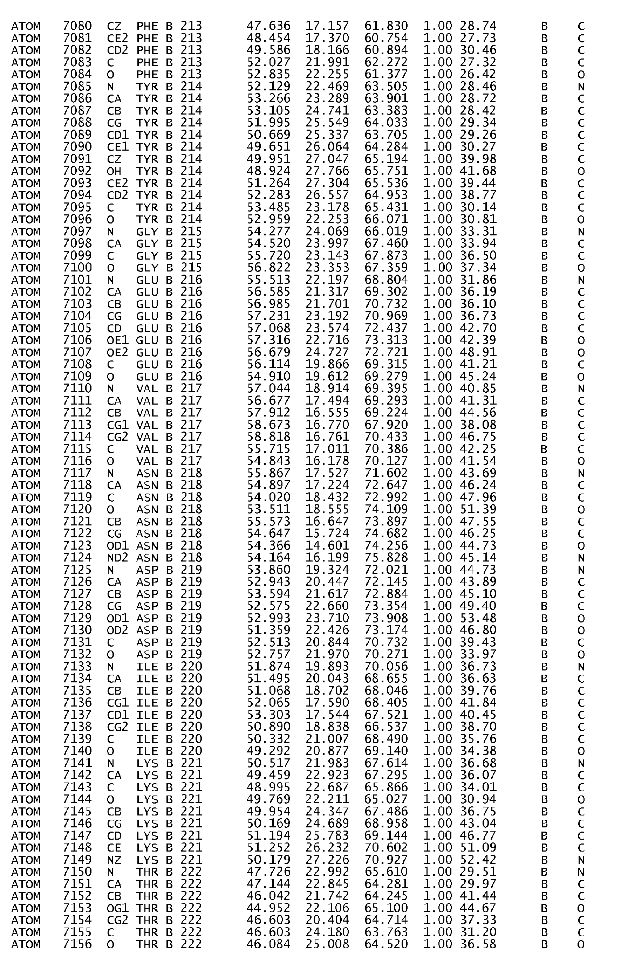

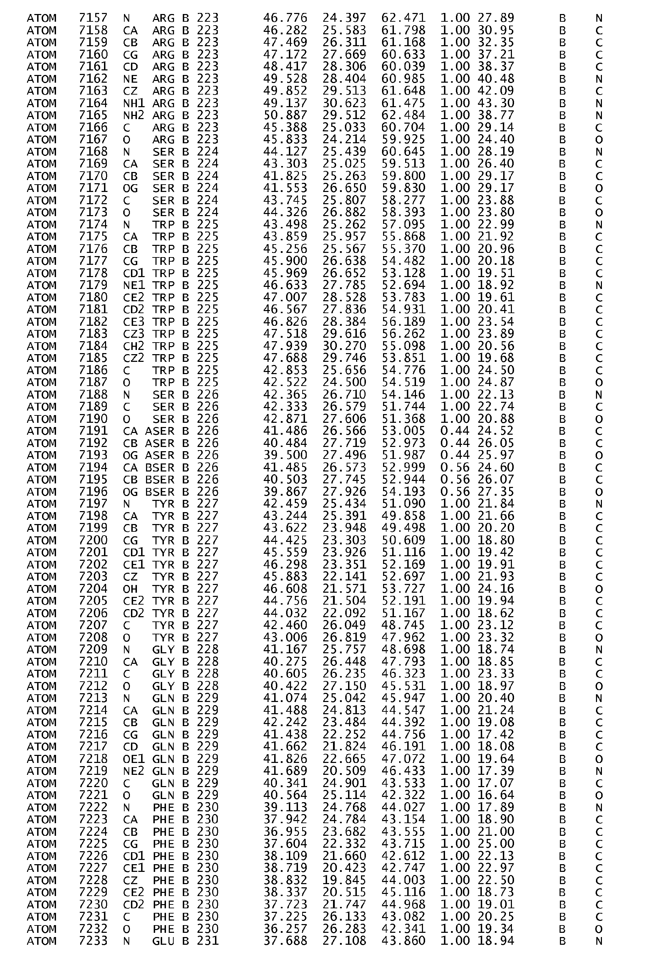

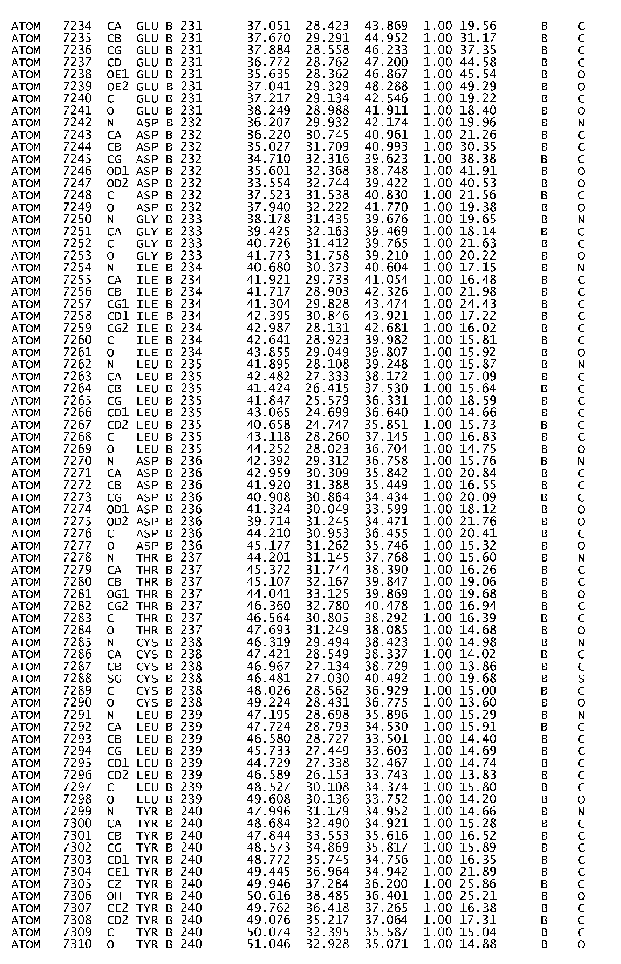

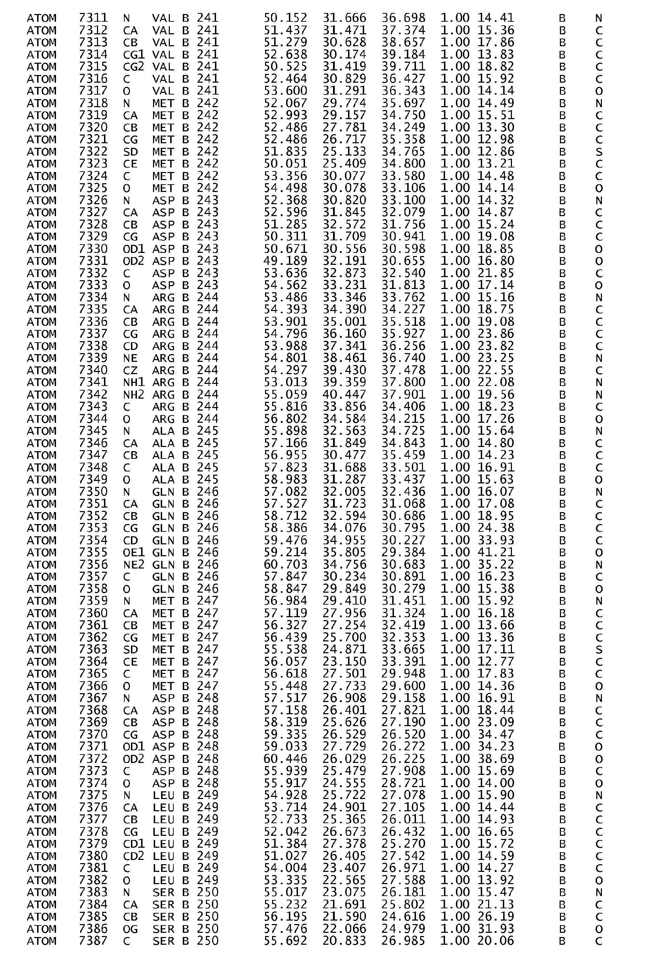

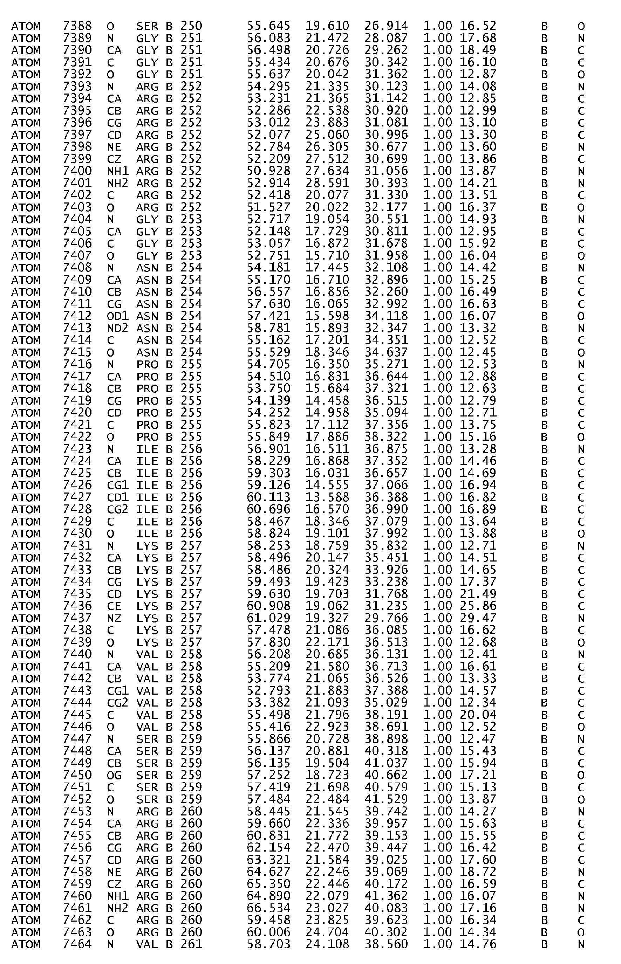

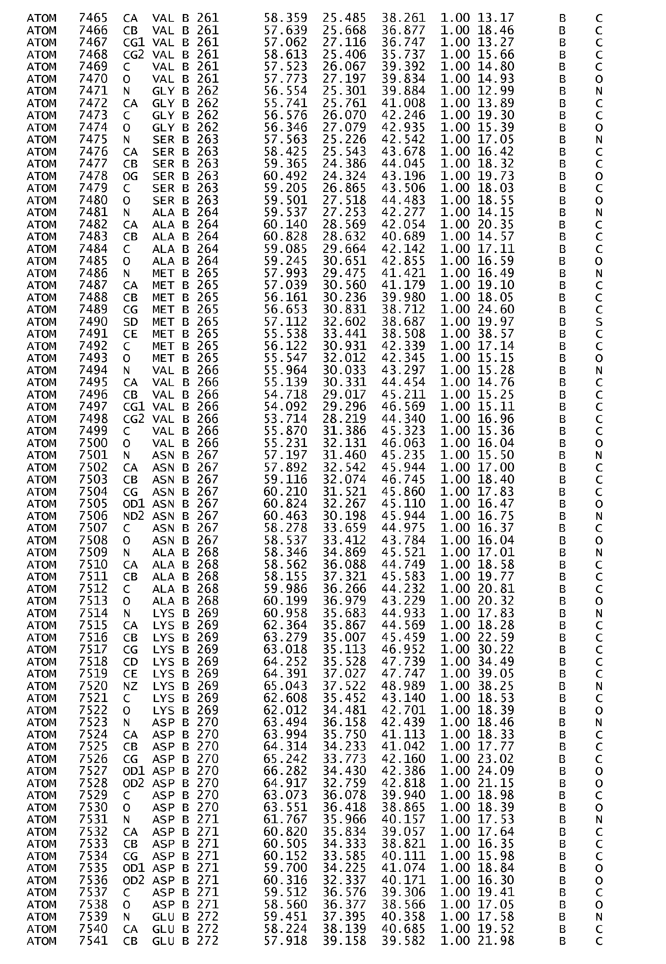

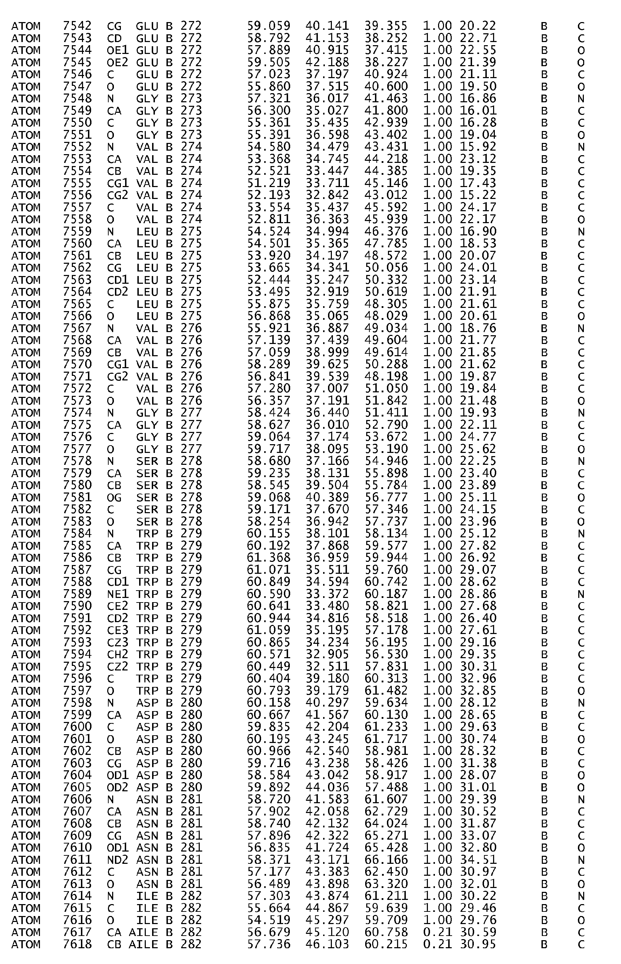

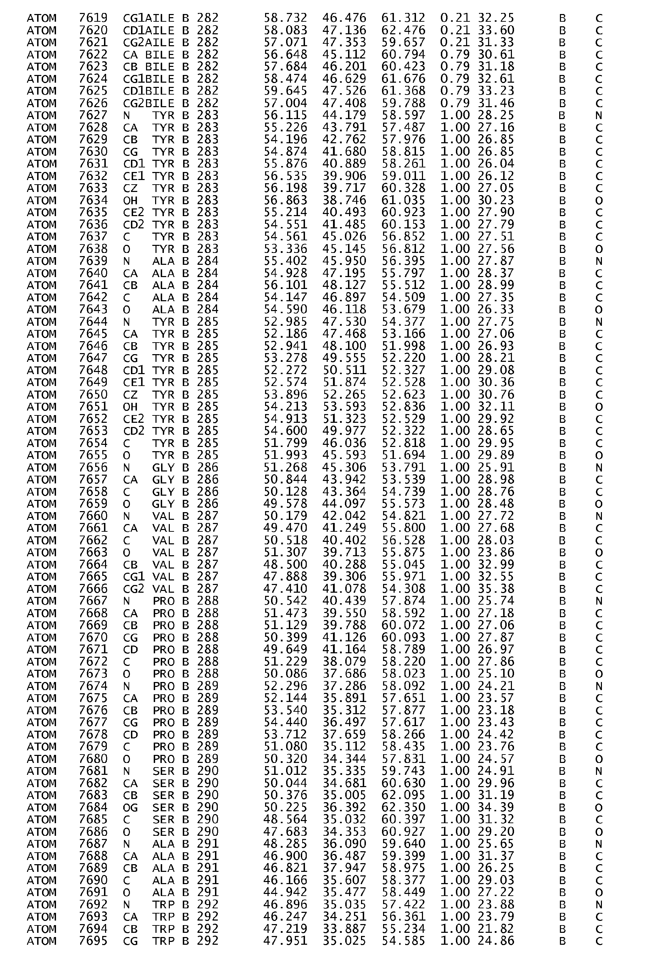

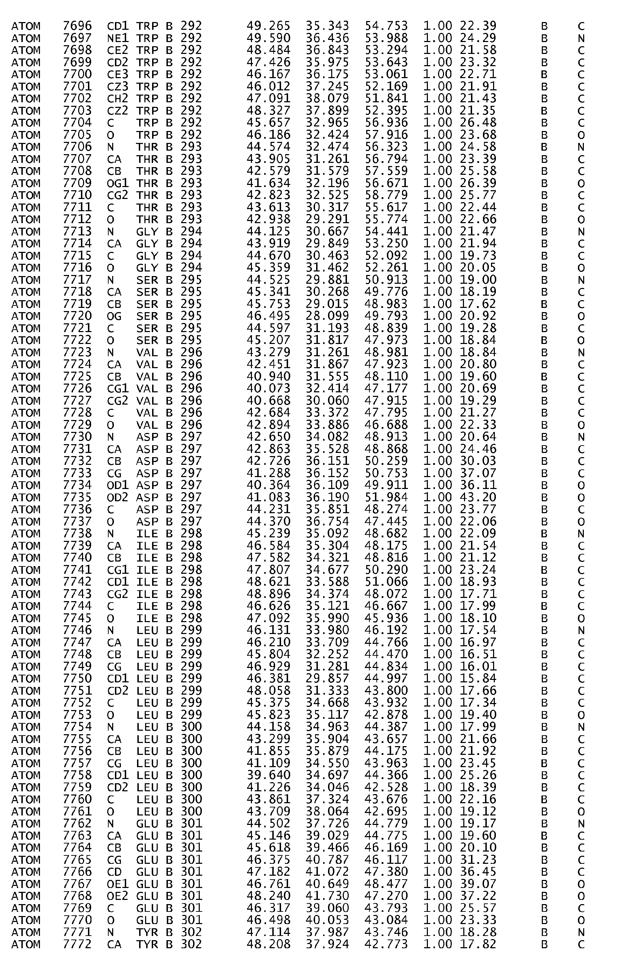

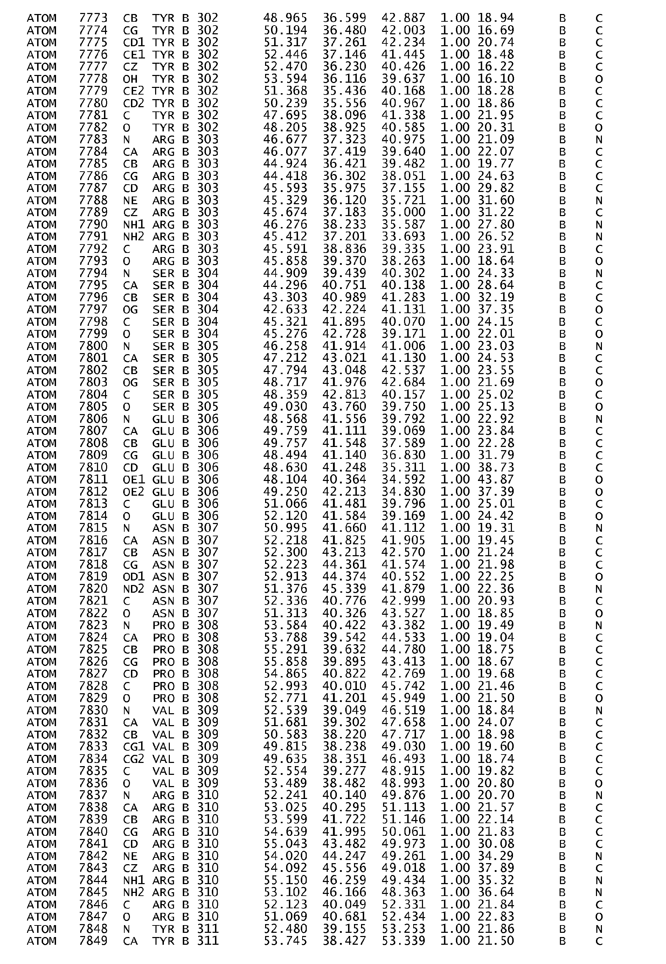

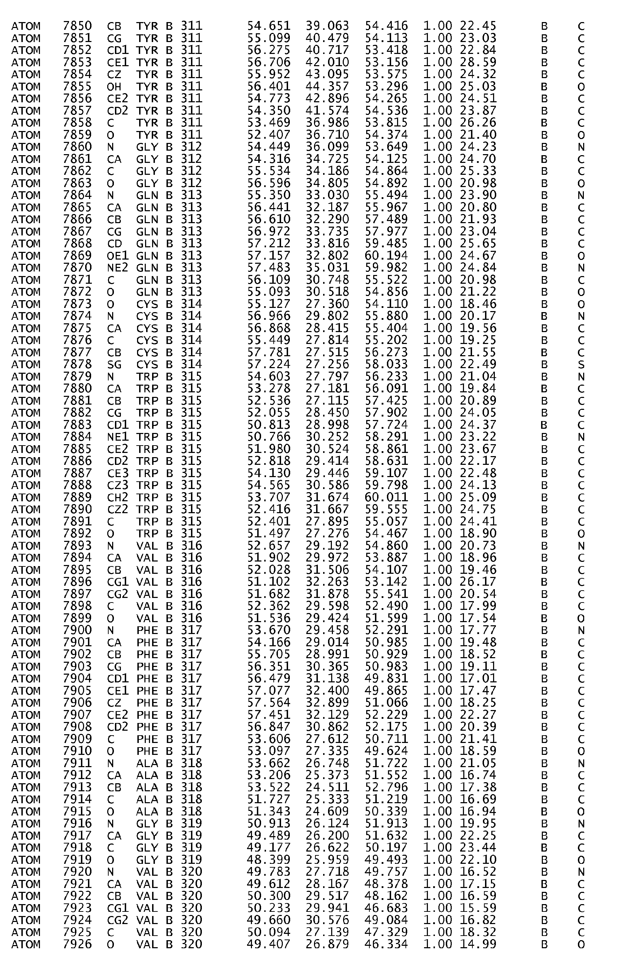

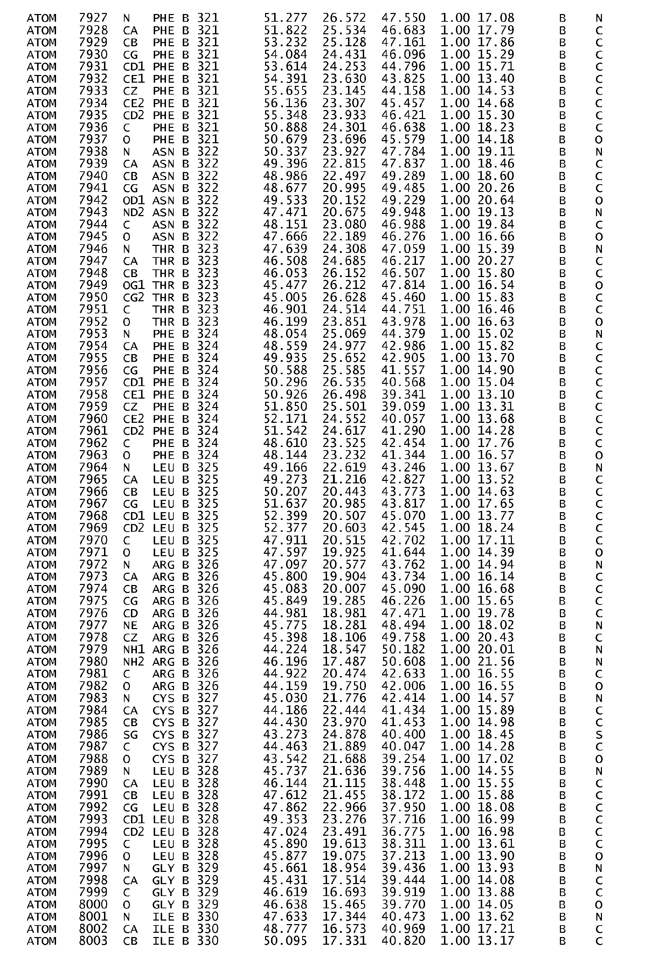

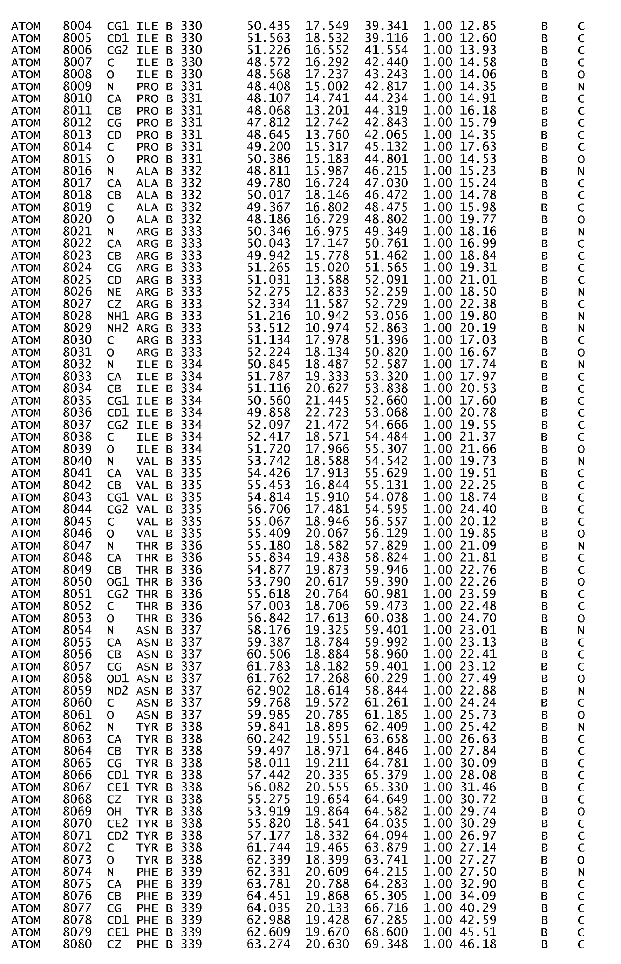

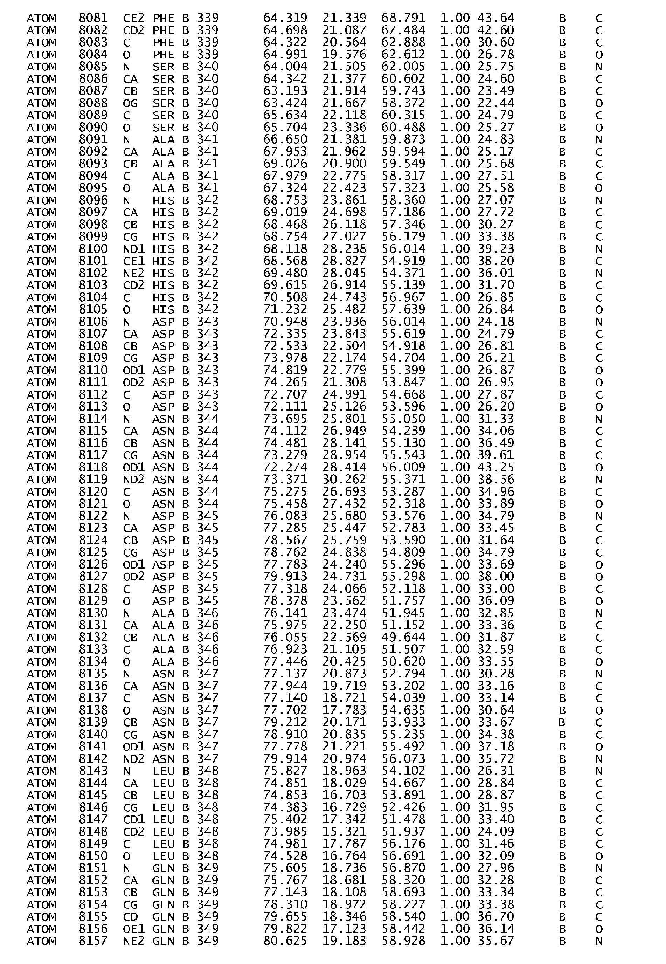

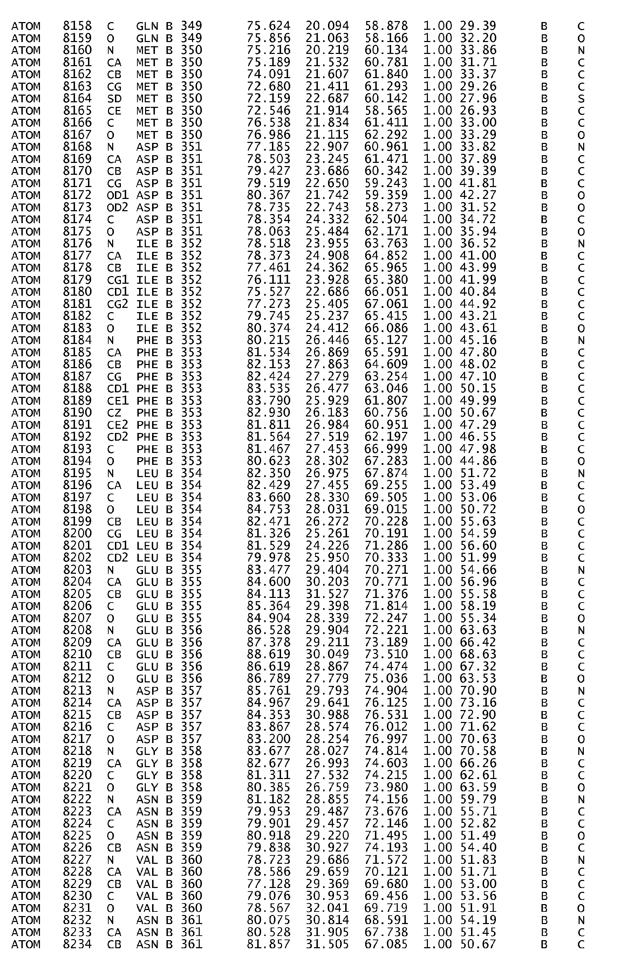

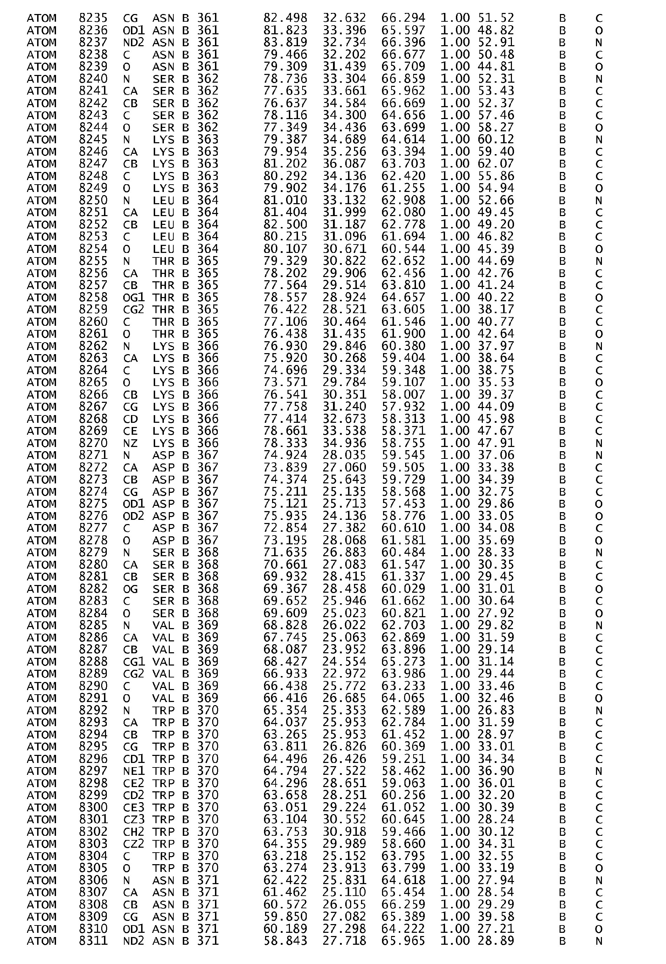

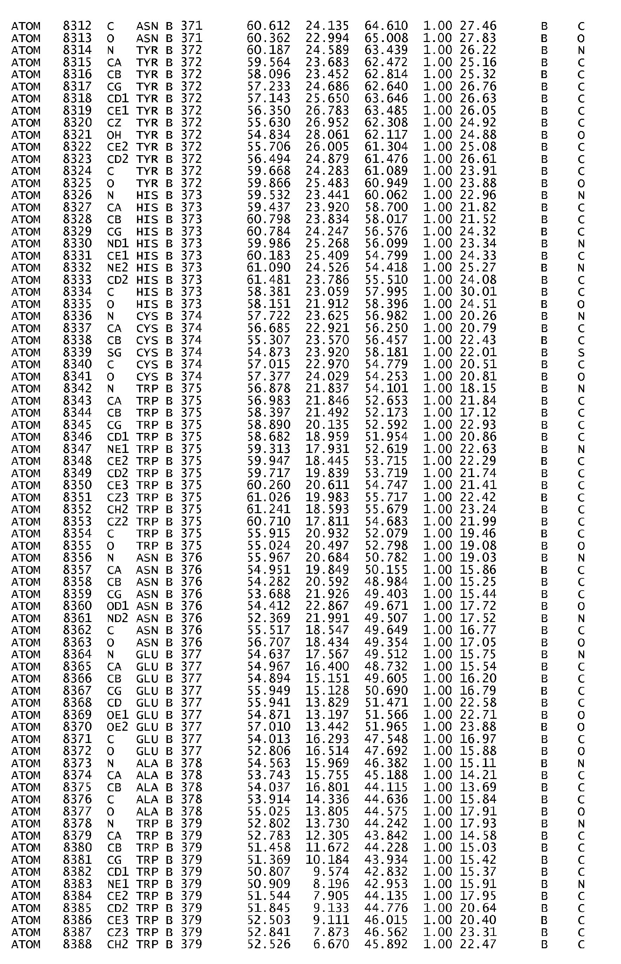

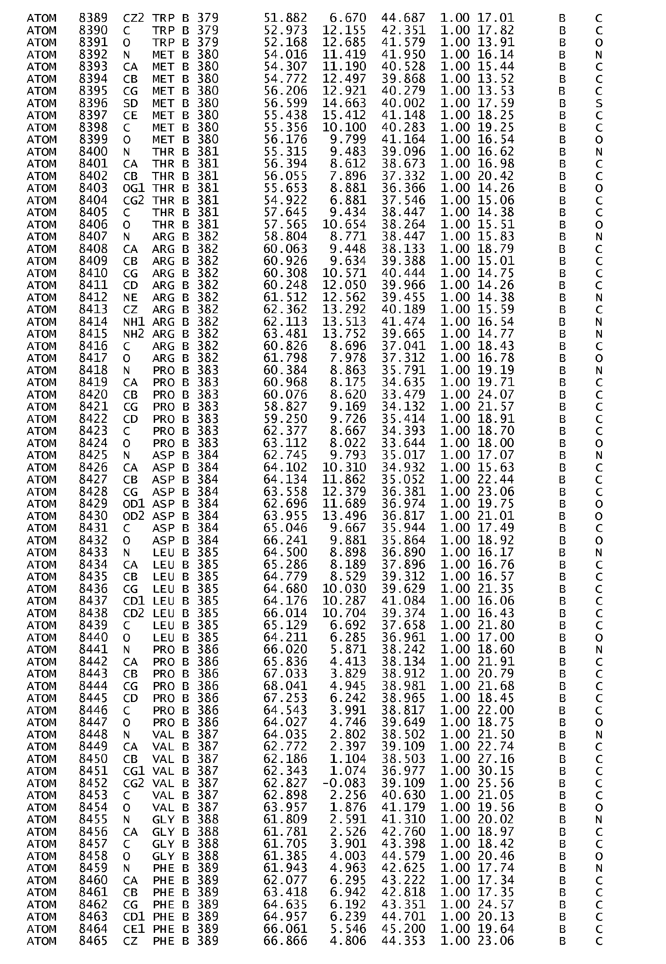

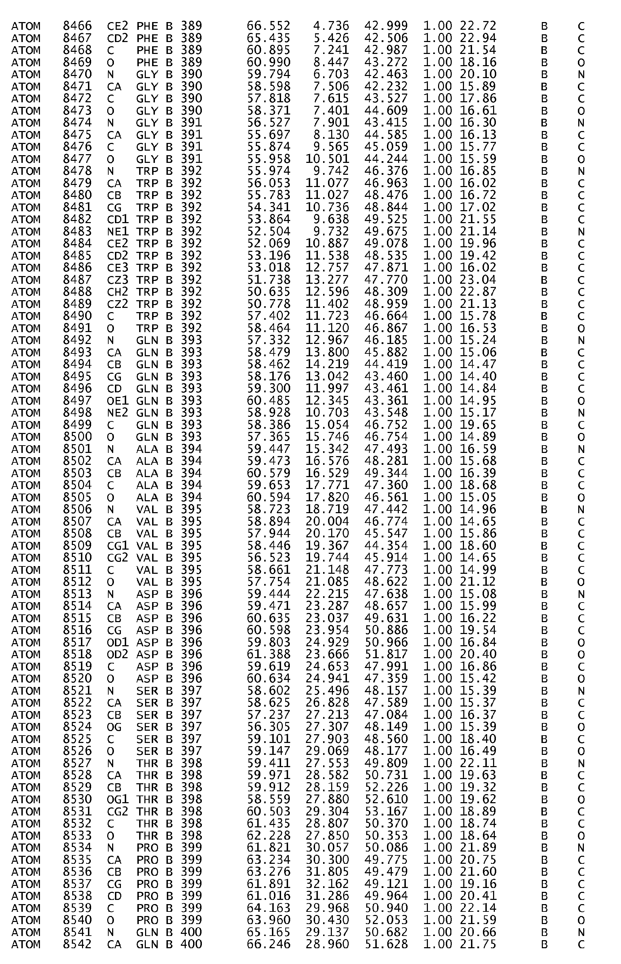

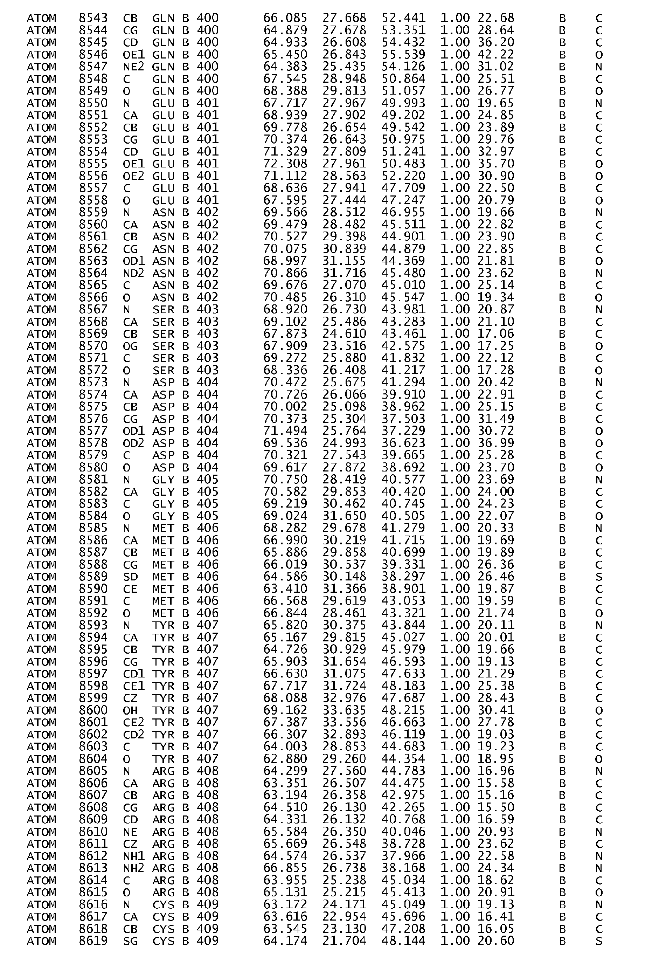

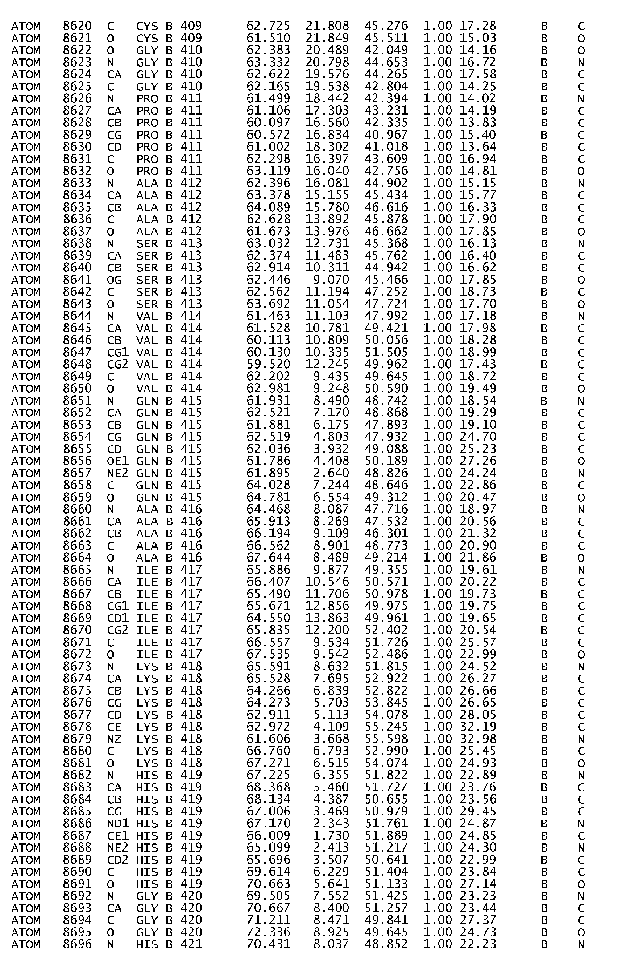

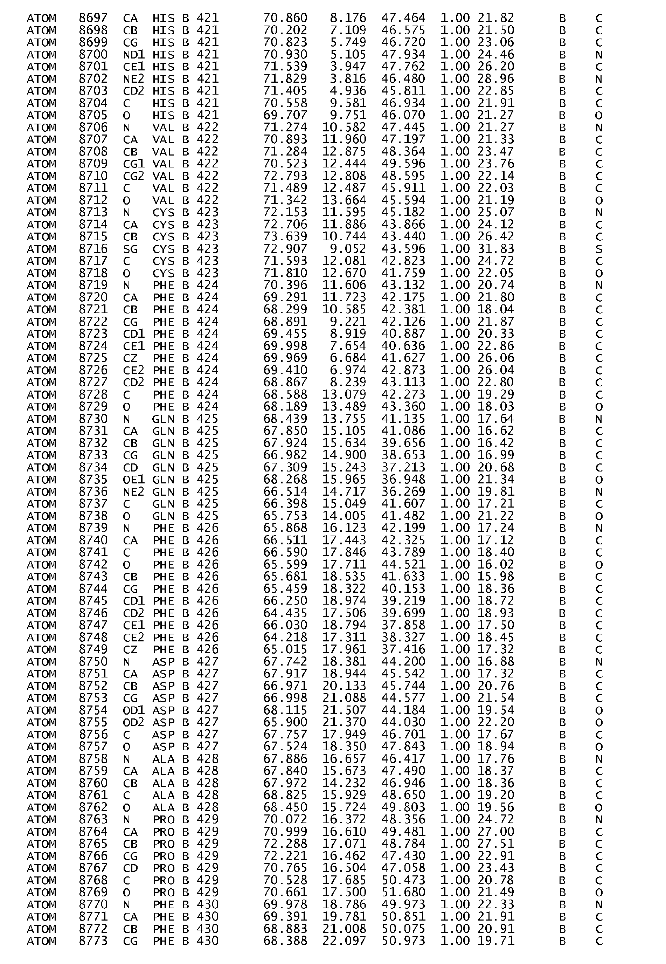

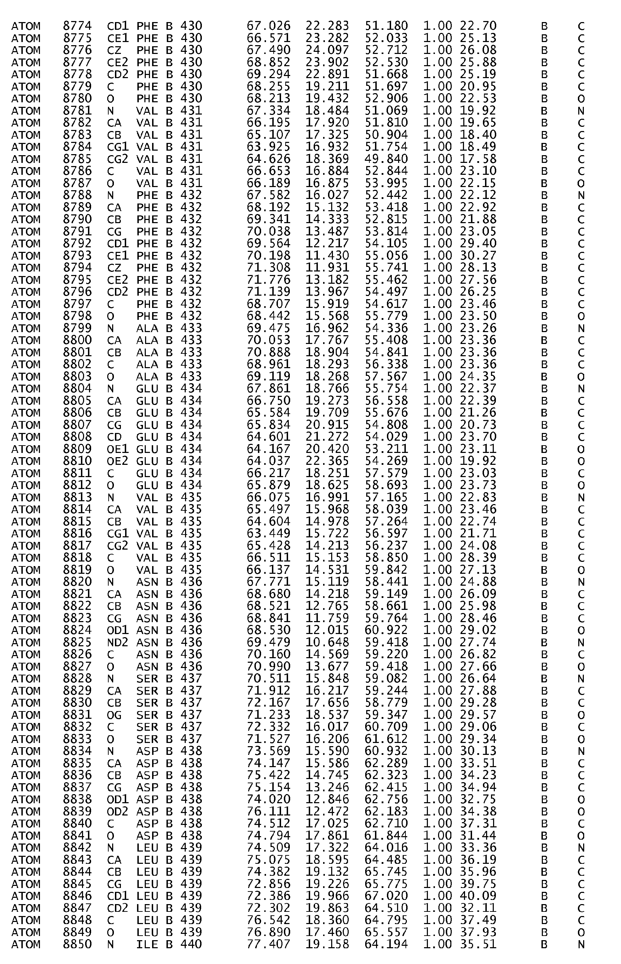

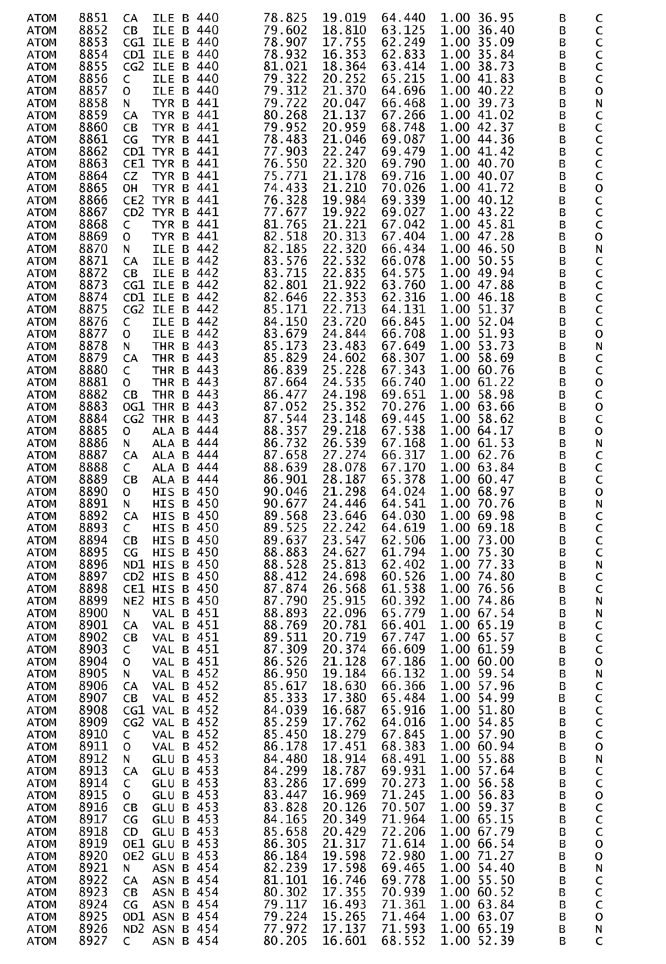

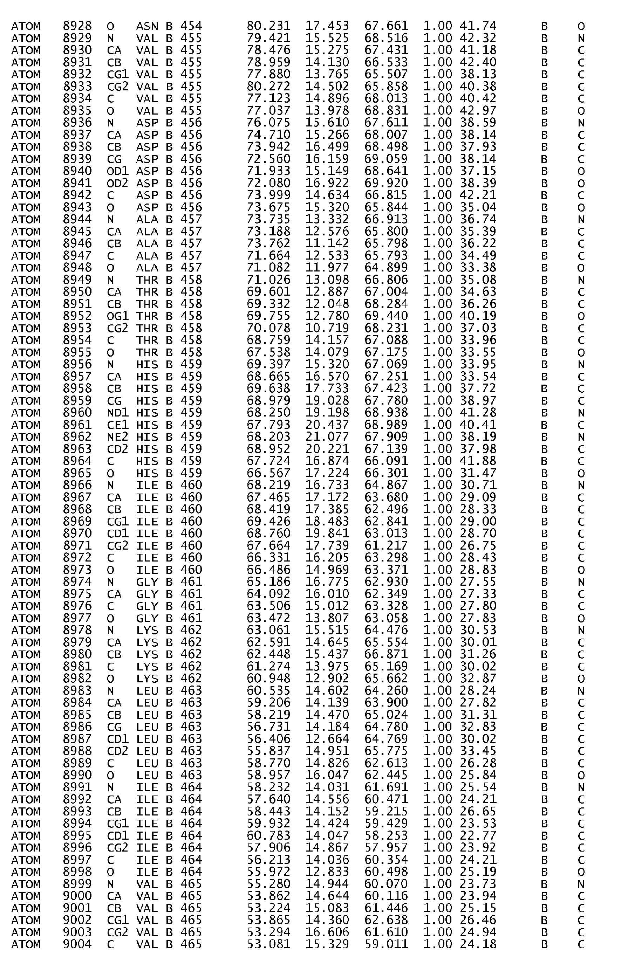

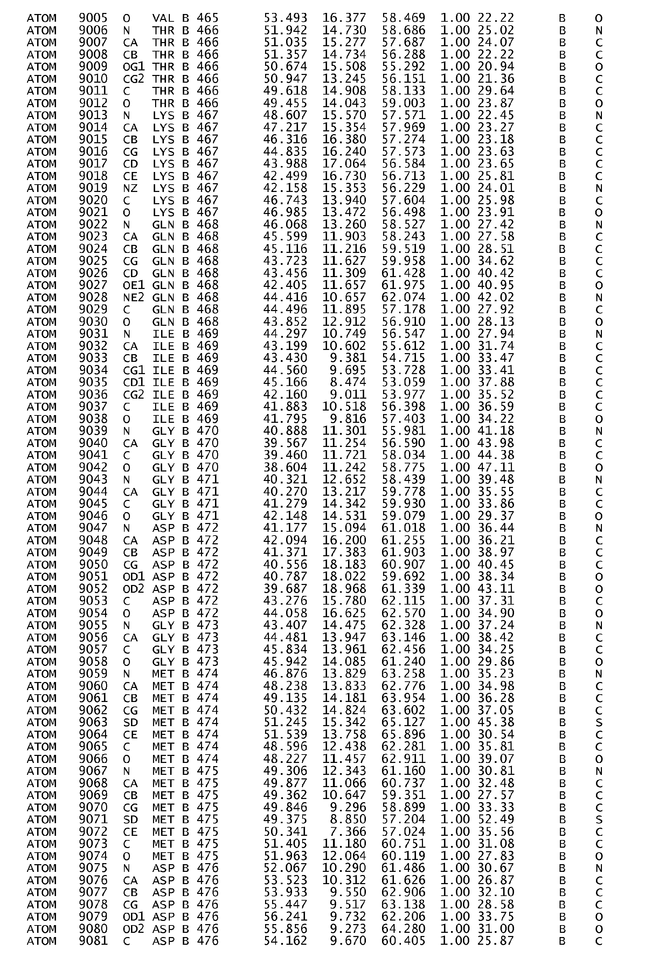

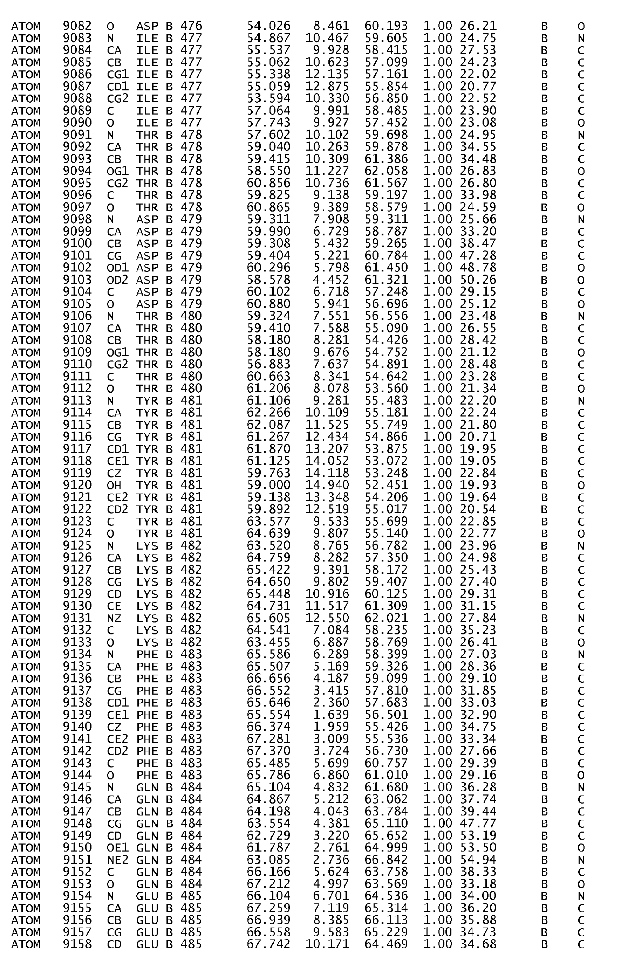

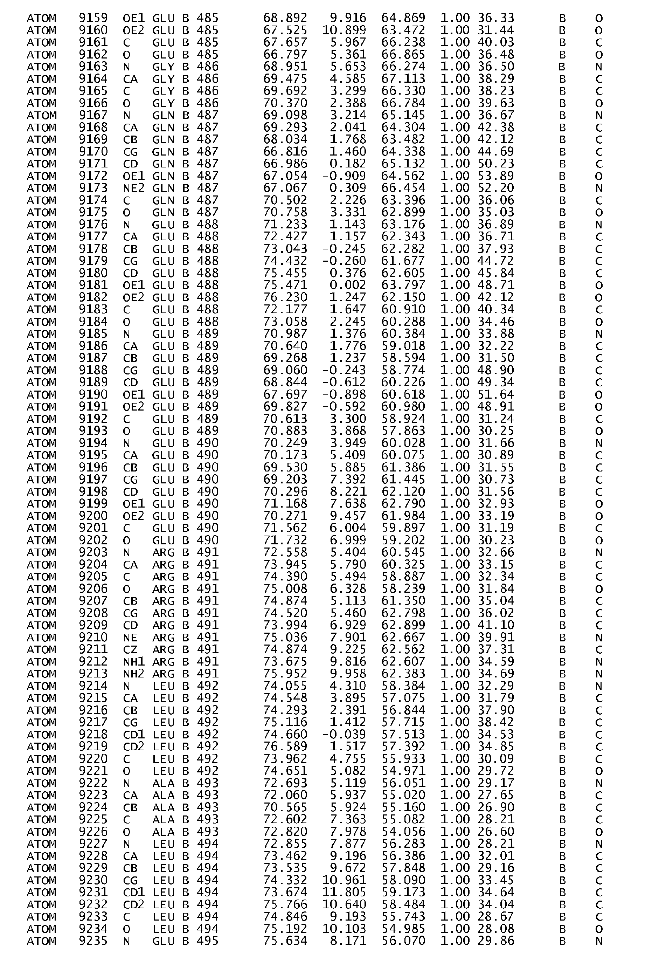

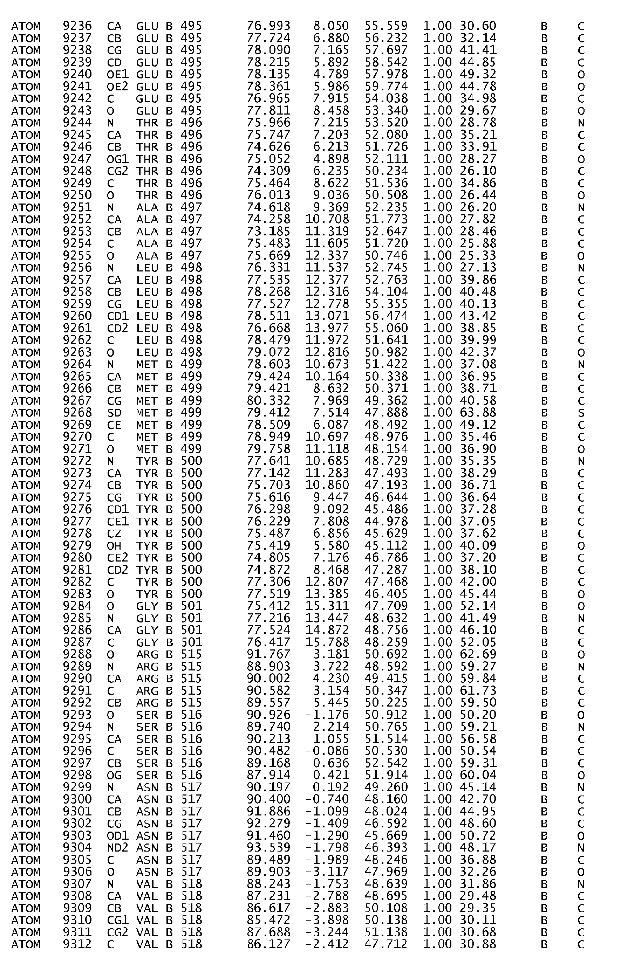

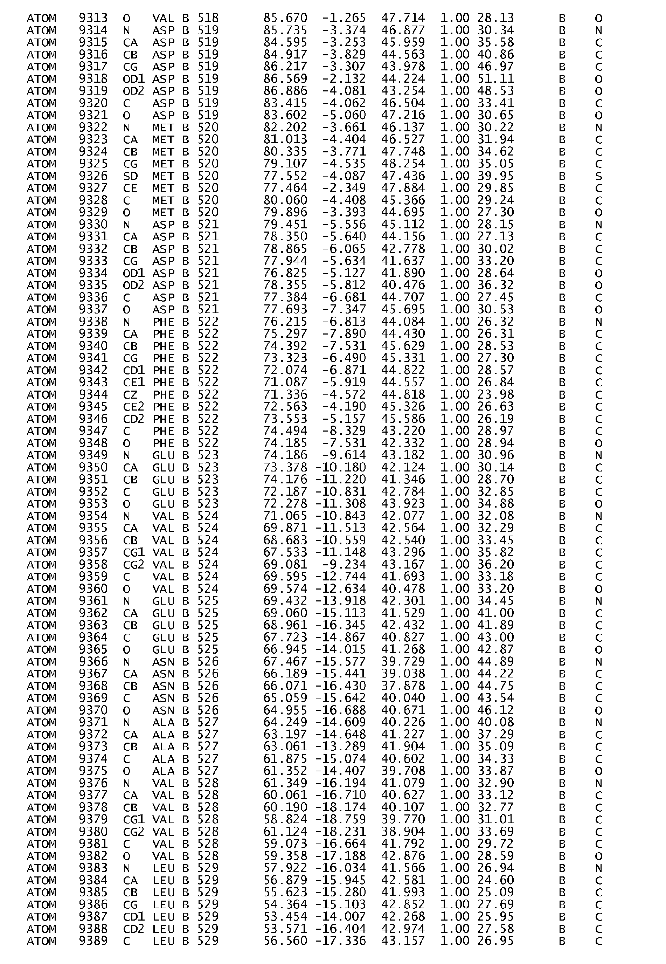

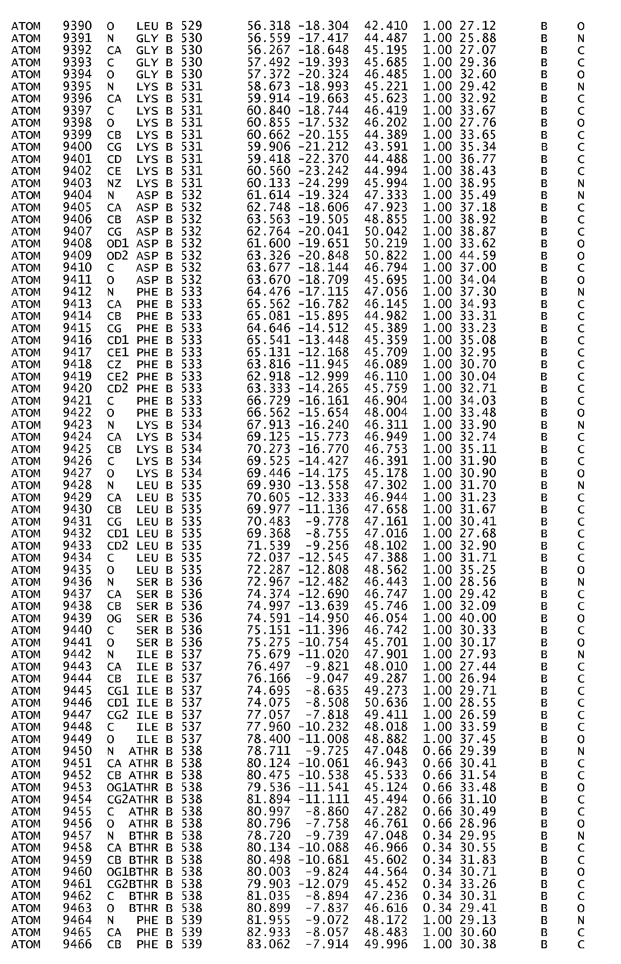

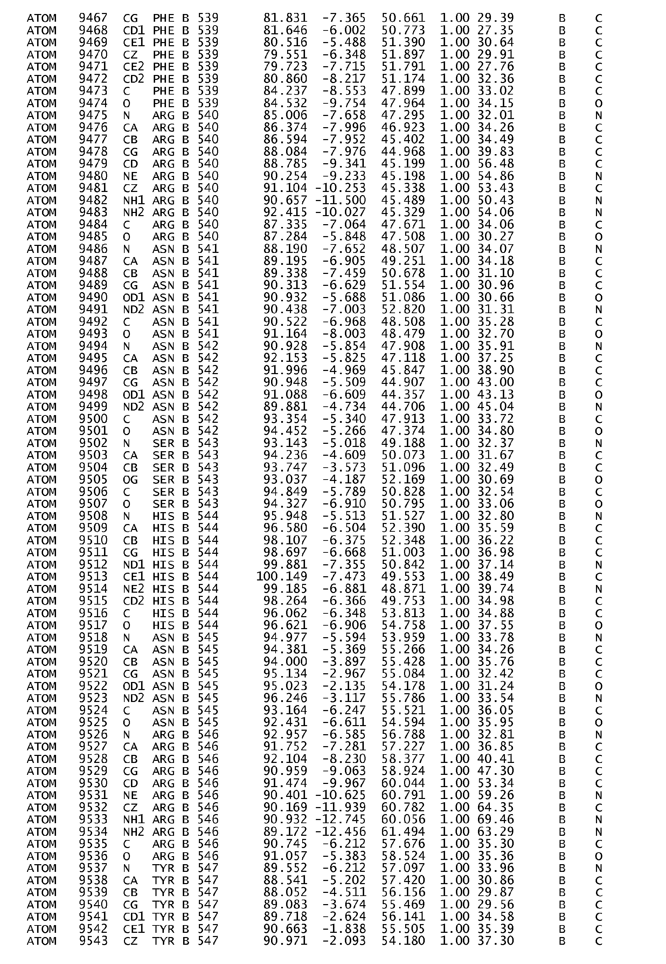

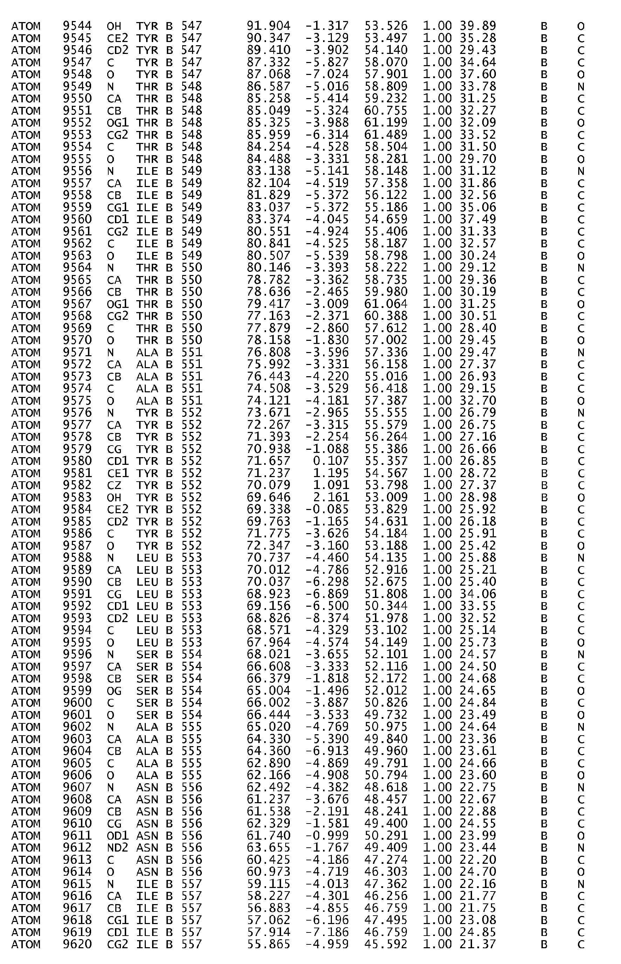

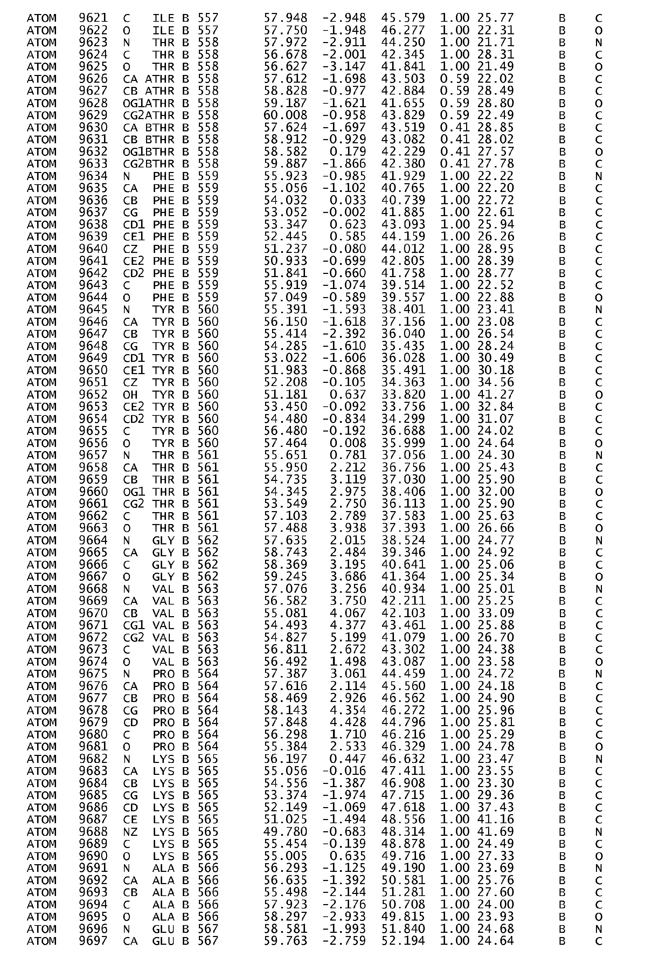

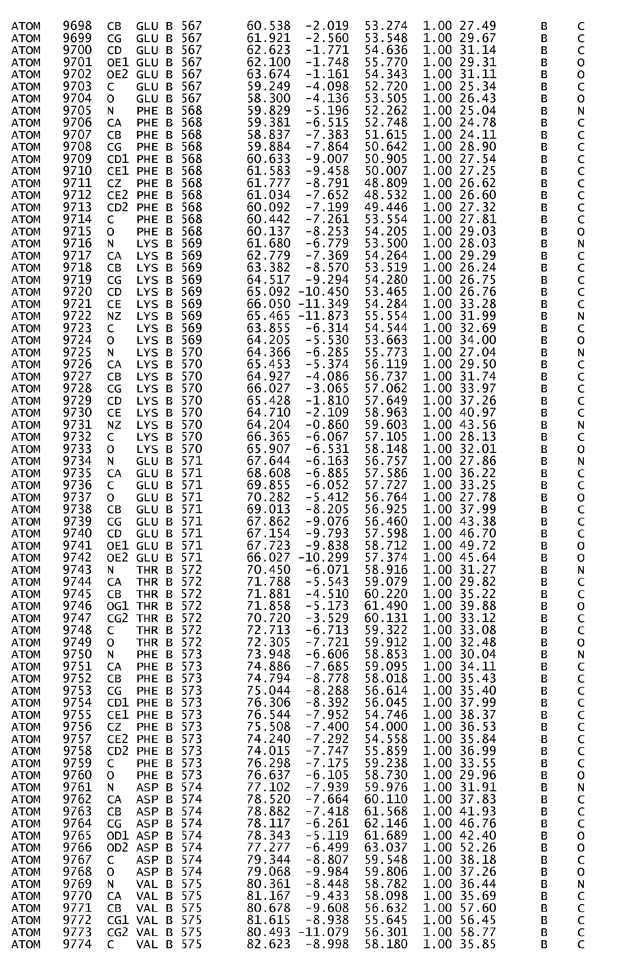

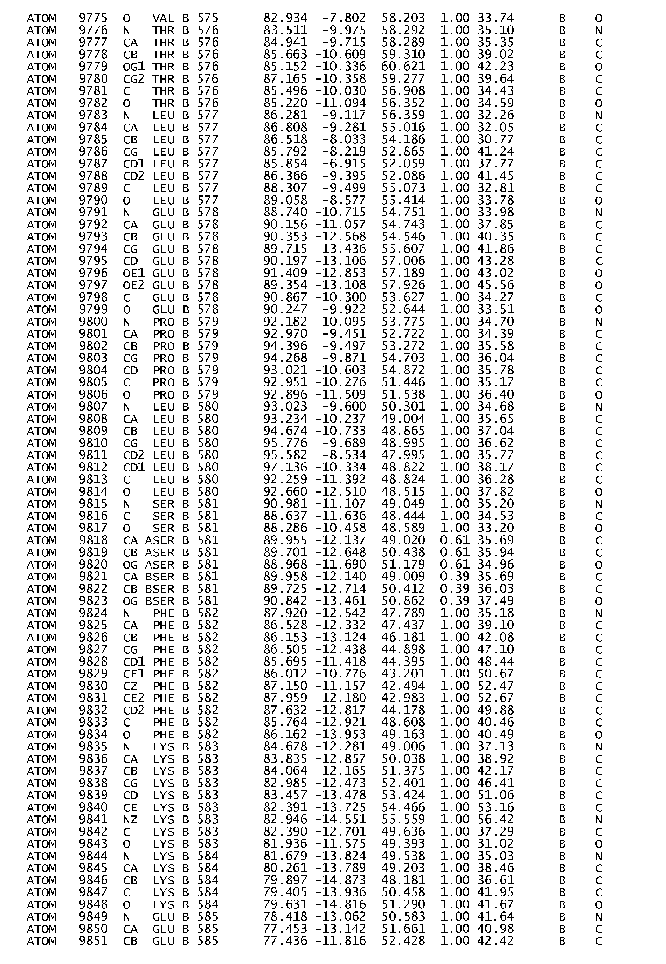

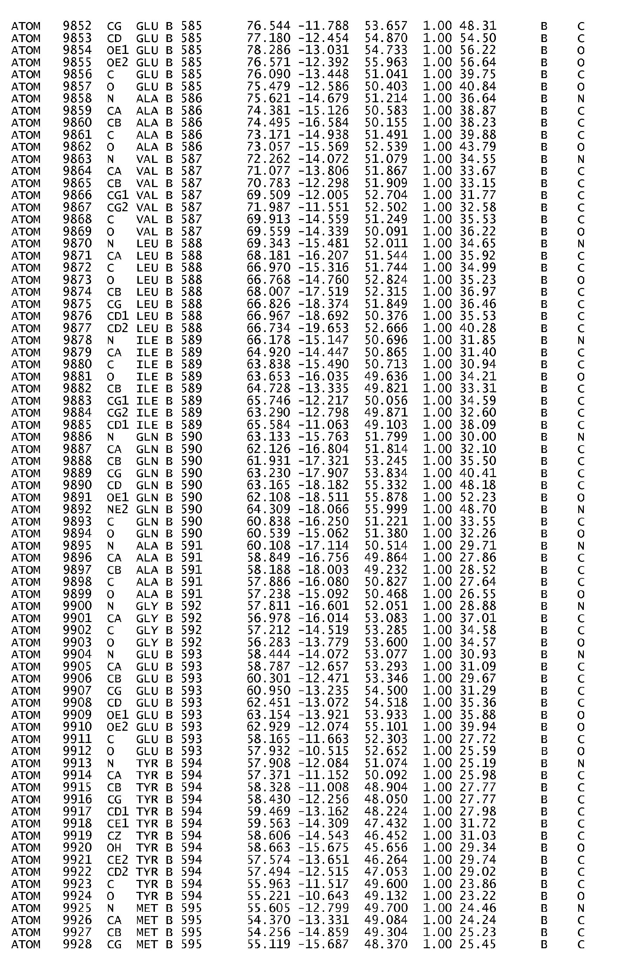

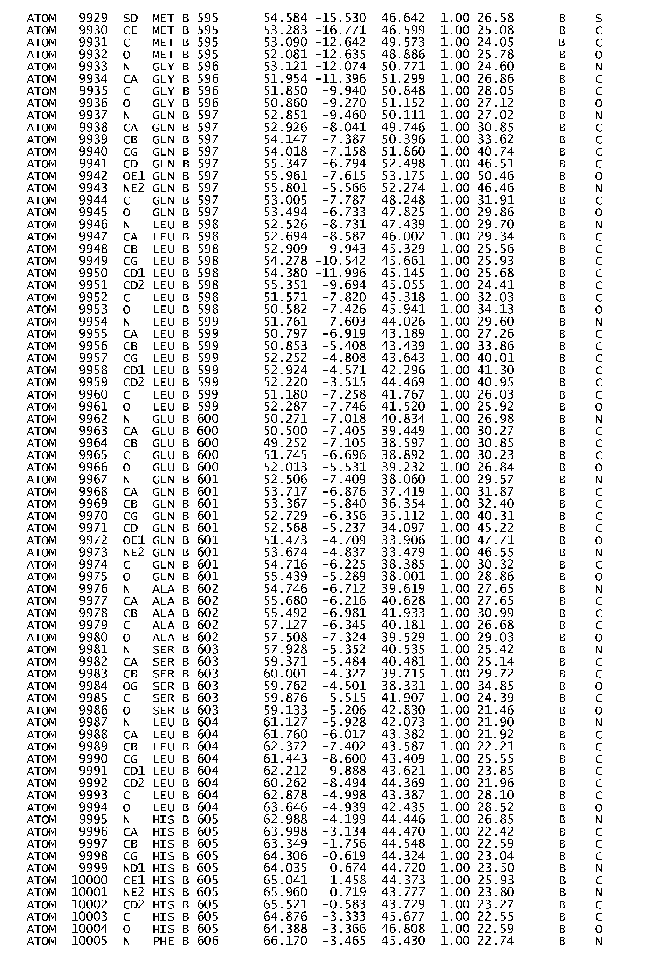

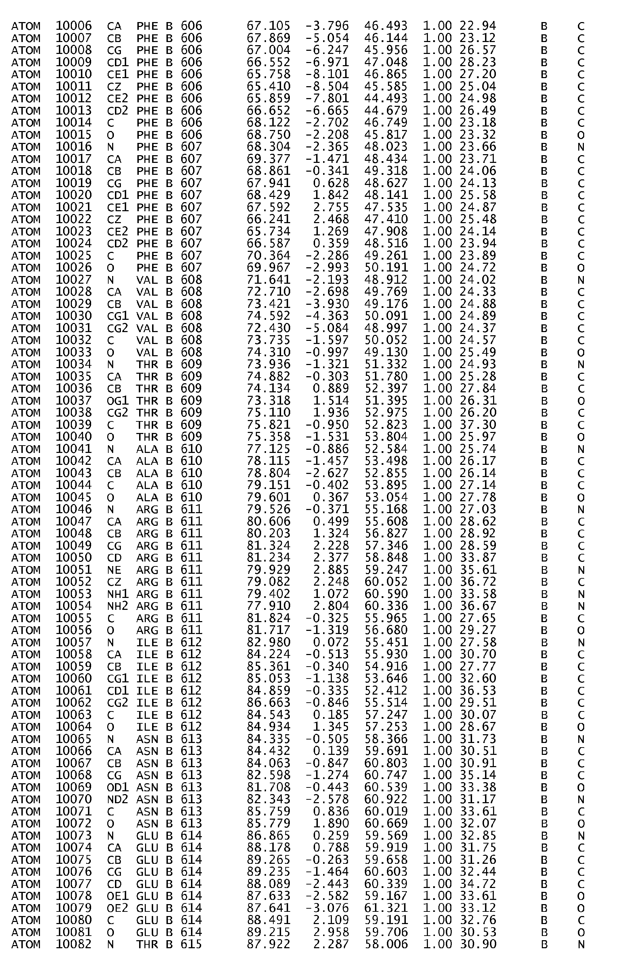

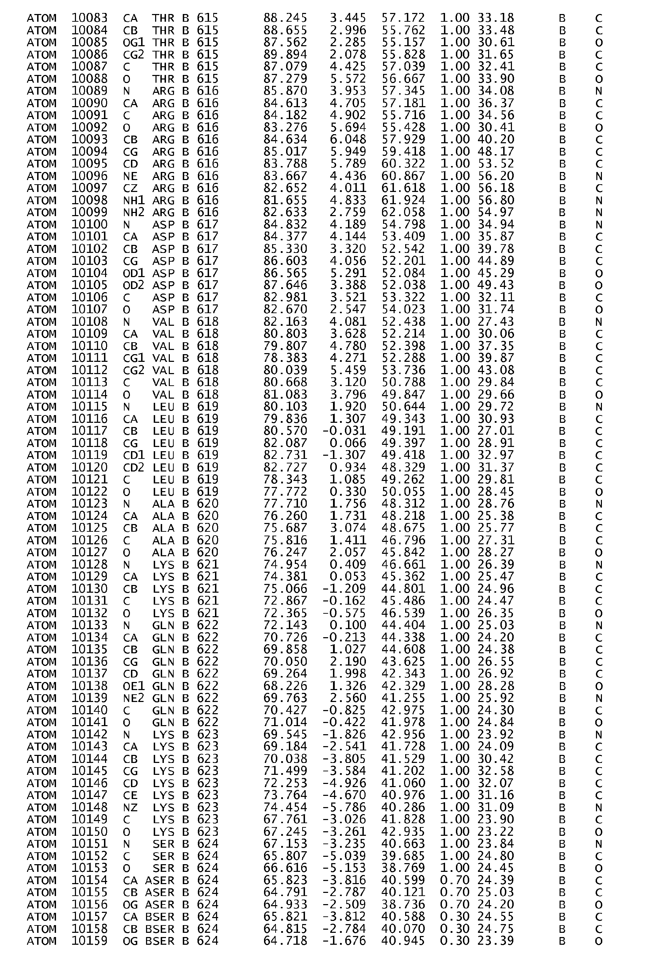

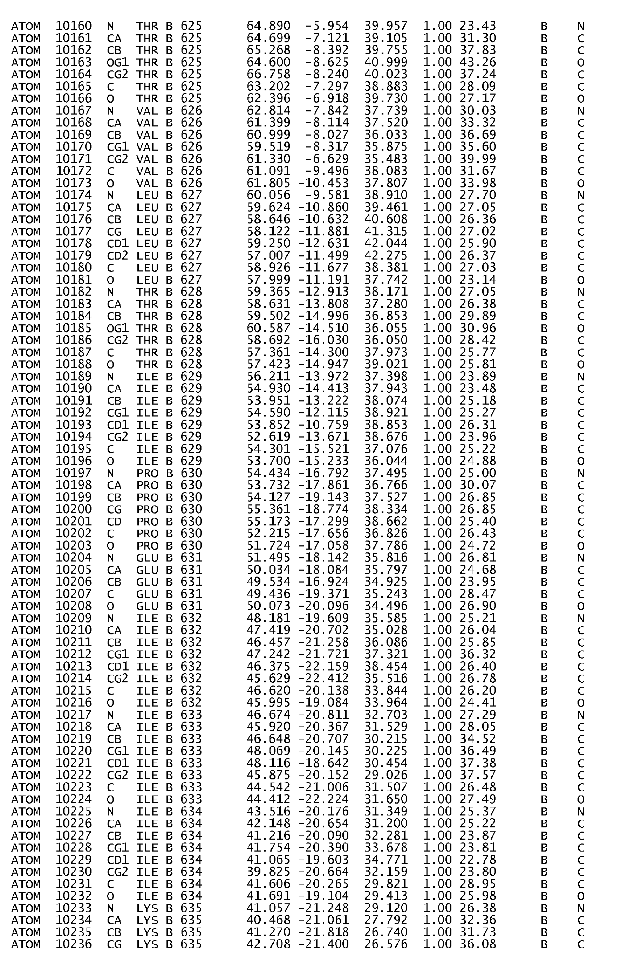

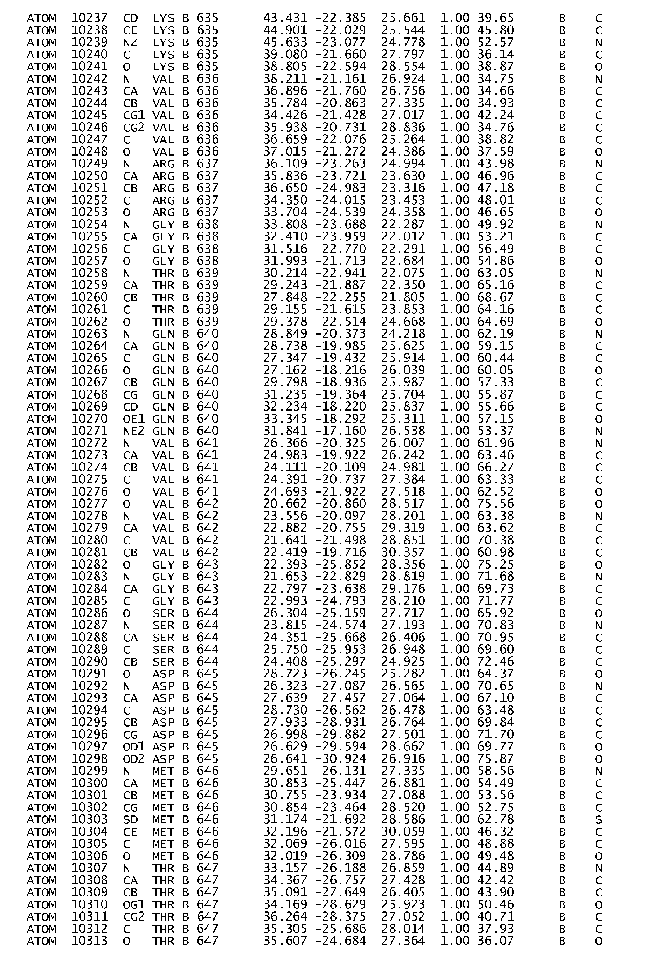

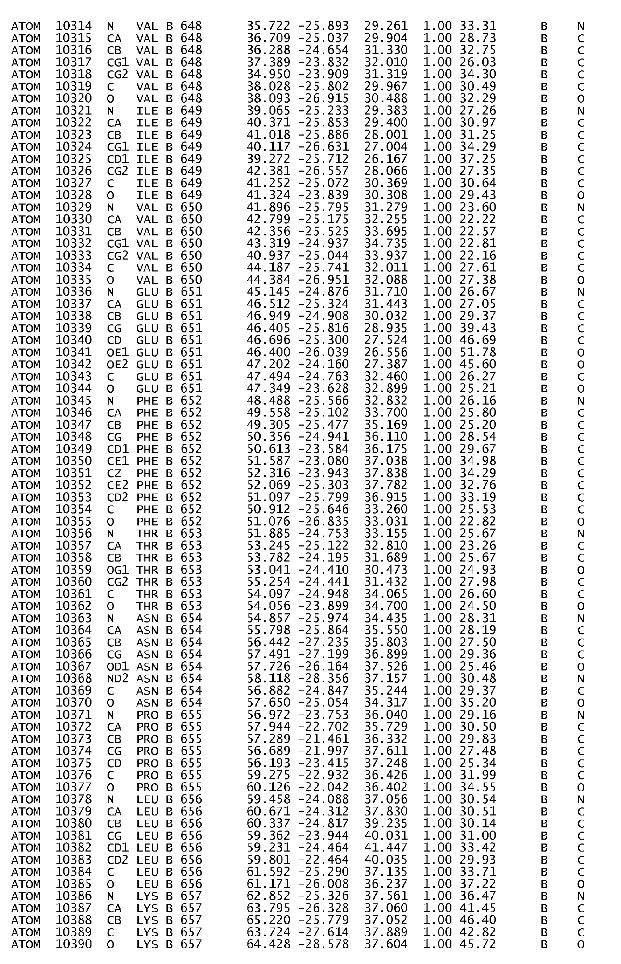

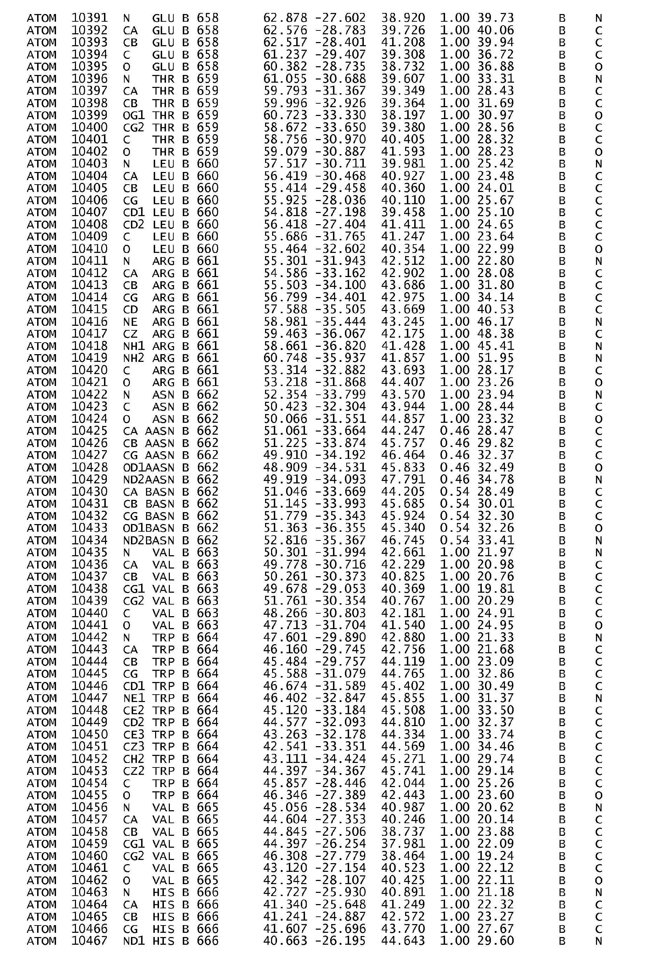

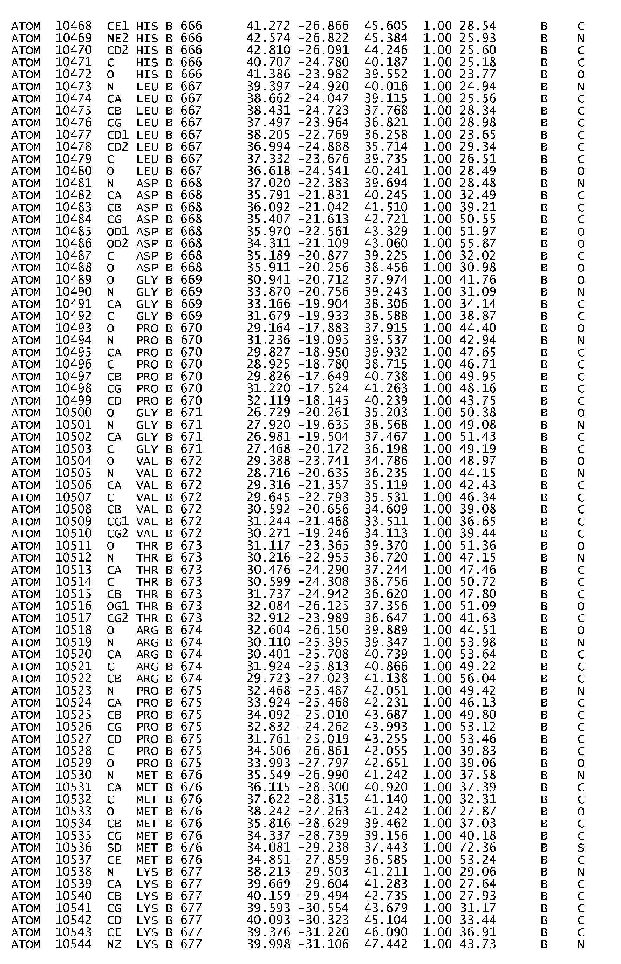

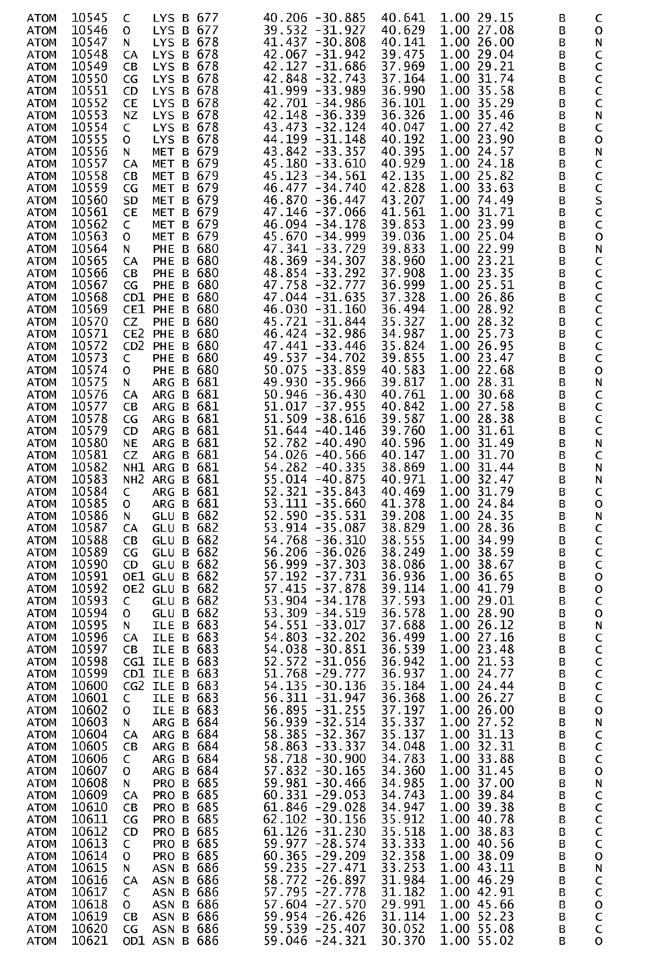

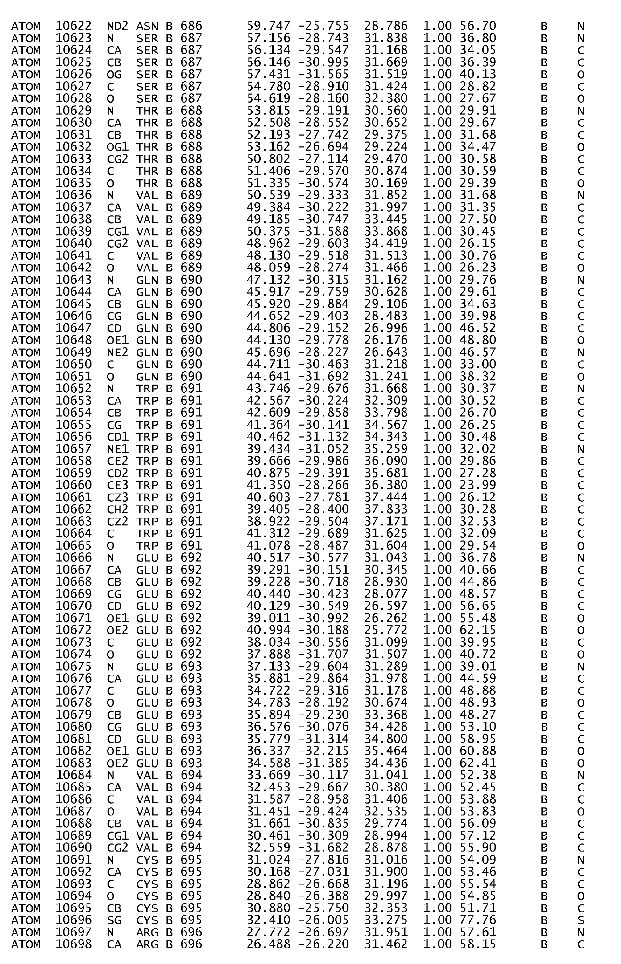

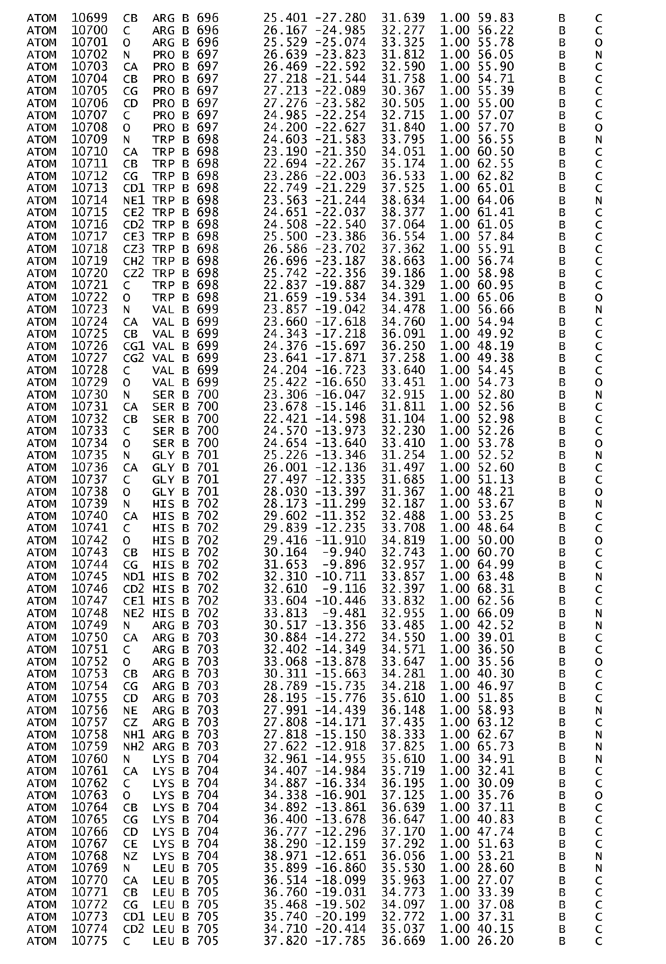

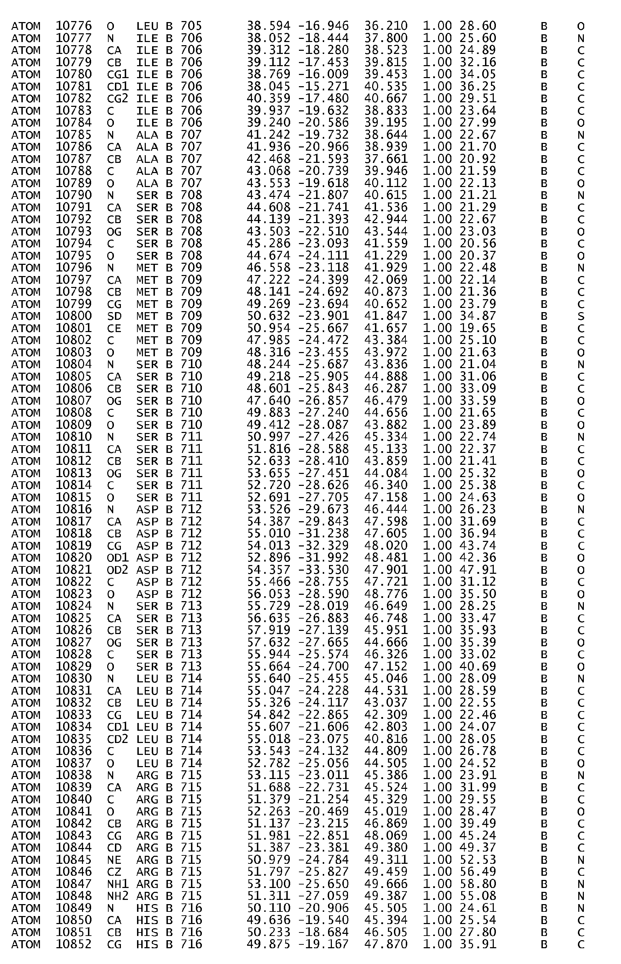

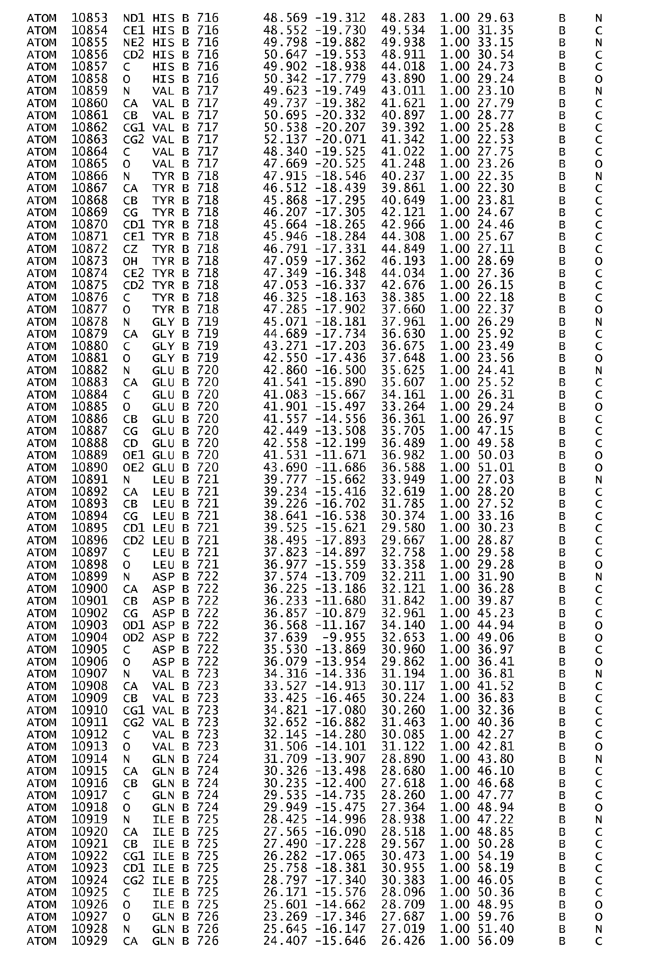

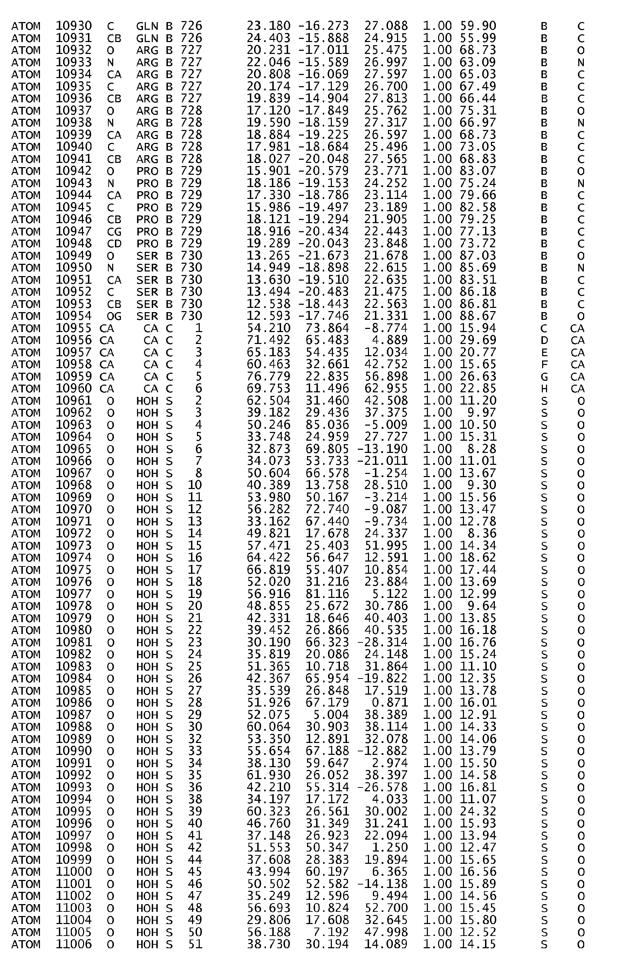

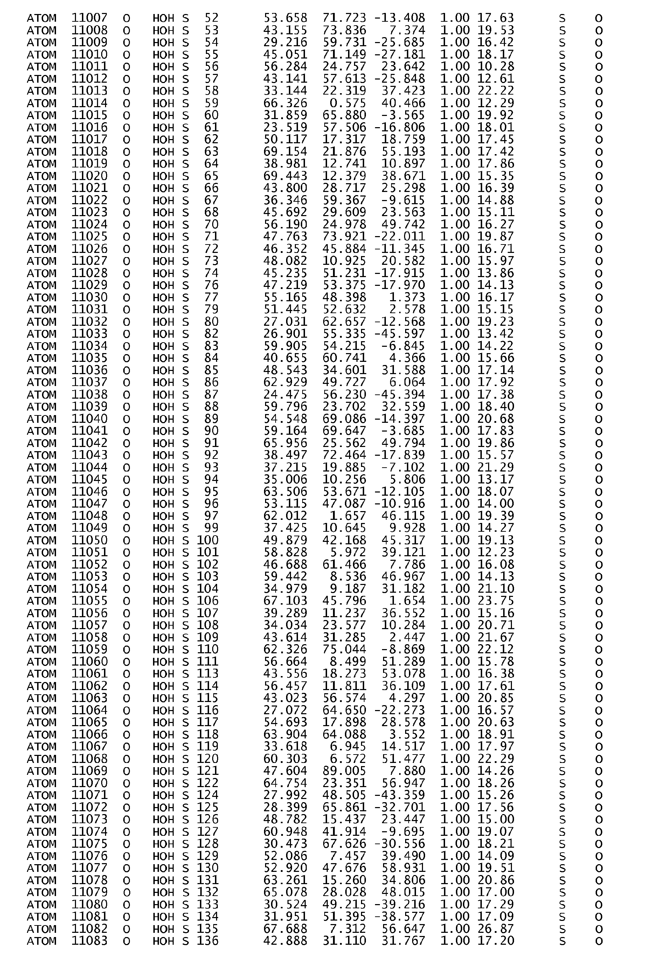

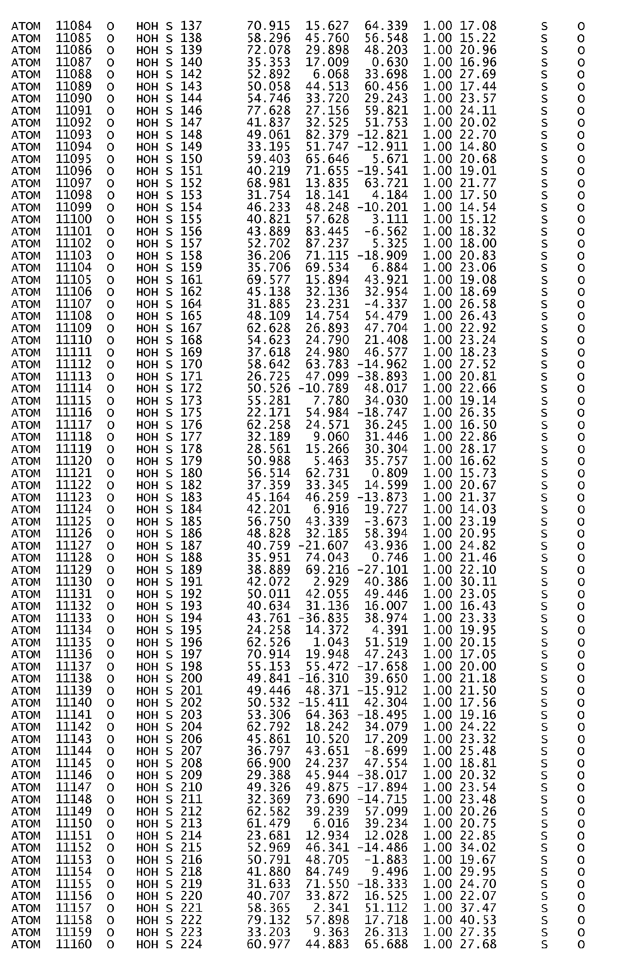

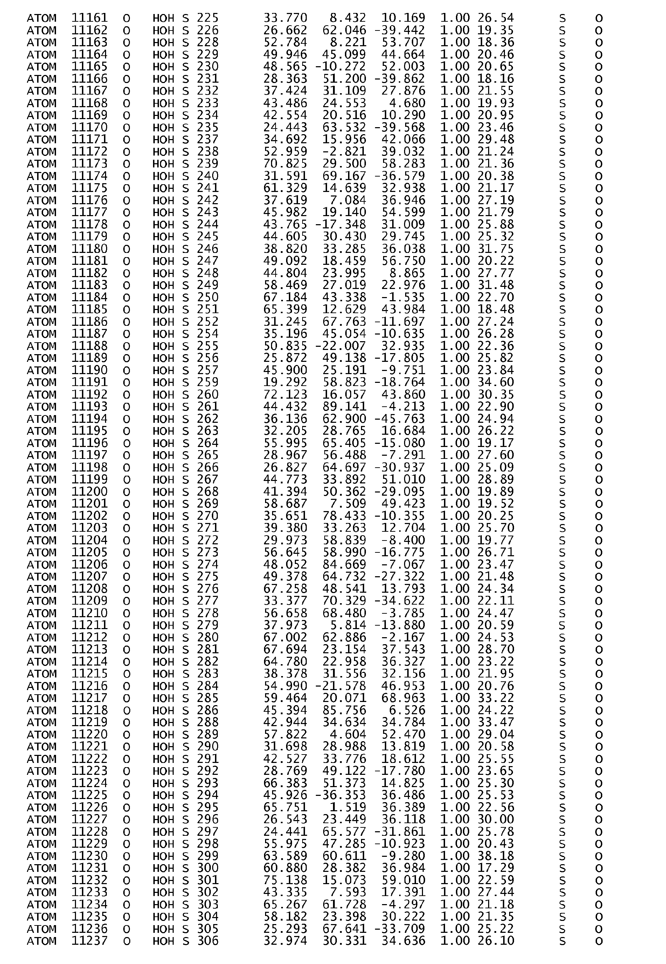

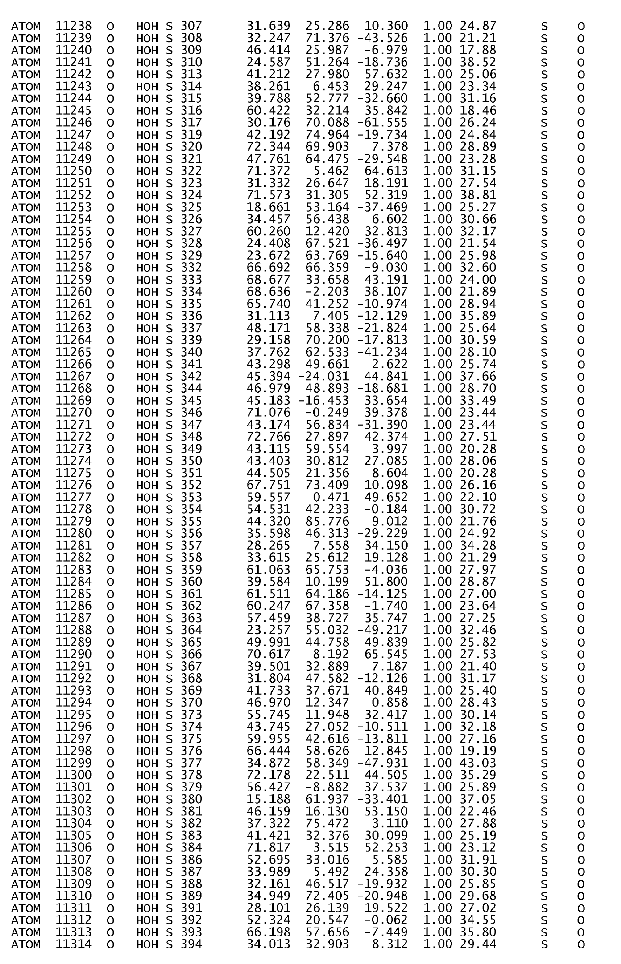

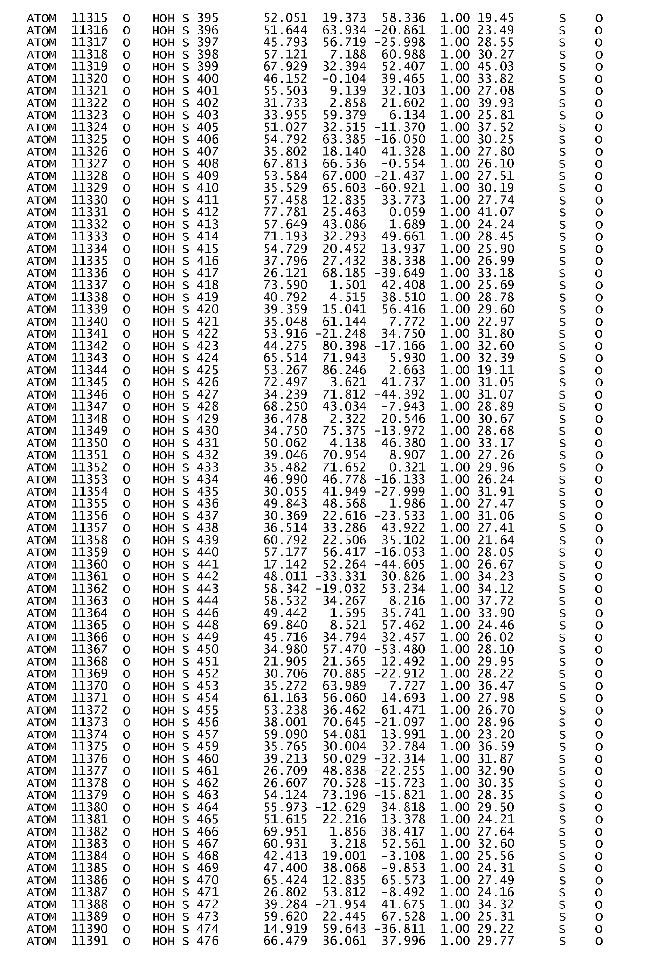

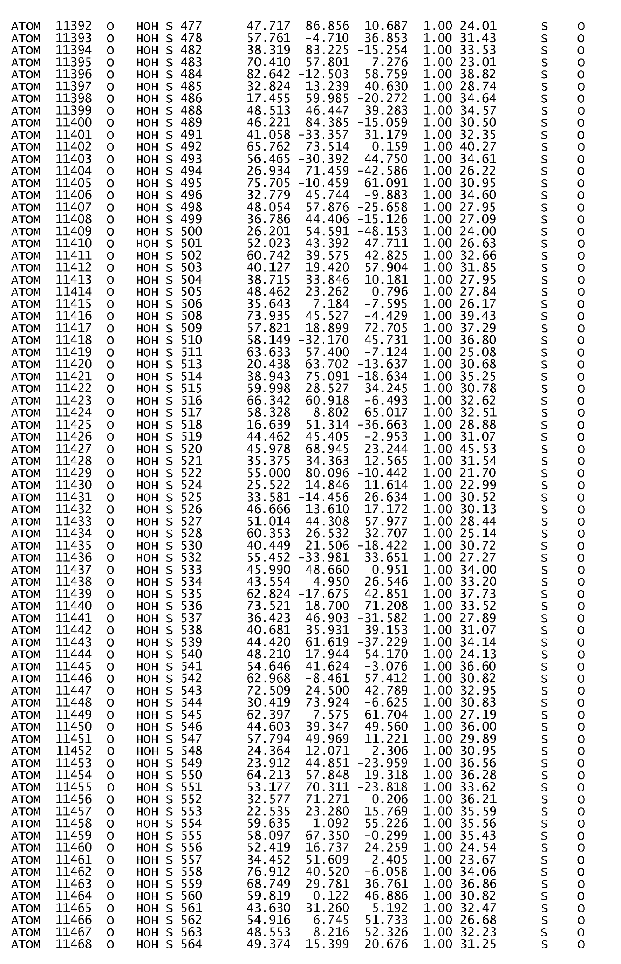

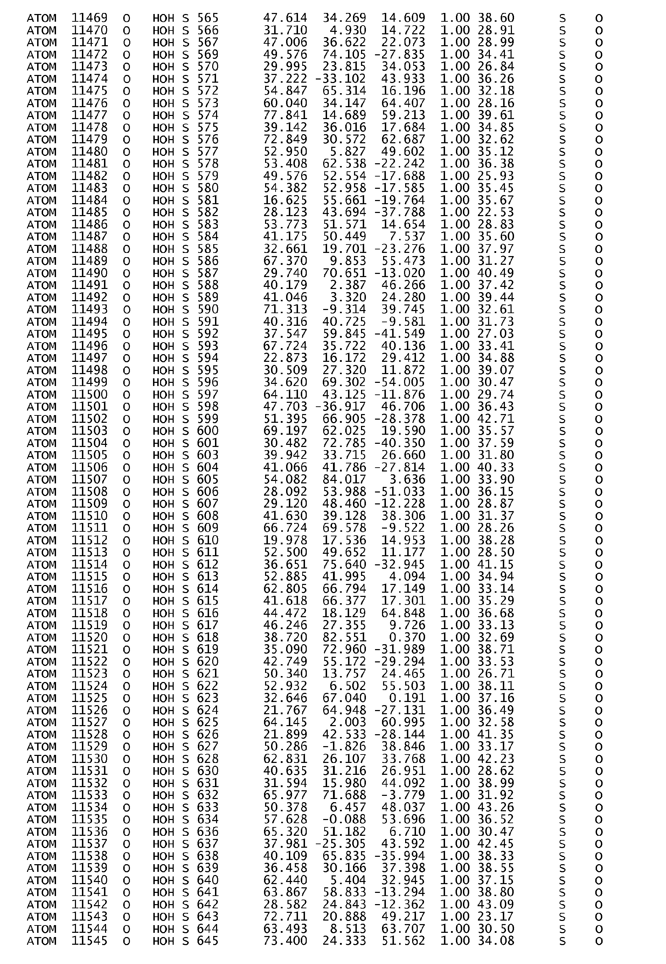

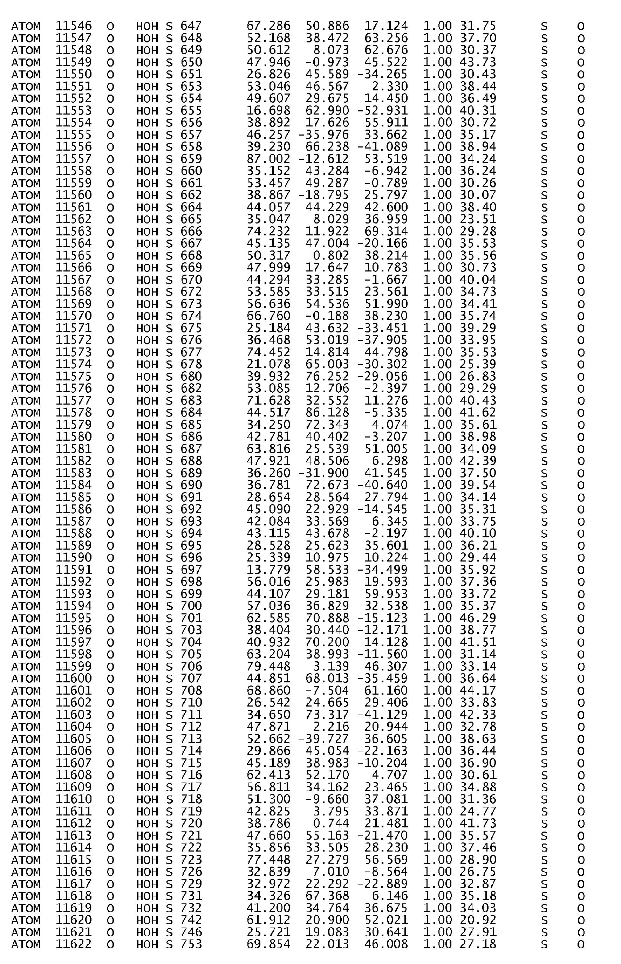

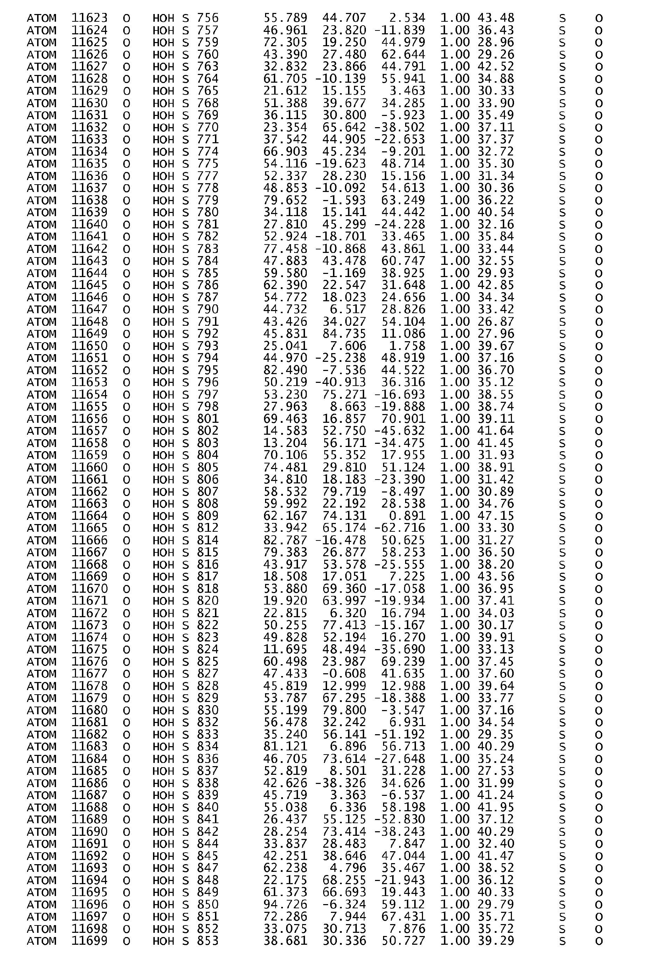

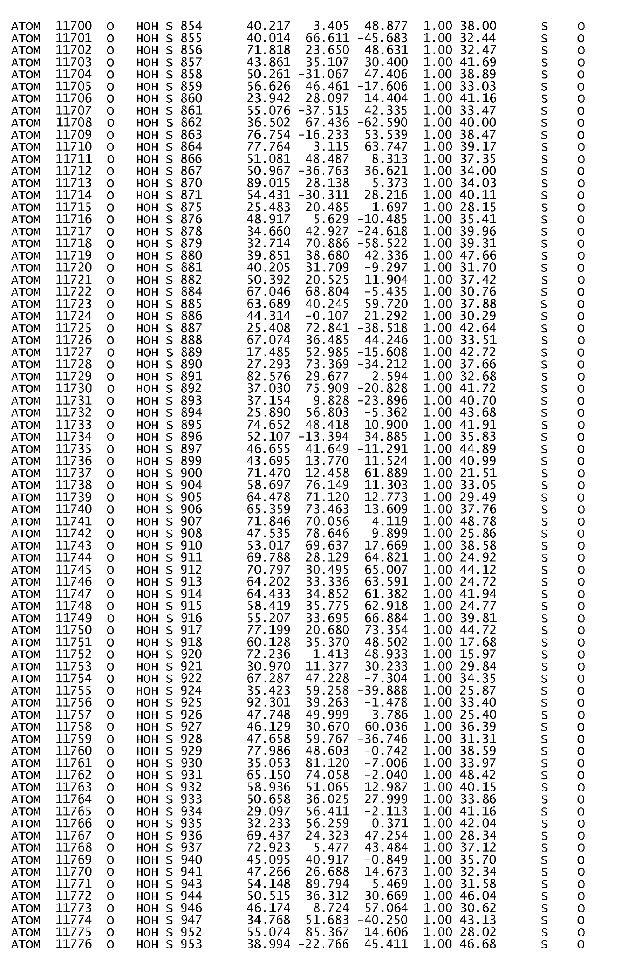

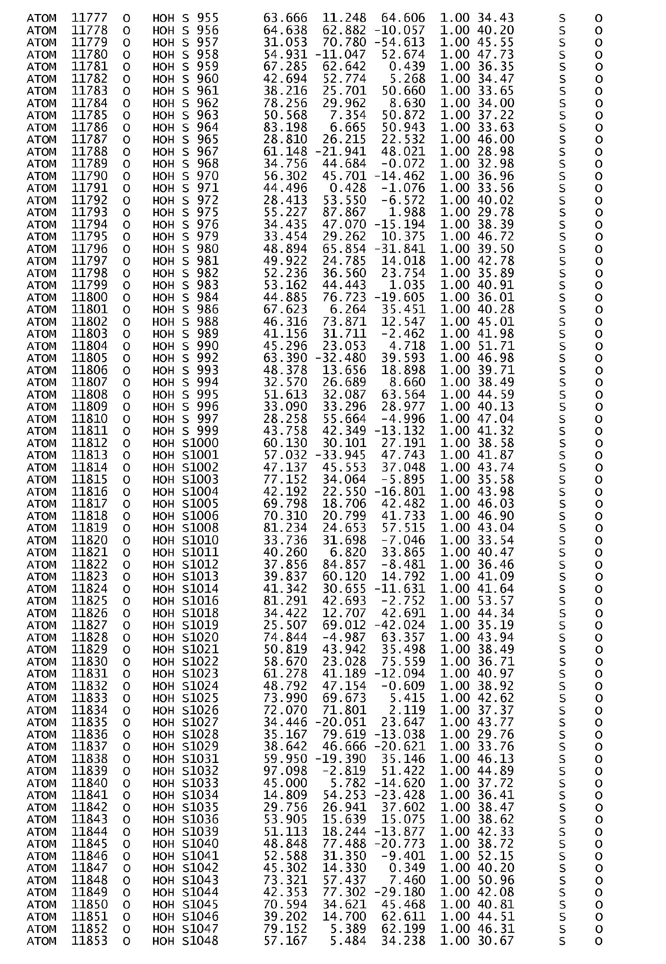

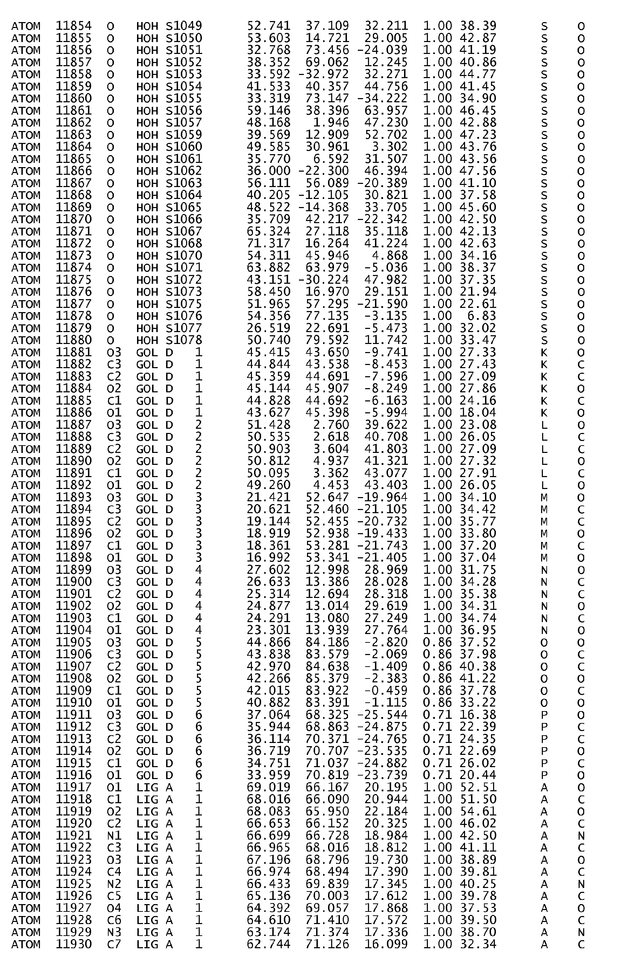

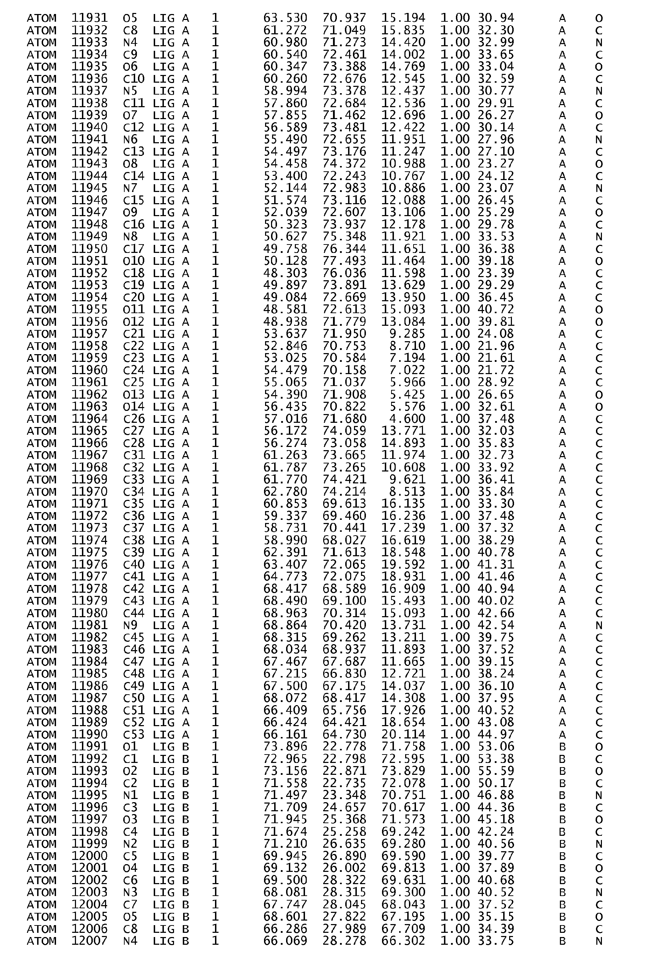

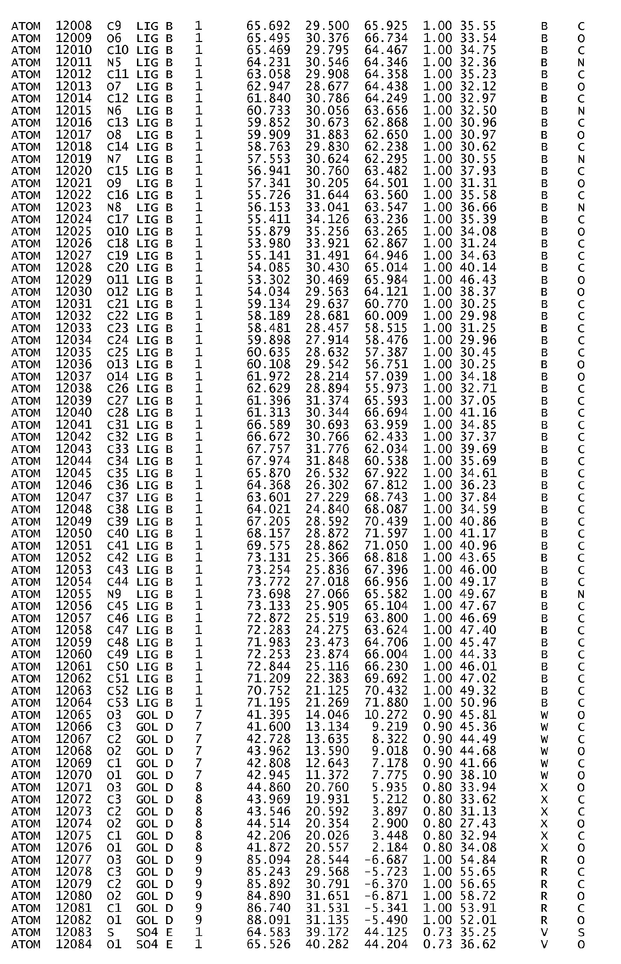

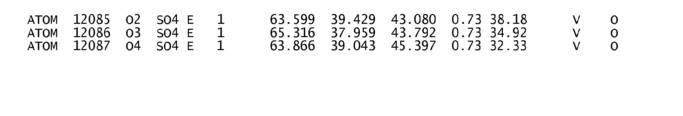

The crystal form of blood coagulation factor XIIIa having a space group of P1, two monomers of coagulation factor XIIIa in the asymmetric unit and unit cell dimension of a₁ = 56 ± 3 Å, b₁ = 80 ± 3Å, C₁ = 103 ± 3 Å, α₁ = 88 ± 3°, β₁ = 77 ± 3° and γ₁ = 82 ± 3° is further characterized by the atomic structure coordinates of figure 6.

The term "crystal form" as used herein, refers to a crystal form of the blood coagulation factor XIIIa with or without inhibitor and/or woth or without detergents and/or lipids bound to the blood coagulation factor XIIIa.

The crystal forms of blood coagulation factor XIIIa can also be characterized as follows so that the present invetion relted to crystal forms of blood coagulation factor XIIIa having a space group of P1, two monomers of coagulation factor XIIIa in the asymmetric unit and unit cell dimension of a₁ = 56 Å ± 5%, b₁ = 80 Å ± 5%, c₁ = 103 Å ± 5%, α₁ = 88° ± 5%, β₁ = 77° ± 5% and γ₁ = 82° ± 5%; or
having a space group of P1, two monomers of coagulation factor XIIIa in the asymmetric unit and unit cell dimension of a₂ = 57 Å ± 5%, b₂ = 64 Å ± 5%, c₂ = 67 Å ± 5%, α₂ = 76° ± 5%, β₂ = 72° ± 5% and γ₂ = 83° ± 5%; or
having a space group of C2, two monomers of coagulation factor XIIIa in the asymmetric unit and unit cell dimension of a₃ = 90 Å ± 5%, b₃ = 65 Å ± 5%, c₃ = 70 Å ± 5%, α₃ = 90° ± 5%, β₃= 96° ± 5% and γ₃= 90° ± 5%.

In other words, the present invention is directed to the crystal forms of blood coagulation factor XIIIa characterized as having a space group of P1, two monomers of coagulation factor XIIIa in the asymmetric unit and unit cell dimension of a₁ = 53 Å - 59 Å, b₁ = 76 Å - 84 Å, c₁ = 98 Å - 108 Å, α₁ = 84° - 92°, β₁ = 73° - 81° and γ₁ = 78° - 86°; or
having a space group of P1, two monomers of coagulation factor XIIIa in the asymmetric unit and unit cell dimension of a₂ = 53 Å - 59 A, b₂ = 61 Å - 67 Å, c₂ = 64 Å - 70 Å, α₂ = 72° - 80°, β₂ = 68° - 76° and γ₂ = 79° - 87°; or
having a space group of C2, two monomers of coagulation factor XIIIa in the asymmetric unit and unit cell dimension of a₃ = 86 Å - 94 Å, b₃ = 62 Å - 68 Å, c₃ = 66 Å - 74 Å, α₃ = 86° - 94°, β₃ = 92° - 100° and γ₃ = 86° - 94°.

The term "unit cell" as used herein refers to the smallest repeating unit that can generate a crystal with only translation operations. The unit cell is the smallest unit of volume that contains all of the structural and symmetry information of a crystal and that by translation can reproduce a pattern in all of space. Structural information is thereby the pattern (atoms) plus all surrounding space and symmetry information means mirrors, glides, axes, and inversion centers. The translation refers to motion along a cell edge the length of the cell edge. An asymmetric unit is the smallest unit that can be rotated and translated to generate one unit cell using only the symmetry operators allowed by the crystallographic symmetry. The asymmetric unit may be one molecule or one subunit of a multimeric protein, but it can also be more than one. Hence the asymmetric unit is the smallest unit of volume that contains all of the structural information and that by application of the symmetry operations can reproduce the unit cell. The shape of the unit cell is constrained by the collection of symmetry elements which make-up the group. For space groups, seven lattice types are possible: triclinic, monoclinic, orthorhombic, tetragonal, hexagonal, rhombic and cubic. In crystallography, space groups are also called the crystallographic or Fedorov groups.

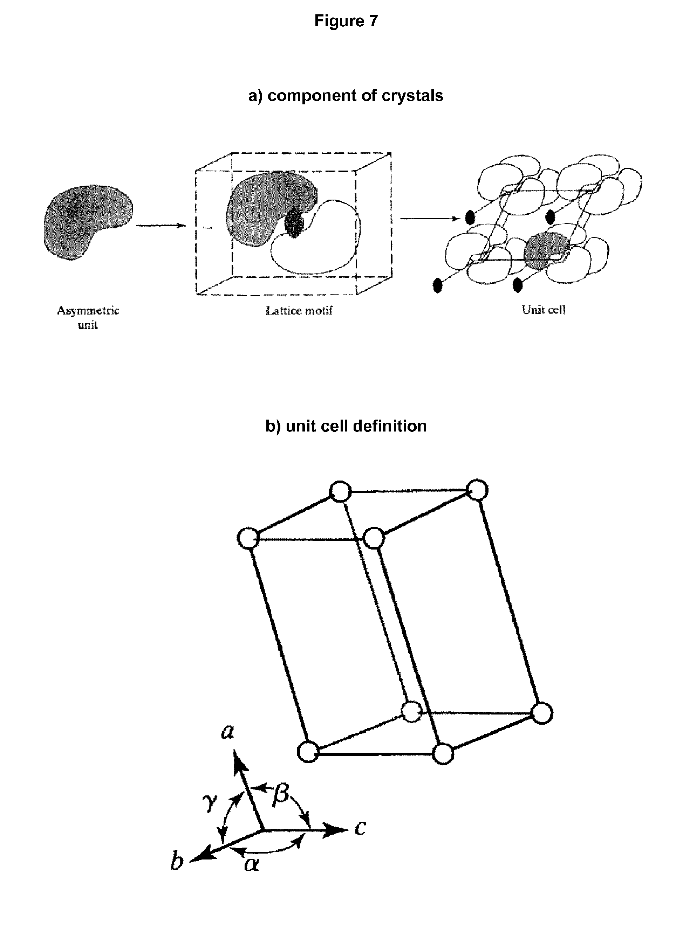

The edges of the unit cell define a set of unit vector axies **a**, **b**, and **c**, with the unit cell dimensions as their respective length a, b, and c (figure 7). These vectors need not be at right angles, and the angles between the axes are denoted α as between the **bc**-axes, β between the **ac**-axes, and γ between the **ab**-axes. The different shapes arise depending on restrictions placed on the lengths of the three edges (*a*, *b,* and *c*) and the values of the three angles (α, β, and γ).

### Active Site of Blood Coagulation Factor XIIIa

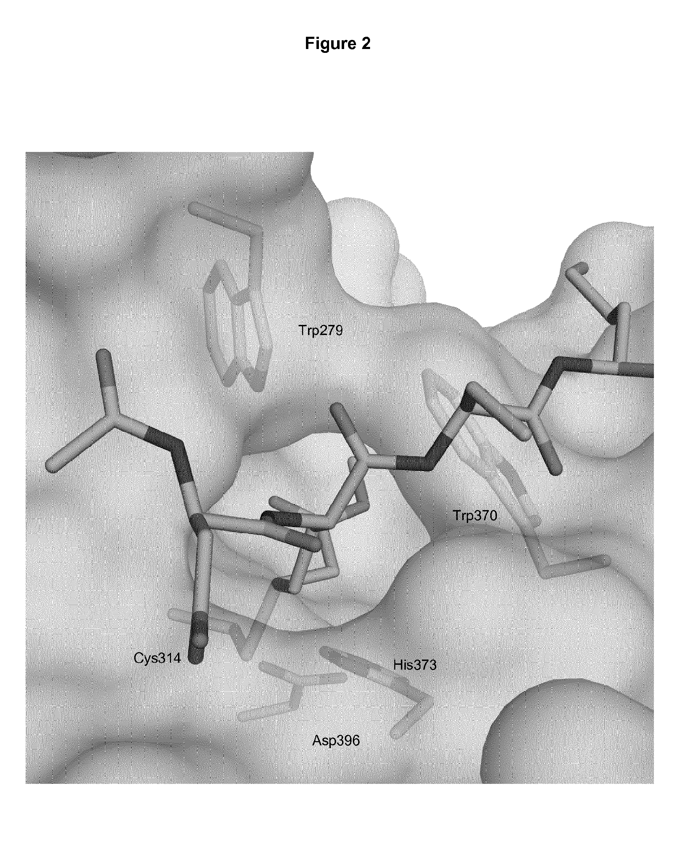

In the present invention crystal forms of blood coagulation factor XIIIa comprise several interaction sites. Active site refers in general to the site where the enzymatic reaction takes place. The association of ligands or substrates with the active site of their corresponding receptors or enzymes is the basis of many biological mechanisms. Similary, many drugs exert their biological effects through association with the active site of receptors and enzymes. Such associations may occur with all or part of the active site. An understanding of such associations will lead to the structure based design of drugs having more favourable associations with their target receptor or enzyme, and thus, improved biological effects. Therefore, this information is valuable in rational designing potential inhibitors of important targets. The active sites of blood coagulation factor XIIIa which can be used for drug design are shown in figures 2 - 5 and can be classified as follows:
a) The catalytic center
b) The hydrophobic pocket
c) The surface between said catalytic center and hydrophobic pocket
d) The pocket neighboring the catalytic center

All or some of these areas might be used for rational drug design targeting efficacy and selectivity.

Thus a significant feature of the present invention resides from the crystal form of blood coagulation factor XIIIa comprising among others four main active sites namely:
a) a catalytic center
b) a hydrophobic pocket
c) a surface between said catalytic center and the hydrophobic pocket
d) a pocket neighboring the catalytic center
which comprise a certain number of important amino acids.

The catalytic center comprises the amino acids Trp279, Cys314, Trp370, His373, Asp396 according to SEQ ID NO: 1. These residues are identified using the program Sybyl. Figure 2 depicts the catalytic center. The aforementioned amino acids might be important in regard to interactions with inhibitors and substrates.

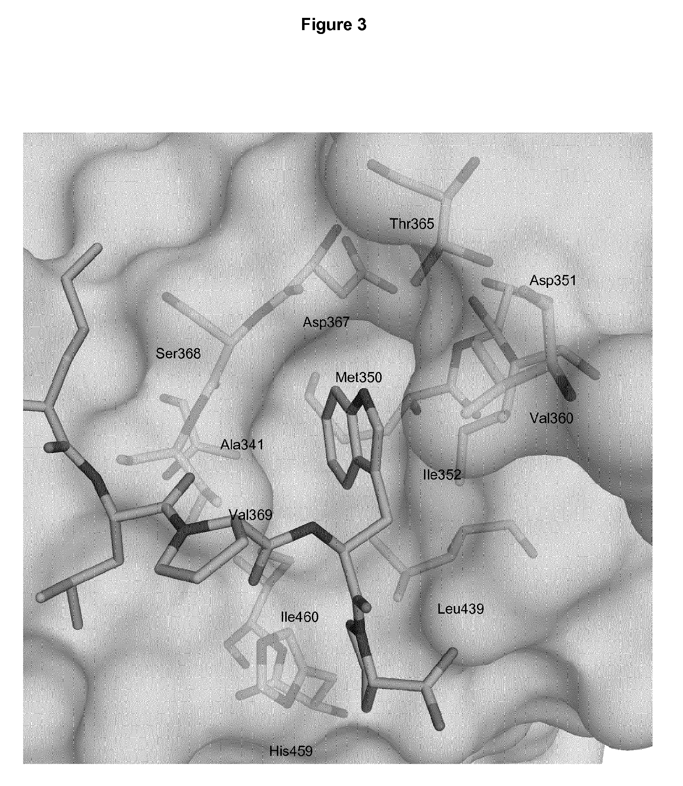

The hydrophobic pocket comprises the amino acids Ala341, Met350, Asp351, Ile352, Val360, Tyr365, Asp367, Ser368, Val369, Leu439, His459, Ile460 according to SEQ ID NO: 1. The hydrophobic pocket in detail is shown by figure 3. The aforementioned amino acids might be important in regard to interactions with inhibitors and substrates.

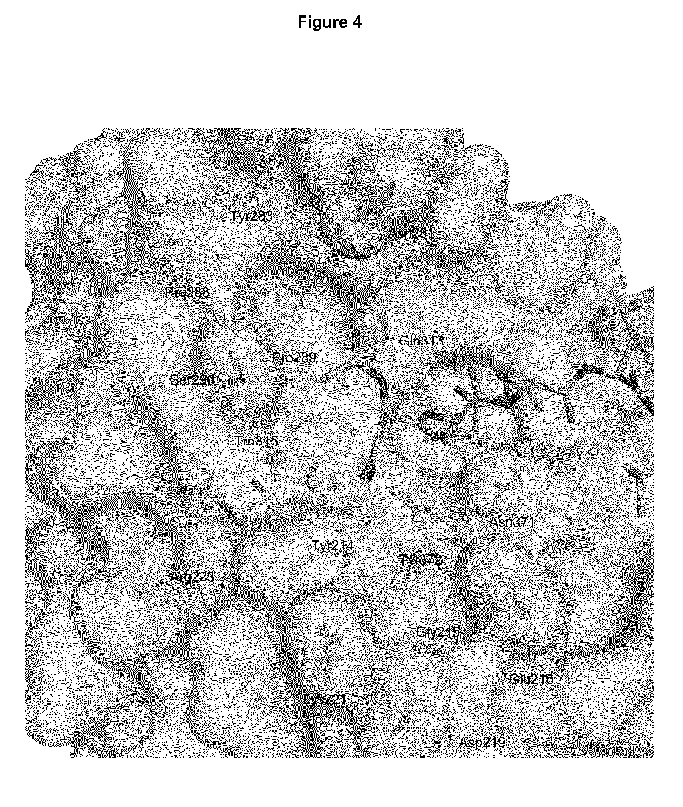

The surface between said catalytic center and hydrophobic pocket comprises the amino acids Phe339, His342, Ser368, Val369, Trp370, Asn371, His459 according to SEQ ID NO: 1. Figure 4 shows the surface between said catalytic center and hydrophobic pocket. The aforementioned amino acids might be important in regard to interactions with inhibitors and substrates.

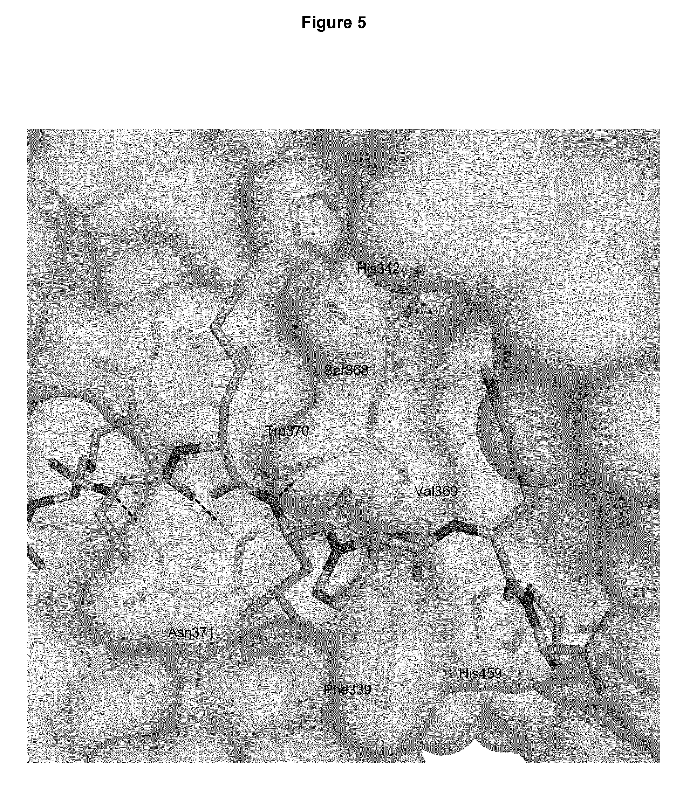

The pocket neighboring the catalytic center comprises the amino acids Tyr214, Gly2l5, Glu216, Asp219, Lys221, Arg223, Asn281, Tyr283, Pro288, Pro289, Gln313, Trp315, Asn371, Tyr372 according to SEQ ID NO: 1. Figure 5 depicts the pocket neighboring the catalytic center in detail. The aforementioned amino acids might be important in regard to interactions with inhibitors and substrates.

In a preferred embodiment of the present invention the crystal form of blood coagulation factor XIIIa contains a catalytic center and/or a pocket neighboring the catalytic center, wherein the catalytic center contains the amino acids Trp279, Cys314, Trp370, His373, and Asp396; and the pocket neighboring the catalytic center contains the amino acids Tyr214, Gly215, Glu216, Asp219, Lys221, Arg223, Asn281, Tyr283, Pro288, Pro289, Gln313, Trp315, Asn371, and Tyr372.

In another preferred embodiment of the present invention the crystal form of blood coagulation factor XIIIa contains or further contains a hydrophobic pocket and a surface between the catalytic center and the hydrophobic pocket, wherein the hydrophobic pocket contains the amino acids Ala341, Met350, Asp351, Ile352, Val360, Tyr365, Asp367, Ser368, Val369, Leu439, His459, and Ile460; and the surface between the catalytic center and the hydrophobic pocket contains the amino acids Phe339, His342, Ser368, Val369, Trp370, Asn371, and His459.

A potential inhibitor of blood coagulation factor XIIIa could bind to one or more of these four active sides in order to decrease the activity of blood coagulation factor XIIIa. Thus a method for designing, identifying and/or optimizing compounds which might have the ability to bind to blood coagulation factor XIIIa could be based on the crystal structure coordinates of one, two, three or four of these active sides or on the crystal structure coordinates of the whole crystal.

### Methods for crystallizing Blood Coagulation Factor Xllla

The crystal forms of blood coagulation factor XIIIa as disclosed herein can be obtained by the following crystallization methods.

Thus the present invention relates to a method for crystallizing blood coagulation factor XIIIa comprising the steps:
(a) providing an aqueous solution containing not more than 0.04 mM of blood coagulation factor XIII; and
(b) mixing an activation agent and an inhibitor with the aqueous solution of step (a) so that a molar ratio of inhibitor to blood coagulation factor XIII from 10:1 to 50:1 is obtained; and
(c) concentrating the solution of step (b); and
(d) diluting the concentrated solution of step (c) with a buffered precipitant solution containing between 10% by weight and 20% by weight of at least one small organic amphiphilic molecule and/or between 50 mM and 200 mM of at least one inorganic salt and/or between 20% by weight and 30% by weight of a precipitating agent so that the final concentration of the inhibited activated blood coagulation factor XIIIa is in the range of 1.0 mg/mL to 10 mg/mL.

The crystallization method starts with providing an aqueous solution of FXIII which is preferably a buffered solution having a pH in the range of 6.5 - 10.0, preferably of 7.0 - 9.5, more preferably 7.5 - 9.0, still more preferably 7.8 - 8.8 and most preferably 8.0 - 8.6. Suitable buffers are disclosed below. The concentration of blood coagulation factor XIII (FXIII) should not exceed 0.04 mM, preferably not 0.02 mM, more preferably not 0.01 mM, still more preferably not 0.005 mM and most preferably should not exceed 0.002 mM in the solution of step (a). Suitable concentration ranges of FXIII are from 0.005 µM to 0.04 mM, preferably from 0.01 µM to 0.02 mM, more preferably from 0.05 µM to 0.01 mM, still more preferably from 0.1 µM to 5.0 µM and most preferably from 0.5 µM to 2.0 µM. The monomer of the blood coagulation factor XIII (FXIII) has a molecular mass of about 84102 Dalton so that an equivalent concentration of FXIII ranges in the solution of step (a) is preferably from 0,1 mg/mL to 0,3 mg/mL. FXIII refers to the not activated form of blood coagulation factor XIII which is present in solution as a dimer while FXIIIa refers to the activated form of blood coagulation factor XIII.

Thus step (a) of the crystallization methods disclosed herein could also read as follows:
(a) providing an aqueous solution containing 0.005 µM to 0.04 mM of blood coagulation factor XIII;
or
(a) providing a buffered aqueous solution of a pH in the range of 7.0 to 9.5 containing not more than 0.04 mM of blood coagulation factor XIII;
or
(a) providing a buffered aqueous solution of a pH in the range of 7.0 to 9.5 containing 0.005 µM to 0.04 mM of blood coagulation factor XIII.

In step (b) an activation agent and an inhibitor are mixed with the solution of step (a) and preferably a solution and more preferably a buffered solution of an activation agent and an aqueous or organic solution of an inhibitor are combined with the solution of step (a).

The activation agent is a calcium salt which preferably dissociates completely or a mixture of calcium salts which preferably dissociate completely so that a concentration of Ca²⁺ ions in the solution of step (b) containing the FXllla is obtained in the range from 25 mM to 500 mM, more preferably from 40 mM to 300 mM, still more preferably from 45 mM to 200 mM and still more preferably from 50 mM to 150 mM Ca²⁺ ions. Since the upper value of the Ca²⁺ ion concentration is not critical or essential, the concentration of the Ca²⁺ ions in the solution of step (b) should be at least 25 mM, preferably at least 40 mM, more preferably at least 45 mM and still more preferably at least 50 mM.
Instead of Ca²⁺ ions a mixture of thrombin and Ca²⁺ ions can be used as activation agent in order to activate the blood coagulation factor XIII. If such a combination is used, the concentration of thrombin in the solution of step (b) is preferably from 500 - 2.000 NIH Units/mL and more preferably from 750 - 1500 NIH Units/mL or the concentration of thrombin in the solution of step (b) is preferably at least 500 NIH Units/mL and more preferably at least 750 NIH Units/mL and the concentration of Ca²⁺ ions is at least 1 mM, preferably at least 5 mM, more preferably at least 10 mM, still more preferably at least 20 mM and still more preferably at least 30 mM or in other words the concentration should be in the range from 1 mM - 250, preferably 3 mM - 100 mM and more preferably 5 mM - 30 mM. The concentration of thrombin defined as NIH Units is in accordance with the NIH Thrombin Reference Standard.

Suitable calcium salts are, for instance, CaCl₂, CaBr₂, calcium hydroxide, calcium glubionate, calcium gluceptate, calcium gluconate, calcium lactate, calcium malate, calcium fumarate, or mixtures thereof. Preferred is the use of CaCl₂.

The activation agent and/or the inhibitor can be added as dry sold substances to the solution of step (a) but are preferably added as an aqueous solution and more preferably as an buffered aqueous solution containing the activation agent and the inhibitor. It is obvious that also the inhibitor could be added to the solution, preferably buffered solution of the activation agent and the solution, preferably buffered solution of the FXIII is then added to the solution of activation agent and inhibitor. The sequence of addition of the components finally present in the solution of step (b) is not critical. However it is preferred to add the inhibitor dissolved in a small volume of an organic solvent miscible with water or in a water / organic solvent mixture to the solution of the activation agent and thereafter add to this solution the solution of FXIII. Suitable bufferes for these solutions are, for instance, Tris-HCl, and triethanolamine buffer. Preferably the same buffer as for the solution of step (a) is used for step (b). In case the desired pH value is not obtained adjusting the pH by addition of a weak or diluted base or a weak or diluted acid such as ammonium chloride, sodium acetate, sodium hydroxide, or potassium hydroxide, can be performed. By mixing the solution of the activation agent and the inhibitor with the solution of step (a) the concentrations of the solution of step (b) are obtained. Thus the concentration of the Ca²⁺ ions as stated above is the concentration of the solution containing the activation agent, the inhibitor and the FXIII / FXllla.

Step (b) of the crystallization methods disclosed herein could also read as follows:
(b) combining a calcium salt or a mixture of calcium salts as activation agent and an inhibitor with the aqueous solution of step (a) so that a concentration of Ca²⁺ ions from 50 mM to 150 mM and a molar ratio of inhibitor to blood coagulation factor XIII from 10:1 to 50:1 is obtained or combining a calcium salt or a mixture of calcium salts and thrombin as activation agent and an inhibitor with the aqueous solution of step (a) so that a concentration of Ca²⁺ ions from 5 mM to 30 mM and a concentration of thrombin from 500 - 2.000 NIH Units/mL and a molar ratio of inhibitor to blood coagulation factor XIII from 10:1 to 50:1 is obtained;
or
(b) combining a calcium salt or a mixture of calcium salts as activation agent and an inhibitor with the aqueous solution of step (a) so that a concentration of Ca²⁺ ions of at least 50 mM and a molar ratio of inhibitor to blood coagulation factor XIII from 10:1 to 50:1 is obtained or combining a calcium salt or a mixture of calcium salts and thrombin as activation agent and an inhibitor to the aqueous solution of step (a) so that a concentration of Ca²⁺ ions of at least 5 mM and a concentration of thrombin of at least 500 NIH Units/mL and a molar ratio of inhibitor to blood coagulation factor XIII from 10:1 to 50:1 is obtained.

Thus a further aspect of the present invention is directed to a method for crystallizing blood coagulation factor XIIIa comprising the steps:
(a) providing a buffered aqueous solution of a pH in the range of 7.0 to 9.5 containing not more than 0.04 mM of blood coagulation factor XIII; and
(b) combining at least one calcium salt as activation agent and an inhibitor with the aqueous solution of step (a) to obtain a concentration of Ca²⁺ ions from 50 mM to 150 mM and a molar ratio of inhibitor to blood coagulation factor XIII from 10:1 to 50:1;
   or combining at least one calcium salt and thrombin as activation agent and an inhibitor with the aqueous solution of step (a) to obtain a concentration of Ca²⁺ ions from 5 mM to 30 mM and a concentration of thrombin from 500 - 2.000 NIH Units/mL and a molar ratio of inhibitor to blood coagulation factor XIII from 10:1 to 50:1; and
(c) concentrating the solution of step (b); and
(d) diluting the concentrated solution of step (c) with a buffered precipitant solution containing between 10% by weight and 20% by weight of at least one small organic amphiphilic molecule and/or between 50 mM and 200 mM of at least one inorganic salt and/or between 20% by weight and 30% by weight of a precipitating agent so that a final concentration of the inhibited activated blood coagulation factor XIIIa of 1.0 mg/mL to 10 mg/mL is obtained.

In other words a further aspect of the present invention is directed to a method for crystallizing blood coagulation factor XIIIa comprising the steps:
(a) providing a buffered aqueous solution of a pH in the range of 7.0 to 9.5 containing not more than 0.04 mM of blood coagulation factor XIII; and
(b) combining at least one calcium salt as activation agent and an inhibitor with the aqueous solution of step (a) to obtain a concentration of Ca²⁺ ions of at least 50 mM and a molar ratio of inhibitor to blood coagulation factor XIII of at least 10:1;
   or combining at least one calcium salt and thrombin as activation agent and an inhibitor with the aqueous solution of step (a) to obtain a concentration of Ca²⁺ ions of at least 5 mM and a concentration of thrombin of at least 500 NIH Units/mL and a molar ratio of inhibitor to blood coagulation factor XIII of at least 10:1; and
(c) concentrating the solution of step (b); and
(d) diluting the concentrated solution of step (c) with a buffered precipitant solution containing between 10% by weight and 20% by weight of at least one small organic amphiphilic molecule and/or between 50 mM and 200 mM of at least one inorganic salt and/or between 20% by weight and 30% by weight of a precipitating agent so that a final concentration of the inhibited activated blood coagulation factor XIIIa of 1.0 mg/mL to 10 mg/mL is obtained.

The inhibitor is added to the solution of step (a) in an amount so that a molar ratio of inhibitor to blood coagulation factor XIII (not the activated form) is in the range from 2:1 to 100:1, preferably 5:1 to 75:1 and more preferably of 10:1 to 50:1 is obtained in the solution of step (b).

A single substantially pure inhibitor should be used having a purity of preferably above 90%. The use of mixtures of inhibitors is not preferred. Furthermore, if a solution of the inhibitor is added to the buffered aqueous solution of step (a), only relative small volumes should be added so that the concentrations defined for the blood coagulation factor XIII and the Ca²⁺ ions preferably remain still within the defined ranges. Suitable solvents for the inhibitor are water, DMSO, acetone, THF, DMF, methanol, ethanol or mixtures thereof. In case organic solvents are used, only small amount should be used such as 1 µL to 20 µL per mL of the solution obtained in step (b). The pH of the solution of step (b) containing the FXIII/FXIIIa, the inhibitor and the activation agent is in the range of 6.5 - 10.0, preferably of 7.0 - 9.5, more preferably 7.5 - 9.0, still more preferably 7.8 - 8.8 and most preferably 8.0 - 8.6.

After mixing an activation agent and an inhibitor or preferably a solution of an activation agent and a solution of an inhibitor with the aqueous solution of step (a), the obtained solution in step (b) is preferably smoothly stirred at room temperature and atmospheric pressure several hours or over night and preferably under exclusion of light.

Concerning the inhibitor used in step (b) reversible or irreversible inhibitiors leading to reversible or irreversible inhibition of blood coagulation factor XIIIa could be used. The few known inhibitors as mentioned herein could be used. Further examples of suitable inhibitors are compounds with a functional group which is able to form a covalent bond with a thiol group of an amino acid of blood coagulation factor XIIIa. Such compounds are irreversible inhibitors which usually covalently bind and thereby modify the enzyme permanently, and inhibition can therefore not be reversed. Reversible inhibitors have inhibition of enzyme activity in which the inhibiting molecular entity can associate and dissociate from the protein's active site. Covalent reversible inhibitors inactivate blood coagulation factor XIIIa probably by reacting with the active site cysteine thiol, resulting in the formation of a hemi-thioacetal, ketal or similar tetrahedral intermediate, the nature of which depends on the warhead. Reversible inhibitors of blood coagulation factor XIIIa contain reactive functional groups such as aldehydes, ketones, α-keto ketones, α-keto esters, α-keto amides, imines and hydrazones. Irreversible inhibitors of blood coagulation factor XIIIa contain reactive functional groups such as nitrogen mustards, haloalkanes, alkenes, Michael acceptors, phenyl sulfonates, or fluorophosphonates. These electrophilic groups react with amino acid side chains to form covalent adducts. The residues modified are those with side chains containing nucleophiles such as hydroxyl (-OH) or sulfhydryl groups (-SH). These include the amino acids serine, cysteine, threonine or tyrosine. Preferred inhibitors of blood coagulation factor XIIIa as disclosed in the present invention have Michael acceptors such as α,β-unsaturated carbonyl moieties. The blood coagulation factor XIIIa has cysteine (Cys314) in catalytic center which could covalently add to the Michael acceptor. Such irreversible inhibitors preferably contain a peptidic or peptidomimetic backbone for recognizing at least one of the active sites of blood coagulation factor XIIa and a side chain preferably attached to the alpha carbon atom of an amino acid or amino acid analogue of the peptidic or peptidomimetic backbone with the Michael acceptor moiety.

Examples of such inhibitors are compounds of the general forumula (I)

R¹-R²-Nle-R³-Leu-Pro-Trp-Pro-OH (I)

wherein
R¹ represents Ac-Asp or 4-oxopyrrolidine-2-carboxyl;
R² represents 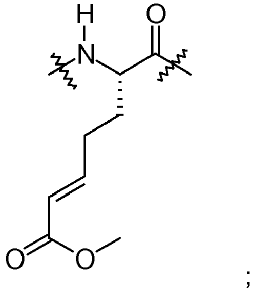
and R³ represents Nle or Ile.

In a preferred embodiment of the present invention an inhibitor **1** (compound **4**) is used, wherein R¹ is Ac-Asp and R³ is Nle. The crystal form of blood coagulation factor XIIIa is characterized as having a space group of P1 and two monomers of coagulation factor XIIIa in the asymmetric unit, with the unit cell dimension of a = 56.8 Å, b = 80.5 Å, c = 102.8 Å, α = 88.1°, β = 76.7° and γ = 82,0° or having a space group of P1, two monomers of coagulation factor XIIIa in the asymmetric unit and unit cell dimension of a = 56.6 Å, b = 64.2 Å, c = 67.1 Å, α = 76.4° and β = 71.7° and γ = 82.8°.

In another preferred embodiment of the present invention another inhibitor **2** (compound **7**) is used, wherein R¹ is 4-oxopyrrolidine-2-carboxyl and R³ is Ile. The crystal form of blood coagulation factor XIIIa is characterized as having a space group of C2 and two monomers of coagulation factor XIIIa in the asymmetric unit with the unit cell dimension of a = 90.2 Å, b = 64.8 Å, c = 70.4 Å, α = 90.0° and β = 95.8° and γ = 90,0°.

One crystal form of blood coagulation factor XIIIa is characterized by the atomic structure coordinates of figure 6 as set forth below.

The crystal form of the invention can be obtained by techniques well-known in the art of protein crystallography, including batch, liquid bridge, free interface diffusion, dialysis, vapor diffusion and hanging or sitting drop method.

Another aspect of the present invention is directed to a method for crystallizing blood coagulation factor XIIIa comprising the steps:
(a) providing an aqueous solution containing 0.005 µM to 0.04 mM of blood coagulation factor XIIIa; and
(b) activating the blood coagulation factor XIII and mixing an inhibitor with the aqueous solution of step (a) at the molar ratio of inhibitor to blood coagulation factor XIII from 10:1 to 50:1; and
(c) concentrating the solution of step (b) to a concentration of inhibited activated blood coagulation factor XIIIa of 2.0 mg/mL to 20 mg/mL; and
(d) diluting the concentrated solution of step (c) with a buffered precipitant solution containing between 10% by weight and 20% by weight of at least one small organic amphiphilic molecule and/or between 50 mM and 200 mM of at least one inorganic salt and/or between 20% by weight and 30% by weight of a precipitating agent so that a final concentration of the inhibited activated blood coagulation factor XIIIa of 1.0 mg/mL to 10 mg/mL is obtained.

Activation of FXIII can be achieved by means of Ca²⁺ ions or by means of thrombin in combination with Ca²⁺ ions.

A further aspect of the present invention is directed to a method for crystallizing blood coagulation factor XIIIa comprising the steps:
(a) providing a buffered aqueous solution of a pH in the range of 7.0 to 9.5 containing not more than 0.04 mM of blood coagulation factor XIII; and
(b) mixing a solution of a calcium salt or a solution of a calcium salt and thrombin as activation agent and an inhibitor with the aqueous solution of step (a) in order to obtain the molar ratio of inhibitor to blood coagulation factor XIII from 10:1 to 50:1; and
(c) concentrating the solution of step (b) to a concentration of inhibited activated blood coagulation factor XIIIa of 2.0 mg/mL to 20 mg/mL; and
(d) diluting the concentrated solution of step (c) with a buffered precipitant solution containing between 10% by weight and 20% by weight of at least one small organic amphiphilic molecule and/or between 50 mM and 200 mM of at least one inorganic salt and/or between 20% by weight and 30% by weight of a precipitating agent so that a final concentration of the inhibited activated blood coagulation factor XIIIa of 1.0 mg/mL to 10 mg/mL is obtained.

Preferably step (b) reads as follows:
(b) mixing a buffered solution of a calcium salt or a buffered solution of a calcium salt and thrombin as activation agent and an inhibitor with the aqueous solution of step (a) so that a molar ratio of inhibitor to blood coagulation factor XIII from 10:1 to 50:1 is obtained;

After the activation of FXIII to FXllla and inhibition of FXllla in step (b), the solution obtained in step (b) is then concentrated preferably by evaporation of water. After the concentration of the solution in step (c) a purification step can advantageously be included. Thus step (c) may actually consist of two steps, namely concentration of the solution and purification of the concentrated solution. The purification might be preferred in order to remove an excess of thrombin (if used) and/or inhibitor. Suitable purification methods such as chromatography, gel permeation chromatography and the like are well known to a skilled person. Moreover after the purification step dilution of the purified solution containing the inhibited FXllla might be necessary so that the purified solution is diluted with a dilution buffer solution before the buffered precipitant solution is added.

Thus in all crystallization methods disclosed herein, step (c) could read as follows:
(c) concentrating the solution of step (b) and purification of the concentrated solution;
or
(c) concentrating the solution of step (b) and purification of the concentrated solution to a concentration of inhibited activated blood coagulation factor XIIIa of 2.0 mg/mL to 20 mg/mL;
or
(c) concentrating the solution of step (b) and purification of the concentrated solution and dilution of the purified solution with water or an aqueous buffer solution;
or
(c) concentrating the solution of step (b) and purification of the concentrated solution to a concentration of inhibited activated blood coagulation factor XIIIa of 2.0 mg/mL to 20 mg/mL and dilution of the purified solution with water or an aqueous buffer solution;

The aqueous buffer solution for dilution contains one of the buffers disclosed herein and preferably a Tris-HCl buffer and has preferably a pH from 7.0 to 9.0, more preferably from 7.5 to 8.5.
After the concentration step (c) or the concentration and purification step (c) the concentration of the inhibited activated blood coagulation factor XIIIa should be in the range of 2.0 mg/mL to 20 mg/mL. This is the concentration of the activated and inhibited FXllla at the end of step (c) before dilution with the buffered precipitant solution of step (d) and before dilution with an aqueous buffer solution. The concentration of the activated and inhibited FXIIIa in the concentrated solution of step (c) or during the concentration in step (c) or after the concentration and purification step (c) could be determined, for instance, by the Bradford protein assay which is described in example 13.

In step (d) a buffered precipitant solution of at least one small organic amphiphilic molecule and/or at least one inorganic salt and/or at least one precipitating agent (also called precipitant) is added to the mixture of step (c). Preferably 2 µL of the concentrated and optionally purified solution of step (c) or the concentrated, purified and diluted solution of step (c) is diluted with 1.0 to 3.0 µL, preferably with 1.4 to 2.6 µL and more preferably with 1.8 to 2.2 µL of the buffered precipitant solution.

Preferably, the small organic amphiphilic molecule is selected from the group comprising or consisting of glycerol, ethylene glycol, butyl ether, benzamidine, dioxane, ethanol, isopropanol, butanol, pentanol, methyl pentanediol, pentanediol, hexanediol, heptanetriol, dithiothreitol, MES, Tris, Tris-HCl, Bis-Tris, imidazole, bicine, trimethylamine N-oxide, succinic acid, DL-malic acid, CHES, CAPS, glycine, CAPSO, ADA, MOPS, Bis-Tris propane, SPG, MIB, PCB, MMT, N-[2-hydroxyethyl]piperazine-N'-[2-ethanesulfonic acid] (HEPES), TRIS and sucrose. Preferred is glycerol as small organic amphiphilic molecule.

The term "at least one small organic amphiphilic molecule" means that mixtures of the afore-mentioned small organic amphiphilic molecules could be used. In case mixtures of small organic amphiphilic molecules are used, the amount used refers to the total amount of all small organic amphiphilic molecules together. The amount used of all small organic amphiphilic molecules together in the buffered precipitant solution is between 1% by weight and 50% by weight, preferably between 5% by weight and 30% by weight, and more preferably between 10% by weight and 20% of the small organic amphiphilic molecule or the mixture of small organic amphiphilic molecules in regard to the total weight of the buffered precipitant solution.

Moreover at least one inorganic salt is used in an amount of 10 mM and 600 mM, preferably 20 mM and 500 mM, more preferably 30 mM and 400 mM, more preferably 40 mM and 300 mM and most preferably 50 mM and 200 mM. In case more than one inorganic salt is used, the afore-mentioned concentration refers to the concentration of all inorganic salts together and not to the concentration of each single salt used in the mixture of inorganic salts.

Preferably, inorganic salts are selected from the group comprising or consisting of ammonium chloride, ammonium sulfate, ammonium acetate, ammonium fluride, ammonium bromide, ammonium iodide, ammonium nitride, cadmium chloride, cadmium sulfate, calcium chloride, calcium acetate, cesium chloride, cesium sulfate, cobalt chloride, ferric chloride, lithium acetate, lithium chloride, lithium nitrate, lithium sulfate, magnesium acetate, magnesium formate, magnesium nitrate, nickel chloride, potassium acetate, potassium bromide, potassium fluoride, potassium formate, potassium iodide, potassium nitrate, potassium thiocyanate, potassium/sodium tartrate, sodium acetate, sodium bromide, sodium fluoride, sodium iodide, sodium nitrate, sodium sulfate, sodium thiocyanate, zinc chloride, zinc sulfate, zinc acetate, magnesium chloride, sodium chloride, sodium cacodylate, sodium hydroxide, potassium chloride, potassium hydroxide, potassium sulfate, sodium acetate, sodium tartrate, ammonium formate, diammonium tartrate, sodium malonate, di-sodium tartrate, sodium succinate, and sodium succinate. A preferred inorganic salt is ammonium sulfate or sodium cacodylate or a mixture thereof.

In theory, the at least one precipitating agent competes with the protein solutes for water, thus leading to supersaturation of the proteins. Crystals can normally only grow from supersaturated states, and thus they can grow from precipitates. Salts, polymers, and organic solvents are suitable precipitating agents which are used in an amount of 5% by weight and 50% by weight, preferably between 10% by weight and 40% by weight, and more preferably between 15% by weight and 35%, and most preferably between 20% by weight and 30% of the precipitating agent or the mixture of precipitating agents in regard to the total weight of the buffered precipitant solution.

The precipitating agent used in the buffered precipitant solution of step (d) may preferably be selected from the group consisting of or comprising: 2-methyl-2,4 pentanediol, glycerol, sodium fluoride, calcium chloride, ammonium sulfate, lithium or sodium sulfate, lithium chloride, sodium or potassium chloride, sodium or ammonium acetate, ammonium chloride, cetyltrimethyl ammonium salt, polyethylene glycol (PEG), jeffamine, MPD, pentaerythritol propoxylate, pentaerythritol ethoxylate, sodium polyacrylate, hexandiol, isopropanol, ethanol, tert-butanol, dioxane, ethylene imine polymer, etylene glycol, propanediol, polyacrylic acid, polyvinylpyrrolidone, 2-ethoxyethanol, butandiol, or mixtures thereof. PEG is a polyether compound with, the structure of PEG is HO-CH₂-(CH₂-O-CH₂-)ₙ-CH₂-OH. PEG has tended to refer to oligomers and polymers with a molecular mass below 20,000 g/mol. Most preferred as the precipitant used in the method for crystallizing of the present invention is PEG, preferably having molecular weights range from PEG200 to PEG20,000, more preferably having molecular weights range from PEG 1,000 to PEG 18,000, yet more preferably having molecular weights range from PEG 5,000 to PEG 15,000 and most preferably having molecular weights range from PEG 7,000 to PEG 10,000. Organic solvents, like isopropanol or 1,3-propanediol may also be part of the reservoir solution. PEG is a very preferred precipitating agent which is preferably used in an amount of 20 - 30 % by weight of the buffered precipitant solution.

The hanging drop or the sitting drop methods are used for crystallization. "The hanging drop vapor diffusion" technique is the most popular method for the crystallization of macromolecules. The principle of vapor diffusion is straightforward. A drop composed of a mixture of sample and reagent is placed in vapor equilibration with a liquid reservoir of reagent. Typically the drop contains a lower reagent concentration than the reservoir. To achieve equilibrium, water vapor leaves the drop and eventually ends up in the reservoir. As water leaves the drop, the sample undergoes an increase in relative supersaturation. Both the sample and reagent increase in concentration as water leaves the drop for the reservoir. Equilibration is reached when the reagent concentration in the drop is approximately the same as that in the reservoir.

Thus another aspect of the present invention is directed to a method for crystallizing blood coagulation factor XIIIa comprising the steps:
(a) providing a buffered aqueous solution of a pH in the range of 7.0 to 9.5 containing not more than 0.04 mM of blood coagulation factor XIII; and
(b) mixing a solution of at least one calcium salt as activation agent and an inhibitor with the aqueous solution of step (a) to obtain a concentration of Ca²⁺ ions of at least 50 mM and a molar ratio of inhibitor to blood coagulation factor XIII of at least 10:1;
   or mixing a solution of at least one calcium salt and thrombin as activation agent and an inhibitor with the aqueous solution of step (a) to obtain a concentration of Ca²⁺ ions of at least 5 mM and a concentration of thrombin of at least 500 NIH Units/mL and a molar ratio of inhibitor to blood coagulation factor XIII of at least 10:1; and
(c) concentrating the solution of step (b) to a concentration of inhibited activated blood coagulation factor XIIIa of 2.0 mg/mL to 20 mg/mL; and
(d) diluting the concentrated solution of step (c) with a buffered precipitant solution containing between 10% by weight and 20% by weight of at least one small organic amphiphilic molecule and/or between 50 mM and 200 mM of at least one inorganic salt and/or between 20% by weight and 30% by weight of PEG so that a final concentration of the inhibited activated blood coagulation factor XIIIa of 1.0 mg/mL to 10 mg/mL is obtained.

Step (c) may comprise a purification and also an optional dilution step. If an aqueous buffer solution is used to dilute the concentrated and purified solution of step (c) preferably the same buffer as used in step (a) and/or (b) is used again which is preferably a Tris-HCl buffer of a pH between 7.0 and 9.0. The buffered precipitant solution is preferably prepared at room temperature and under atmospheric pressure which is than added to the concentrated or concentrated and purified or to the concentrated, purified and diluted solution of step (c).

The following buffers could be used in step (a) for preparing the buffered aqueous solution as well as in step (b) if the inhibitor and/or the activation agent is added as a buffered aqueous solution and in step (d) for preparing the buffered precipitant solution:

**Table 1: Buffers:**

| **effective pH range** | **pKa 25°C** | **buffer** |
|---|---|---|
| 5.0-7.4 | 6.27 | cacodylate |
| 5.5-6.5 | 5.64 | succinate (pK2) |
| 5.5-6.7 | 6.10 | MES |
| 5.5-7.2 | 6.24 | maleate (pK2) |
| 5.5-7.4 | 1.70, 6.04, 9.09 | histidine |
| 5.8-7.2 | 6.46 | bis-tris |
| 6.0-12.0 | 9.50 | ethanolamine |
| 6.0-7.2 | 6.59 | ADA |
| 6.1-7.5 | 6.78 | ACES |
| 6.1-7.5 | 6.76 | PIPES |
| 6.2-7.6 | 6.87 | MOPSO |
| 6.2-7.8 | 6.95 | imidazole |
| 6.3-9.5 | 6.80, 9.00 | BIS-TRIS propane |
| 6.4-7.8 | 7.09 | BES |
| 6.5-7.9 | 7.14 | MOPS |
| 6.8-8.2 | 7.48 | HEPES |
| 6.8-8.2 | 7.40 | TES |
| 6.9-8.3 | 7.60 | MOBS |
| 7.0-8.2 | 7.52 | DIPSO |
| 7.0-8.2 | 7.61 | TAPSO |
| 7.0-8.3 | 7.76 | triethanolamine (TEA) |
| 7.1-8.5 | 7.85 | HEPPSO |
| 7.2-8.5 | 7.78 | POPSO |

Preferred are the buffers having an effective pH range of from 6.0 to 9.5, and more preferred of from 7.0 to 8.5. The effective pH range for each buffer can be defined as pKa - 1 to pKa + 1, where Ka is the ionization constant for the weak acid in the buffer and pKa = - log K. Thus buffers are preferred having a pKa from 7.0 to 8.0.

The buffered aqueous solutions of step (a), (b) and (d) of the crystallization method are preferably prepared at room temperature and under atmospheric pressure. Preferably these aqueous solutions should not contain substances which denaturate the blood coagulation factor XIII or its activated and inhibited form. The solutions of step (a) and (b) should not contain substances which complex calcium or form slightly water soluble calcium salts. Thus these aqueous solutions should, for instance, not contain substances such as complexing agents, EDTA, EGTA, sulfides, citrates, phosphates, carbonates, hydrogen peroxide, acrylamide, acrylates, metacrylates, which might interact or denaturate blood coagulation factor XIIIa or its activated and inhibited form or might form hardly water soluble calcium salts.

However detergens or at least one detergent might be present in these solutions. Preferred detergents are selected from the group comprising nonionic detergents or sugar-containing detergents such as n-octyl-β-D-glucopyranoside (β-OG), n-octanoylsucrose, DDM (dodecyl maltoside), alkyl oligooxyethylenes, C₈E₅, and polydisperse polyoxylethylene, Triton and zwitterionic detergents (amphotheric detergents) comprising sulfobetaines such as zwittergent, and betaines, and cationic detergents comprising alkyldimtheylamine oxides, such as lauryldimehtylamine oxide (LDAO), and the CHAPS series such as CHAPS. Preferably, the detergent is a cationic detergent. More preferably the detergent is CHAPS.
The term "at least one detergent" means that mixtures of the afore-mentioned detergents could be used. In case mixtures of detergents are used, the amount used refers to the total amount of all detergents together. The amount used of all detergents together in the buffered thrombin solution is preferably between 1 mM and 50 mM, preferably between 1 mM and 30 mM, and more preferably between 1 mM and 10 mM of the detergent or the mixture of detergents in regard to the total volume of the buffered thrombin solution.
The complete crystallization method is performed at room temperature under atmospheric pressure. Exclosure of light could be advantageous.

Another important aspect of the present invention is directed to the crystal forms of blood coagulation factor XIIIa obtainable by any one of the methods for crystallizing blood coagulation factor XIIIa as disclosed in the present invention.

### Rational Drug Design by use of structure of Blood Coagulation Factor XIIIa

Further important aspects of the invention are related to the use of the crystal forms of blood coagulation factor XIIIa for the determination of the three-dimensional structure of blood coagulation factor XIIIa and for the identification, optimization and/or design of inhibitors of blood coagulation factor XIIIa.

Knowing the exact positions of the atoms of the amino acids in the active sides and especially in the catalytic center provides the possibility to design suitable inhibitors, identify suitable inhibitors e.g. from a compound library or optimize a known suitable inhibitor by increasing the inhibitory potential. Design, identification and optimization of suitable inhibitors can be performed with standard computer based methods and software programs well known in the art.

A variety of commercially available software programs are available for conducting the analysis and comparison of data in the computer-based system. One skilled in the art will readily recognize which of the available algorithms or implementing software packages for conducting computer analyses can be utilized or adapted for use in the computer-based system. A target structural motif or target motif refers to any rationally selected sequence or combination of sequences in which the sequence(s) are chosen based on a three-dimensional configuration or electron density map which is formed upon the folding of the target motif. There are a variety of target motifs known in the art. Protein target motifs include, but are not limited to, enzymatic active sites, structural subdomains, epitopes, functional domains and signal sequences. A variety of structural formats for the input and output means can be used to input and output the information in the computer-based systems of the present invention.

A variety of comparing means can be used to compare a target sequence or target motif with the data storage means to identify structural motifs or interpret electron density maps derived in part from the atomic coordinate/x-ray diffraction data. One skilled in the art can readily recognize any one of the publicly available computer modeling programs that can be used.

Suitable software that can be used to view, analyze, design, and/or model a protein comprise MOLOC (Roche,1985), FRED, MAIN, FlexX, Gold, XtalView, Alchemy TM, LabVision TM, Sybyl TM, Molcadd TM, Leapfrog TM, Matchmaker TM , Genefold TM and Sitel TM (available from Tripos Inc., St. Louis, MO.); Quanta TM, Cerius2 TM, X-Plor TM, CNS TM, Catalyst TM , Modeller TM, ChemX TM, Ludi TM, Insight TM, Discover TM, Cameleon TM and Iditis TM (available from Accelrys Inc., Princeton NJ); Rasmol TM (available from Glaxo Research and Development, Greenford, Middlesex, U.K.); MOE TM (available from Chemical Computing Group, Montreal, Quebec, Canada); Maestro TM (available from Shrödinger Inc.,); COOT / CCP4; Midas/MidasPlus TM (available from UCSF, San Francisco, CA); JyMol, Pymol (Schrödinger LLC); Jmol (freeware on the intemet); VRML, (webviewer- freeware on the internet); Chime (MDL - freeware on the intemet); AutoDock (available from The Scripps Research Institute); MOIL (available from University of Illinois, Urbana-Champaign, IL); MacroModel TM and GRASP TM (available from Columbia University, New York, NY); Ribbon TM (available from University of Alabama, Tuscaloosa, Alabama); NAOMI TM (available from Oxford University, Oxford, UK); Explorer Eyechem TM (available from Silicon Graphics Inc., Mountain View, CA.); Univision TM (available from Cray Research Inc., Seattle WA); Molscript TM and O (available from Uppsala University, Uppsala, Sweden); Chem 3D TM and Protein Expert TM (available from Cambridge Scientific); and upgraded versions thereof.

Thus in a further aspect the present invention is related to methods for designing, identifying and optimizing inhibitors of blood coagulation factor XIIIa by applying the crystal form and the related structure coordinates of the crystal form or at least of one of the actives sides in order to design, identify or optimize inhibitors by means of computer based methods or software programs.

The atomic coordinate/x-ray diffraction data may be used to create a physical model which can then be used to design molecular models of compounds that should have the ability or property to inhibit and/or interact with the determined active sites or other structural or functional domains or subdomains of coagulation factor XIIIa such as the catalytic center, the hydrophobic pocket, the surface between said catalytic center and hydrophobic pocket and/or the pocket neighboring the catalytic center. Alternatively, the atomic coordinate/x-ray diffraction data of the complex may be represented as atomic model output data on computer readable media which can be used in a computer modeling system to calculate different molecules expected to inhibit and/or interact with the determined active sites, or other structural or functional domains or subdomains of the coagulation factor XIIIa. For example, computer analysis of the data allows one to calculate the three-dimensional interaction of the coagulation factor XIIIa and the compound to confirm that the compound binds to, or changes the conformation of, particular domain(s) or subdomain(s) of coagulation factor XIIIa. Compounds identified from the analysis of the physical or computer model can then be synthesized and tested for biological activity with an appropriate assay.

Thus the present invention is also directed to a method for designing a compound that interacts with or inhibits blood coagulation factor XIIIa, comprising the steps of
(a) generating a three-dimensional model of blood coagulation factor XIIIa using the structure coordinates as listed in figure 6; and
(b) employing said three-dimensional model to design a compound that should have the ability to interact with or inhibit blood coagulation factor XIIIa.

The generation of a three-dimensional model of blood coagulation factor XIIIa in accordance with step (a) of said method could also involve the application of a root mean square deviation so that a preferred method comprises the following steps:
(a) generating a three-dimensional model of blood coagulation factor XIIIa using the structure coordinates as listed in figure 6 and a root mean square deviation from the backbone atoms or from the alpha carbon backbone atoms of said amino acids of not more than 3.0 Å; and
(b) employing said three-dimensional model to design a compound that should have the ability to interact with or inhibit blood coagulation factor XIIIa.

The term "root mean square deviation" means the square root of the arithmetic mean of the squares of the deviations from the mean. It is a way to express the deviation or variation from a trend or object. For purposes of this invention, the "root mean square deviation" defines the variation in the backbone of a protein from the backbone of FXllla, an active site, a binding pocket, or a portion thereof, as defined by the structure coordinates of FXllla described herein. It would be apparent to the skilled person that calculation of root mean square deviation involves a standard error.

A preferred embodiment of the present invention refers to the methods disclosed herein, wherein the root mean square deviation from the backbone atoms of said amino acids of not more than 2.0 Å and more preferably not more than 1.0 Å. The interaction of the selected compound with the blood coagulation factor XIIIa leads most preferably to inhibition.

The term "compound" as used herein preferably refers to a small chemical molecule but could also refer to a fragment of a molecule such a a fragment of an antibody.
The above method refers to the design of a compound on the basis of the crystal structure coordinates of a crystal form of FXIIIa which should have the ability to interact with or preferably inhibit blood coagulation factor XIIIa.. Such a designed compound could be a completely novel compound, thus not existing before or could be a derivative of a known compound, for instance, derived by scaffold hopping. In order to verify the inhibitory activity of the compound, actual synthesis of the compound is desired with subsequent testing of the compound in a suitable assay in order to determine the inhibitory activity of FXIIIa. Examples 10 provides a suitable assay for testing the inhibitory effect of potential inhibitors of FXIIIa.

Thus the above method preferably further comprises the step of
(c) obtaining the designed compound; and
(d) contacting the obtained designed compound with blood coagulation factor XIIIa in order to determine the inhibitory effect on blood coagulation factor XIIIa.

For screening purposes of a compound library in order to identify an inhibitor of FXllla or for optimization of a know inhibitor of FXllla in order to increase the inhibitory potential of this known inhibitor, the following method for identifying and/or optimizing a compound that inhibits or should inhibit blood coagulation factor XIIIa is provided, comprising the steps of
(a) generating a three-dimensional model of blood coagulation factor XIIIa using the structure coordinates as listed in figure 6; and
(b) employing said three-dimensional model to identify or optimize a compound that should have the ability to inhibit FXIIIa.

Also in this method, step (a) could involve the root mean square deviation and thus read as follows:
(a) generating a three-dimensional model of blood coagulation factor XIIIa using the structure coordinates as listed in figure 6, wherein the root mean square deviation of the backbone atoms of the blood coagulation factor XIIIa amino acid residues is not more than 3 Å.

In case an inhibitor is identified from a compound library or in case a known inhibitor is optimized by theoretical chemical modifications, testing of the actual compound is desired in order to verify the inhibitory effect and to continue the optimization process. Thus preferably the above method for identifying and/or optimizing a compound further comprising the steps of
(c) obtaining the identified or optimized compound; and
(d) contacting the identified or optimized compound with blood coagulation factor XIIIa in order to determine the inhibitory effect on blood coagulation factor XIIIa.

It is not necessary to use all the structure coordinates as listed in figure 6, it might also be sufficient to use a selected set of structure coordinates as presented in figure 6. Thus only the structure coordinates of the catalytic center, the hydrophobic pocket, the surface between said catalytic center and hydrophobic pocket and/or the pocket neighboring the catalytic center could be used. Moreover it might also not to be necessary to generate a three-dimensional model of the complete blood coagulation factor XIIIa. It could also be sufficient to generate a model of one, two, three or all four active sides of FXllla and more preferably to generate a model of the catalytic center of FXIIIa.

Thus another aspect of the present invetion is directed to a method for designing, identifying or optimizing a compound which should have the ability to inhibit FXllla, wherein the three-dimensional model of the catalytic center and/or of the pocket neighboring the catalytic center and/or of the hydrophobic pocket and/or of the surface between the catalytic center and the hydrophobic pocket of the blood coagulation factor XIIIa is generated by using the structure coordinates as listed in figure 6 and wherein said three-dimensional model is employed to design, identify or optimize a compound that should have the ability to interact with or bind to the catalytic center, the pocket neighboring the catalytic center, the hydrophobic pocket and/or respectiviely the surface between the catalytic center and the hydrophobic pocket. Such a compound should have the ability to inhibit FXllla.

In some instances it may be particulary advantageous to delete and / or exchange and/or add amino acids or even complete domains to the native Factor XIII. This may be particulary interesting with regard to at least one of the beta barrel domains. However, since the catalytic domain is the central element of the present invention the structure of the active site is the major claim and the prerequisite of the structure based drug design. Basically, we also claim shorter variants of Factor XIII comprising the amino acids necessary to form at least the active site.

### Biological Activities of Blood Coagulation Factor XIIIa Inhibitors

Kinetic data of the inhibitor 1 (compound 4) and inhibitor 2 (compound 7) using the commercial fluorogenic assay kit F001 (Zedira) show an IC₅₀ of about 0.11 and 0.08 µM respectively.

Subsequently, selectivity of the blocker for the active site cysteine was confirmed. Coagulation factor XIII has nine free cysteine residues per subunit; none of them forms disulfide bonds. The inhibited factor XIIIa was digested by trypsin and the fragments were analyzed using mass spectrometry. As expected, only Cys314 reacts with the inhibitor since the active site thiol is most nucleophilic due to the catalytic triade.

The following abbreviations are used for the common and modified amino acids referred to herein.

| Abbreviation | Amino acid |
|---|---|
| Ala | Alanine |
| Arg | Arginine |
| Asn | Asparagine |
| Asp | Aspartic acid (Aspartate) |
| Cys | Cysteine |
| Gln | Glutamine |
| Glu | Glutamic acid (Glutamate) |
| Gly | Glycine |
| His | Histidine |
| Ile | Isoleucine |
| Leu | Leucine |
| Lys | Lysine |
| Met | Methionine |
| Nle | Norleucine |
| Phe | Phenylalanine |
| Pro | Proline |
| Ser | Serine |
| Sec | Selenocysteine |
| Thr | Threonine |
| Trp | Tryptophan |
| Tyr | Tyrosine |
| Val | Valine |

### Description of the figures

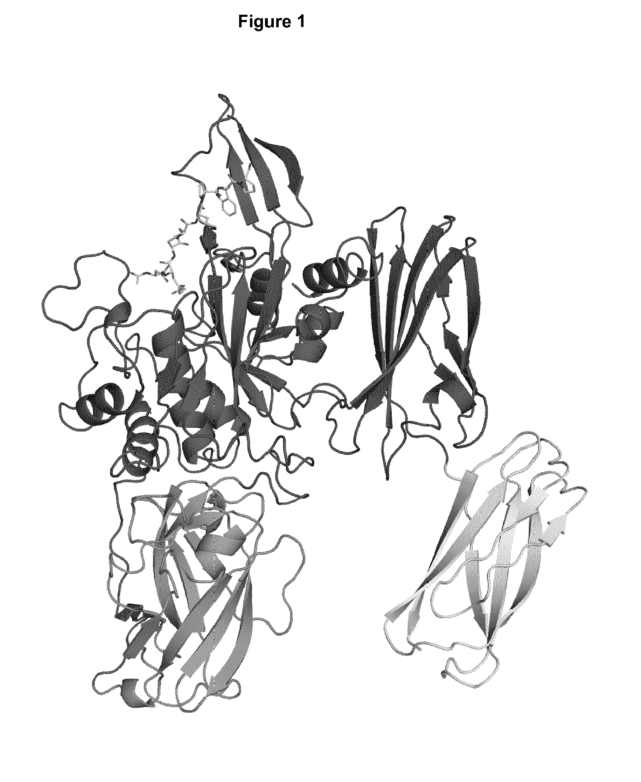

**Figure 1****:** A Ribon diagram of the overall fold of blood coagulation factor XIIIa with inhibitor 1 which is identical to compound 4. The following abbreviations are used:
   "Atom type" refers to the element whose coordinates are measured. The first letter in the column defines the element. "Resid" refers to the amino acid residue identity in the molecular model. "#" refers to the sequence number of amino acid residue. "X, Y, Z" define the atomic position of the element measured. "B" is a thermal factor that measures movement of the atom around its atomic center. "Occ" is an occupancy factor that refers to the fraction of the molecules in which each atom occupies the position specified by the coordinates. A value "1" indicates that each atom has the same conformation, i.e., the same position, in the molecules. "Mol" refers to the molecule in the asymmetric unit.
**Figure 2****:** A detailed represention of the catalytic site of blood coagulation factor XIIIa showing the essential amino acids Trp279, Cys314, Trp370, His373, Asp396.
**Figure 3****:** A detailed represention of the hydropobic pocket of blood coagulation factor XIIIa showing the essential amino acids Ala341, Met350, Asp351, Ile352, Val360, Tyr365, Asp367, Ser368, Val369, Leu439, His459, Ile460.
**Figure 4****:** A detailed represention of the pocket neighboring the catalytic center of blood coagulation factor XIIIa showing the essential amino acids Phe339, His342, Ser368, Val369, Trp370, Asn371, His459.
**Figure 5****:** A detailed represention of the surface between catalytic site and hydrophobic pocket of blood coagulation factor XIIIa showing the essential amino acids Tyr214, Gly215, Glu216, Asp219, Lys221, Arg223, Asn281, Tyr283, Pro288, Pro289, Gln313, Trp315, Asn371, Tyr372.
**Figure 6****:** The atomic structure coordinates for blood coagulation factor XIIIa having a space group of P1, two monomers of coagulation factor XIIIa in the asymmetric unit and unit cell dimension of a = 56.8 Å, b = 80.5 Å, c = 102.8 Å, α = 88.1°, β = 76.7° and γ = 82.0° as derived by X-ray diffraction.
Figure 7**:** (a) Component of a crystal. The asymmetric unit is that part of the crystal that shows no symmetry. A symmetry operator (for example, a C2 axis) generates the lattice motif. Repeating this motif by translation generates the corner of the unit cell, which is the basic repeating unit of the crystal lattice; (b) unit cell definition.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.
Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### EXAMPLES

Abbreviations used in the present description have the following meanings:
- 2CT: 2-Chlorotrityl
- ACN: Acetonitrile
- Boc: tert-Butyloxycarbonyl
- DIPEA: N,N-Diisopropylethylamine
- DMF: Dimethylformamide
- DMSO: Dimethyl sulfoxide
- Fmoc: Fluorenylmethyloxycarbonyl
- HATU: 2-(7-Aza-1H-benzotriazole-1-yl)-1,1,3,3-tetramethyluronium hexafluorophosphate
- HOBt: Hydroxybenzotriazole
- MAP: Michael acceptor pharmacophore
- OtBu: tert-butyl ester
- TBTU: O-(Benzotriazol-1-yl)-N,N,N',N'-tetramethyluronium tetrafluoroborate
- THF: Tetrahydrofuran

### PREPARATIVE EXAMPLES

### General considerations

NMR spectra were recorded on a Bruker DRX 500 (DMSO-d₆, 500 MHz). The following abbreviations were used to explain the multiplicities: s) singlet, d) doublet, t) triplet, m) multiplet, bs) broad singlet. ESI mass spectra were recorded on a Shimadzu LCMS-2020. Characterization of the purity of the compounds by LC-analysis: Merck-Hitachi LaChrom L-7100, Phenomenex Kinetex C18 column (2.6 µm, 3.0x100 mm), gradient from 16%B to 76%B with 4%/min, 0.6 ml/min (A-eluent: 0.01 % TFA in water, B-eluent: 0.01 % TFA in ACN/H₂0 9/1, 40°C), UV detection at 214 nm. Preparative HPLC was carried out on a Varian ProStar system: Phenomenex Synergi Max-RP 80A column (4 µm, 21.2x250 mm), 8 ml/min, varying gradients (A-eluent: 0.01% TFA in water, B-eluent: 0.01% TFA in ACN/H₂0 9/1, room temperature), UV detection at varying wave lengths. Column chromatography was performed using silica gel 60 from Macherey-Nagel (0.04-0.063 mm). Alumina sheets from Merck, coated with silica gel 60 F254 were used for thin-layer chromatography (TLC) using UV light as a visualizing agent and ninhydrine staining respectively.

All reagents were used as obtained from the supplier. All reactions were carried out under argon or nitrogen atmosphere.

The solid phase reactions were performed in a 250 ml flask equipped with a glass frit (por. 1) and a receiving flask with vacuum connection at room temperature. The peptide sequences were built by standard Fmoc solid-phase peptide synthesis (reactions in DMF, coupling with TBTU/HOBt/DIPEA, deprotection with piperidine). Cleavage from the resin was accomplished by using 95% TFA / 2.5 % water / 2.5 % triisopropylsilane). Coupling reactions to primary amines were monitored by the Kaiser test, couplings to secondary amines by the chloranil test.^{[1]} H-Pro-2CT-resin as starting material was purchased from Iris Biotech GmbH. All amino acids and coupling reagents were purchased from GL Biochem except Ac-Asp(OtBu)-OH (Bachem).

### Example 01: Preparation of (S,E)-2-(tert-Butoxycarbonylamino)-7-methoxy-7-oxohept-5-enoic acid, Boc-MAP-OH (compound 1)

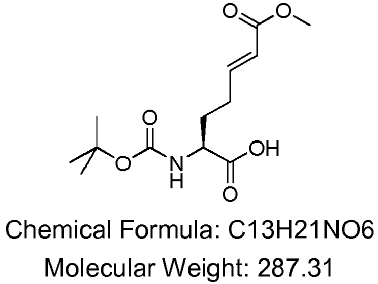

The synthesis of compound **1** was reported earlier.

### Example 02: Preparation of (S)-1-((S)-2-((S)-1-((8S,11S,14S,17S,E)-8-Amino-11,14-dibutyl-17-isobutyl-3,9,12,15-tetraoxo-2-oxa-10,13,16-triazaoctadec-4-ene)pyrrolidine-2-carboxamido)-3-(1H-indol-3-yl)propanoyl)pyrrolidine-2-carboxylic acid trifluoroacetate (compound 2)

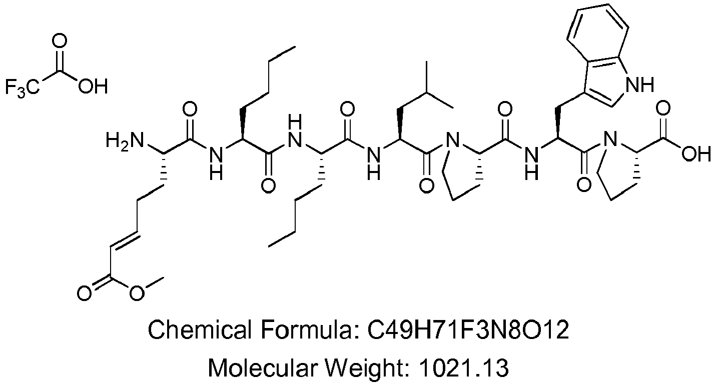

Compound **2** was synthesized by standard Fmoc solid-phase peptide synthesis as described above by using 2.00 g (1.50 mmol) H-Pro-2CT-resin (0.75 mmol/g) and 2.37 g (4.50 mmol) Fmoc-Trp(Boc)-OH, 1.52 g (4.50 mmol) Fmoc-Pro-OH, 1.59 g (4.50 mmol) Fmoc-Leu-OH, 1.59 g (4.50 mmol) Fmoc-Nle-OH (twice), 0.86 g (3.00 mmol) Boc-MAP-OH (1). For each coupling step TBTU (2.94 or 1.96 eq), HOBt (3 or 2 eq) and DIPEA (7 or 5 eq) were used, depending on the amount of used amino acid.
Compound **2** was obtained as a pale yellow solid (1.24 g, 1.21 mmol, 81%) and used without further purification.

### Example 03: Preparation of (S)-1-((S)-2-((S)-1-((6R,9S,12S,15S,18S)-6-acetamido-12,15-dibutyl-9-((E)-5-methoxy-5-oxopent-3-enyl)-2,2,20-trimethyl-4,7,10,13,16-pentaoxo-3-oxa-8,11,14,17-tetraazahenicosanecarbonyl) pyrrolidine-2-carboxamido)-3-(1H-indol-3-yl)propanoyl)pyrrolidine-2-carboxylic acid (compound 3)

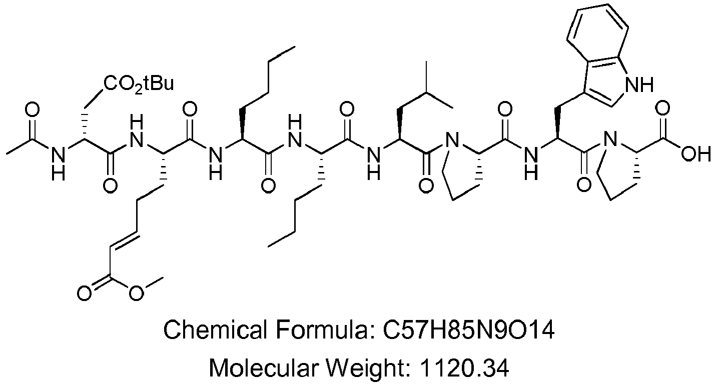

1.24 g (1.21 mmol) **2** were dissolved in 10 ml DMF. To this solution 280 mg (1.21 mmol) Ac-Asp(OtBu)-OH, 460 mg (1.21 mmol) HATU and 617 µl (3.63 mmol) DIPEA in 10 ml DMF were added. The resulting solution was set to pH 8.0 (DIPEA) and stirred at room temperature overnight. The solvent was removed under reduced pressure and the residue was purified by preparative HPLC (gradient from 50%B to 90%B with 1 %/min, 220 nm).
Compound **3** was obtained as a pale yellow solid (387 mg, 0.35 mmol, 28%).
ESI-MS: 1120.9 [M+H]⁺

### Example 04: Preparation of (S)-1-((S)-2-((S)-1-((4R,7S,10S,13S,16S)-10,13-d ibutyl-4-(carboxymethyl)-7-((E)-5-methoxy-5-oxopent-3-enyl)-18-methyl-2,5,8,11,14-pentaoxo-3,6,9,12,15-pentaazanonadecanecarbonyl)pyrrolidine-2-carboxamido)-3-(1H-indol-3-yl)propanoyl)pyrrolidine-2-carboxylic acid (compound 4)

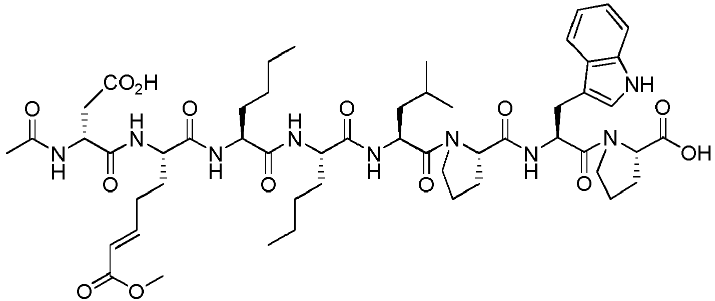

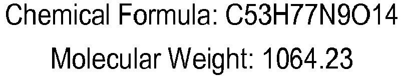

224 mg (0.20 mmol) **3** and 300 µl triisopropylsilane were dissolved in 30 ml dichloromethane. 15 ml TFA were added. The resulting solution was stirred for 3 h at room temperature before the solvent was removed under reduced pressure. The residue was purified by preparative HPLC (gradient from 40%B to 75%B with 1 %/min, 254 nm).
**Compound 4** was obtained as a colourless solid (154 mg, 0.14 mmol, 72%).

ESI-MS: 1064.8 [M+H]⁺

¹H-NMR (DMSO-d₆, 500 MHz): δ[ppm] = 0.82 (m, 2xNle-CH₃), 0.83, 0.86 (m, Leu-CH₃), 1.22 (m, 2xNle-χ-CH₂), 1.22 (m, 2xNle-δ-CH₂), 1.36, 1.40 (m, Leu-β-CH₂), 1.44, 1.59 (m, 2xNle-β-CH₂), 1.58 (m, Leu-χ-CH), 1.60, 1.80 (m, MAP-β-CH₂), 1.77, 1.80, 1.94, 2.10 (m, 2xPro-β-CH₂), 1.80 (m, 2xPro-χ-CH₂), 1.84 (s, Acetyl-3H), 2.17 (m, MAP-χ-CH), 2.48, 2.64 (m, Asp-CH₂), 2.90, 3.10 (m, Trp-β-CH₂), 3.26, 3.42, 3.53, 3.59 (m, 2xPro-δ-CH₃), 3.64 (m, MAP-CH₃), 4.19, 4.23 (m, 2xNle-CH), 4.22 (m, MAP-α-CH), 4.22, 4.33 (m, 2xPro-CH), 4.49 (m, Leu-α-CH), 4.53 (m, Asp-CH, ³J_{αH-βH1} = 5.59 Hz, ³J_{αH-βH2} = 8.02 Hz), 4.69 (m, Trp-CH), 5.83 (d, MAP-ε-CH, J = 15.57 Hz), 6.87 (dt, MAP-δ-CH, J = 6.90, 15.75 Hz), 6.95-7.57 (5xTrp-Ar-CH), 7.72, 7.94 (d, 2xNle-NH, J = 8.12, 8.82 Hz), 7.87 (d, MAP-NH, J = 8.00 Hz), 7.87 (d, Leu-NH, J = 8.00 Hz), 7.96 (d, Trp-NH, J = 8.47 Hz), 8.18 (d, Asp-NH, J = 7.69 Hz), 12.34 (bs, Asp-COOH).

### Example 05: Preparation of (S)-1-((S)-2-((S)-1-((8S,11S,14S,17S,E)-8-Amino-14-sec-butyl-11-butyl-17-isobutyl-3,9,12,15-tetraoxo-2-oxa-10,13,16-triazaoctadec-4-ene)pyrrolidine-2-carboxamido)-3-(1H-indol-3-yl)propanoyl)pyrrolidine-2-carboxylic acid trifluoroacetate (compound 5)

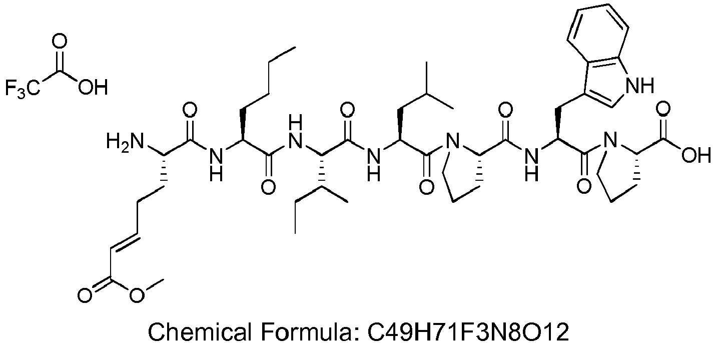

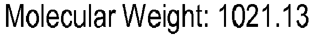

Compound **5** was synthesized by standard Fmoc solid-phase peptide synthesis as described above by using 500 mg (0.30 mmol) H-Pro-2CT-resin (0.75 mmol/g) and 474 mg (0.90 mmol) Fmoc-Trp(Boc)-OH, 304 mg (0.90 mmol) Fmoc-Pro-OH, 318 mg (0.90 mmol) Fmoc-Leu-OH, 318 mg (0.90 mmol) Fmoc-Ile-OH, 318 mg (0.90 mmol) Fmoc-Nle-OH, 172 mg (0.60 mmol) Boc-MAP-OH (1). For each coupling step TBTU (2.94 or 1.96 eq), HOBt (3 or 2 eq) and DIPEA (7 or 5 eq) were used, depending on the amount of used amino acid.
Compound **5** was obtained as a white solid (221 mg, 0.22 mmol, 72%) and used without further purification.

### Example 06: Preparation of (S)-1-((S)-2-((S)-1-((8S, 11S, 14S, 17S,E)-8-((S)-1-(tert-butoxycarbonyl)-4-oxopyrrolidine-2-carboxamido)-14-sec-butyl-11-butyl-17-isobutyl-3,9,12,15-tetraoxo-2-oxa-10,13,16-triazaoctadec-4-ene)pyrrolidine-2-carboxamido)-3-(1H-indol-3-yl)propanoyl)pyrrolidine-2-carboxylic acid (compound 6)

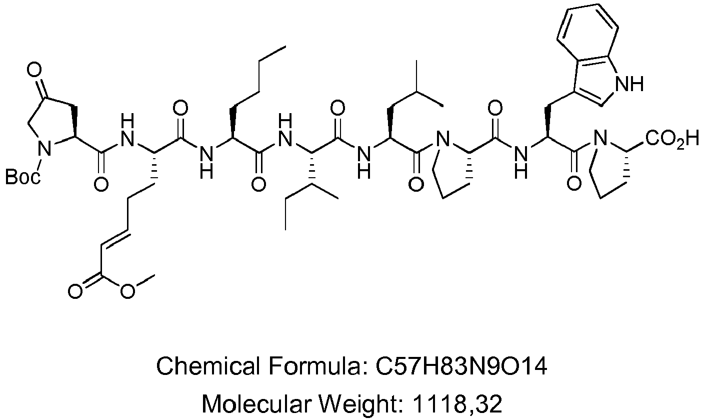

35.4 mg (0.15 mmol) N-Boc-4-oxo-L-proline, 57 mg (0.15 mmol) HATU and 51 µl (0.30 mmol) DIPEA were dissolved in 1.5 ml DMF. To this solution 153 mg (0.15 mmol) **5** in 1.5 ml DMF were added. The resulting solution was set to pH 8.0 (DIPEA) and stirred at room temperature overnight. The solvent was removed under reduced pressure and the residue was purified by preparative HPLC (gradient from 50%B to 90%B with 1 %/min, 220 nm).
Compound **6** was obtained as a white solid (100 mg, 89 µmol, 60%).

### Example 07: Preparation of (S)-1-((S)-2-((S)-1-((8S, 11S, 14S, 17S, E)-14-sec-butyl-11-butyl-17-isobutyl-3,9,12,15-tetraoxo-8-((S)-4-oxopyrrolidine-2-carboxamido)-2-oxa-10,13,16-triazaoctadec-4-ene)pyrrolidine-2-carboxamido)-3-(1H-indol-3-yl)propanoyl)pyrrolidine-2-carboxylic acid trifluoroacetate (compound 7)

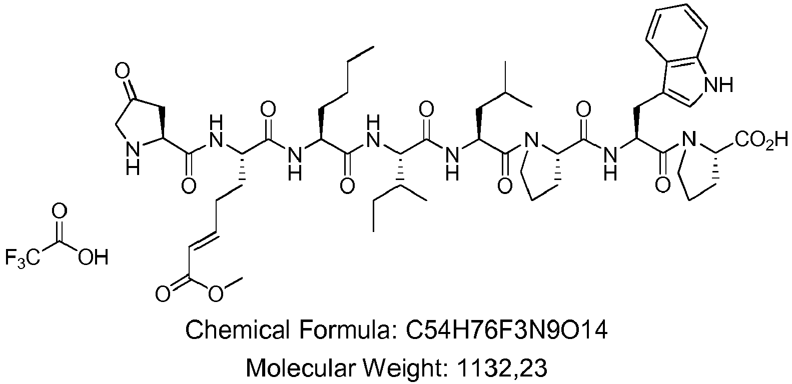

100 mg (89 µmol) **6** were dissolved in 5 ml dichloromethane. 5 ml TFA were added. The resulting solution was stirred for 3 h at room temperature before the solvent was removed under reduced pressure. The residue was purified by preparative HPLC (gradient from 30%B to 90%B with 1 %/min, 230 nm).
Compound **7** was obtained as a white solid (56 mg, 49 µmol, 56%).

ESI-MS: 1040.8 [M+Na]⁺

### BIOCHEMICAL EXPERIMENTS

### Example 08: Crystallization of blood coagulation Factor Xllla in the absence of thrombin

As used herein, the term "FXIII-A₂" refers to the dimer (dimeric form) of the not activated blood coagulation factor XIII. It is known to a skilled person that the dimeric form of blood coagulation factor XIII is the stable conformation under physiologic conditions.

### Protein production and purification

The coding sequence of blood coagulation factor XIII (A subunit) was amplified by PCR from cDNA clone MGC:34260 IMAGE:5222962 (Genebank BC027963) and cloned into the pFASTBac™ vector. Recombinant production of His₆-FXIII-A₂ was performed using the baculovirus system (Bac-to-Bac, Invitrogen) according to the manufacturer. Recombinant FXIII-A₂ was purified by immobilized metal affinity chromatography (IMAC) and subsequent anion exchange chromatography. The purified and concentrated His₆-FXIII-A₂ (7.4 mg/ml) was stored in 10 mM Tris-HCl buffer pH 8.0, 300 mM NaCl, 15% (v/v) glycerol.

### Factor XIIIA activation and inhibition

Activation of blood coagulation factor XIII was performed by high concentrations of calcium instead of thrombin. However activation by thrombin is feasible also and is claimed accordingly. A solution of purified His₆-FXIII-A₂ (0.2 mg/mL) in 10 mM Tris-HCl pH 8.0, 300 mM NaCl, 15% (v/v) glycerol containing 100 mM Ca²⁺ was mixed with the inhibitor 1 (compound 4) or 2 (compound 7) at a molar ratio of 1 : 25. The mixture was slightly stirred and incubated at room temperature for 1 h. The inhibited His₆-FXIIIa [inhibitor **1** or **2**] complex was concentrated using centrifugal concentrators (Vivaspin® 20, 30 kDa cut-off). This solution was applied to a Superdex75pg HiLoad column equilibrated with 10 mM Tris-HCl pH 8.0, 300 mM NaCl, 15% (v/v) glycerol containing 3 mM CaCl₂. The eluted protein was subsequently concentrated to 9.7 mg/mL using said centrifugal concentrators again. The inhibited blood coagulation factor XIIIa was stored at -80°C prior to crystallization.

### His₆-FXIIIa[inhibitor (I)] crystallization

Protein crystals were grown at room temperature using the hanging drop vapour diffusion method. Crystallization was achieved by mixing 2 µl protein solutions with 2 µl precipitation solution (170 mM ammonium sulphate, 85 mM sodium cacodylate pH 6.5, 25.5% (w/v) PEG 8,000, 15% (v/v) glycerol). Crystals appeared after one to two weeks.

### Example 09: Crystallization of blood coagulation Factor XIIIa in the presence of thrombin

### Protein production and purification

Recombinant FXIII-A₂ was purified by immobilized metal affinity chromatography (IMAC) and subsequent anion exchange chromatography. The purified His₆-FXIIIA₂ (30ml; 1.65 mg/ml) was stored in 50 mM Tris-HCl buffer pH 7.4, 300 mM NaCl, 15% (v/v) glycerol at 4°C until activation.

### Factor XIIIA activation by thrombin and subsequent inhibition

Activation of said 30ml of recombinant coagulation factor XIII was performed by thrombin. The His₆-FXIII-A₂ solution was diluted with 220 ml of 50 mM Tris-HCl buffer pH 7.4, 300 mM NaCl, 15% (v/v) glycerol, 3 mM Calciumchloride and 2.5 mM CHAPS.
Thrombin (1,250 NIH Units) in 1.25 ml of 50 mM Tris-HCl buffer pH 7.4, 300 mM NaCl, 15% (v/v) glycerol, 3 mM Calciumchloride and 2.5 mM CHAPS was added and the mixture was incubated at 25°C for 150 minutes.
Thrombin was inactivated by adding 10 µl PPACK (Phe-Pro-Arg-chloromethylketone) with a concentration of 1.33 mg/ml. The activated FXIIIa was subsequently inhibited adding Inhibitor (I) (10 mg in 200µl DMSO) and reaction of 45 minutes at 25 °C.

The FXIIIa[Inhibitor(I)] complex was concentrated using centrifugal concentrators (Vivaspin® 20, 30 kDa cut-off). This solution was applied to a Superdex75pg HiLoad column equilibrated with 10 mM Tris-HCl pH 8.0, 300 mM NaCl, 15% (v/v) glycerol containing 3 mM CaCl₂. The eluted protein was subsequently concentrated to 10.7 mg/mL using said centrifugal concentrators again. The inhibited blood coagulation factor XIIIa was stored at -80°C prior to crystallization.
Protein crystals were grown by above-mentioned method.in example 08.

### Example 10: Measurement of crystal

For X-ray diffraction measurement a single crystal of blood coagulation factor XIIIa complex was selected after removal of small satellites on the surface and frozen in liquid nitrogen. A dataset consisting of 400 images with 0.5 degree rotation and a wavelength of 0.91841 Å was collected at BL14.2 operated at the BESSY II electron storage ring and processed with a resolution to 1.98 Å in P1 symmetry using the program HKL2000. The structure was determined by molecular replacement with the CCP4 program Phaser. The molecular replacement model was divided into four subunits. The β-sandwich domain and the two β-barrel domains of the inactive form of factor XIII (PDB ID: 1 F13) were used. For the catalytic domain a homology model based on the active form of transglutaminase 2 (PDB ID: 2Q3Z) was generated with the program Modeller. The appropriate sequence alignment was performed by taking the secondary structure parts with PROMALS into account.

Subsequently a significant improvement of the obtained starting model was achieved by automated methods implemented in ARP/wARP. The model was manually further improved with Coot and refined with Phenix. At the beginning of the refinement a simulated annealing step was performed. The structure was refined with coordinates, individual B-factors, occupancies, TLS groups, optimized X-ray/stereochemistry weight and optimized X-ray/ADP weight. The ligand was minimized by using the Tripos Force Field implemented in Sybyl. The ligand restraints were created with Monomer Library Sketcher implemented in the CCP4 Program Suite.

**Table 1.**

| Data collection and refinement statistics | | | |
|---|---|---|---|
| Data Collection | | | |
| Wavelength (A) | | | 0.91841 |
| Space group | | | P1 |
| Resolution¹ (A) | | | 50.00 - 1.98 (2.03 - 1.98) |
| Unique reflections¹ | | | 119138 (7864) |
| Completeness (%)¹ | | | 97.1 (95.8) |
| Redundancy | | | 2.2 (2.1) |
| R_{merge} (%)¹ | | | 8.4 (36.3) |
| <I/σ>¹ | | | 10.0 (2.0) |
| Unit Cell (Å,°) | | | a = 56.8, b =80.5,c=102.8 α = 88.1, β = 76.7, γ = 82.0 |
| Matthews coefificient (Å³/Da) | | | 2.7 |
| Solvent content (%) | | | 54.8 |
| | | | |

| Refinement | | | |
|---|---|---|---|
| Rcrvst (%) | | | 16.9 |
| R_{free} (%) | | | 20.6 |
| Protein residues | | | |
| | Molecule 1 | | 695 |
| | Molecule 2 | | 698 |
| Ligand atoms | | | |
| | Molecule 1 | | 74 |
| | Molecule 2 | | 74 |
| Water molecules | | | 920 |
| Glycerol molecules | | | 9 |
| Sulfate molecules | | | 1 |
| Calcium atoms | | | 6 |
| Average B-factor (Å²) | | | |
| | Protein | | |
| | | Molecule 1 | 27.3 |
| | | Molecule 2 | 27.9 |
| | Ligand | | |
| | | Molecule 1 | 35.3 |
| | | Molecule 2 | 38.9 |
| | Water | | 29.7 |
| | Calcium | | 21.9 |
| | Glycerol | | 34.7 |
| | Sulfate | | 35.5 |
| R.m.s. deviations from ideality | | | |
| | Bond lengths (A) | | 0.010 |
| | Bond angles (°) | | 1.247 |
| Ramachandran Plot² (%) | | | |
| | Most favoured | | 90.0 (1107 residues) |
| | Allowed | | 9.8 (120 residues) |
| | Disallowed | | 0.2 (3 residues) |

| | | | |
|---|---|---|---|
| ¹ numbers in brackets refer to the highest resolution shell ² according to Procheck^{[1]} [1] R. A. Laskowski, M. W. Macarthur, D. S. Moss, J. M. Thornton, Procheck - a Program to Check the Stereochemical Quality of Protein Structures. J Appl Crystallogr 26, 283-291 (1993). | | | |

### Example 11: Fluorogenic assay F001

The fluorogenic assay kit F001 (Zedira) is ready to use. Briefly, recombinant factor XIII (Zedira, product number T027) or human blood derived factor XIII (T007) is reconstituted in water. The enzymatic activity is measured in a suitable buffer (e.g. TRIS buffer pH 7.5 containing calcium chloride, sodium chloride, poly ethylene glycol (PEG), glycine methyl ester, clot inhibitor peptide GPRP-amide) after activation by thrombin. Factor XIIIa cleaves a dark quenching molecule from the side chain of the modified peptide incorporating glycine methyl ester (so called iso-peptidase activity). Subsequently, the fluorescence of an N-terminal coupled dye increases and can be monitored on-line (excitation wavelength 313 nm; emission wavelength 418 nm). The inhibitor of interest can be added after the activation of Factor XIII by thrombin is completed. The IC₅₀ value represents the inhibitor concentration leading to a 50% inhibition of FXllla activity.

### Example 12: Use of blood factor XIIIa coordinates in drug design

Based on computational methods like docking, two options have been identified potentially improving ligands. One option would be the introduction of a methyl group of the indole moiety to enhance the hydrophobic surface of the ligand or increasing rigidity by introduction a ring system like pyridine.

### Example 13: Bradford Protein Assay

1. Prepare dye reagent by diluting 1 part Dye Reagent Concentrate with 4 parts deionized water (18.2 MΩ*cm).
   Filter through Whatman #1 filter (or equivalent) to remove particulates. This diluted reagent may be used for approximately 1 weeks when kept at room temperature.
2. Add 1.0 mL of diluted dye reagent to each tube. Use in all steps low bind test tubes (Eppendorf Protein LoBind® Tube Nr.: 0030 079.221).
3. Prepare five dilutions of a BSA protein standard. The linear range of the assay for BSA is 0.05 to 0.5 mg/mL.
4. Pipet 20 µL of each standard and sample solution into 1 mL of dye reagent and vortex. Protein solutions are normally assayed in duplicate. Use buffer or water as a control.
5. Incubate at room temperature for at least 15 minutes. Absorbance will increase over time; samples should incubate at room temperature for no more than 1 hour.
6. Measure absorbance at 595 nm.

SEQ ID NO: 1
## Claims

1. Crystal form of blood coagulation factor XIIIa characterized as having a space group of P1, two monomers of coagulation factor XIIIa in the asymmetric unit and unit cell dimension of a₁ = 56 ± 3 Å, b₁ = 80 ± 3 Å, c₁ = 103 ± 3 Å, α₁ = 88 ± 3°, β₁ = 77 ± 3° and γ₁ = 82 ± 3°; or
having a space group of P1, two monomers of coagulation factor XIIIa in the asymmetric unit and unit cell dimension of a₂ = 57 ± 3 Å, b₂ = 64 ± 3 Å, c₂ = 67 ± 3 Å, α₂ = 76 ± 3°, β₂ = 72 ± 3° and γ₂ = 83 ± 3°; or
having a space group of C2, two monomers of coagulation factor XIIIa in the asymmetric unit and unit cell dimension of a₃ = 90 ± 3 Å, b₃ = 65 ± 3 Å, c₃ = 70 ± 3 Å, α₃ = 90 ± 3°, β₃ = 96 ± 3° and γ₃ = 90 ± 3°.

2. Crystal form of blood coagulation factor XIIIa according to claim 1, **characterized by** the atomic structure coordinates of figure 6.

3. Crystal form of blood coagulation factor XIIIa according to claim 1 or 2 containing a catalytic center and/or a pocket neighboring the catalytic center, wherein the catalytic center contains the amino acids Trp279, Cys314, Trp370, His373, and Asp396; and the pocket neighboring the catalytic center contains the amino acids Tyr214, Gly215, Glu216, Asp219, Lys221, Arg223, Asn281, Tyr283, Pro288, Pro289, Gln313, Trp315, Asn371, and Tyr372.

4. Crystal form of blood coagulation factor XIIIa according to claim 3 further containing a hydrophobic pocket and a surface between the catalytic center and the hydrophobic pocket,
wherein the hydrophobic pocket contains the amino acids Ala341, Met350, Asp351, Ile352, Val360, Tyr365, Asp367, Ser368, Val369, Leu439, His459, and Ile460; and the surface between the catalytic center and the hydrophobic pocket contains the amino acids Phe339, His342, Ser368, Val369, Trp370, Asn371, and His459.

5. Method for crystallizing blood coagulation factor XIIIa comprising the steps:
(a) providing an aqueous solution containing not more than 0.04 mM of blood coagulation factor XIII; and
(b) mixing an activation agent and an inhibitor with the aqueous solution of step (a) so that a molar ratio of inhibitor to blood coagulation factor XIII from 10:1 to 50:1 is obtained; and
(c) concentrating the solution of step (b); and
(d) diluting the concentrated solution of step (c) with a buffered precipitant solution containing between 10% by weight and 20% by weight of at least one small organic amphiphilic molecule and/or between 50 mM and 200 mM of at least one inorganic salt and/or between 20% by weight and 30% by weight of a precipitating agent so that the final concentration of the inhibited activated blood coagulation factor XIIIa is in the range of 1.0 mg/mL to 10 mg/mL.

6. Method according to claim 5, wherein the aqueous solution of step (a) is a buffered aqueous solution of a pH in the range of 7.0 to 9.5.

7. Method according to claim 5, wherein the activation agent of step (b) is Ca²⁺ in a concentration of at least 50 mM or the activation agent of step (b) is a combination of Ca²⁺ in a concentration of at least 5 mM and thrombin in a concentration of at least 500 NIH Units/mL.

8. Method according to claim 5, wherein the precipitating agent of step (d) is PEG.

9. Crystal form of blood coagulation factor XIIIa obtainable by a method according to any one of the claims 5-8.

10. Use of the crystal form according to any one of the claims 1 - 4 and 9 for the determination of the three-dimensional structure of blood coagulation factor XIIIa and for the identification and/or design of inhibitors of blood coagulation factor XIIIa.

11. Method for designing a compound that interacts with or inhibits blood coagulation factor XIIIa, comprising the steps of
(a) generating a three-dimensional model of blood coagulation factor XIIIa using the structure coordinates as listed in figure 6; and
(b) employing said three-dimensional model to design a compound that should have the ability to interact with or inhibit blood coagulation factor XIIIa.

12. Method according to claim 11, further comprising the steps of
(c) obtaining the designed compound; and
(d) contacting the obtained designed compound with blood coagulation factor XIIIa in order to determine the inhibitory effect on blood coagulation factor XIIIa.

13. Method for identifying and/or optimizing a compound that inhibits blood coagulation factor XIIIa, comprising the steps of
(a) generating a three-dimensional model of blood coagulation factor XIIIa using the structure coordinates as listed in figure 6; and
(b) employing said three-dimensional model to identify or optimize a compound that should have the ability to inhibit blood coagulation factor XIIIa.

14. Method according to claim 13, further comprising the steps of
(c) obtaining the identified or optimized compound; and
(d) contacting the identified or optimized compound with blood coagulation factor XIIIa in order to determine the inhibitory effect on blood coagulation factor XIIIa.

15. Method according to claim 11 or 13, wherein the three-dimensional model of the catalytic center and/or of the pocket neighboring the catalytic center and/or of the hydrophobic pocket and/or of the surface between the catalytic center and the hydrophobic pocket of the blood coagulation factor XIIIa is generated by using the structure coordinates as listed in figure 6 and wherein said three-dimensional model is employed to design, identify or optimize a compound that should have the ability to interact with or bind to the catalytic center, the pocket neighboring the catalytic center, the hydrophobic pocket and/or respectiviely the surface between the catalytic center and the hydrophobic pocket.


SEQUENCE LISTING

<110> Zedira GmbH

<120> Crystal structure of blood coagulation factor XIIIa

<130> ZED-P03535EP

<160> 1

<170> PatentIn version 3.3

<210> 1
<211> 738
<212> PRT
<213> Homo sapiens

<400> 1

Met His His His His His His Ser Glu Thr Ser Arg Thr Ala Phe Gly
1               5                   10                  15

Gly Arg Arg Ala Val Pro Pro Asn Asn Ser Asn Ala Ala Glu Asp Asp
            20                  25                  30

Leu Pro Thr Val Glu Leu Gln Gly Val Val Pro Arg Gly Val Asn Leu
        35                  40                  45

Gln Glu Phe Leu Asn Val Thr Ser Val His Leu Phe Lys Glu Arg Trp
    50                  55                  60

Asp Thr Asn Lys Val Asp His His Thr Asp Lys Tyr Glu Asn Asn Lys
65                  70                  75                  80

Leu Ile Val Arg Arg Gly Gln Ser Phe Tyr Val Gln Ile Asp Phe Ser
                85                  90                  95

Arg Pro Tyr Asp Pro Arg Arg Asp Leu Phe Arg Val Glu Tyr Val Ile
            100                 105                 110

Gly Arg Tyr Pro Gln Glu Asn Lys Gly Thr Tyr Ile Pro Val Pro Ile
        115                 120                 125

Val Ser Glu Leu Gln Ser Gly Lys Trp Gly Ala Lys Ile Val Met Arg
    130                 135                 140

Glu Asp Arg Ser Val Arg Leu Ser Ile Gln Ser Ser Pro Lys Cys Ile
145                 150                 155                 160

Val Gly Lys Phe Arg Met Tyr Val Ala Val Trp Thr Pro Tyr Gly Val
                165                 170                 175

Leu Arg Thr Ser Arg Asn Pro Glu Thr Asp Thr Tyr Ile Leu Phe Asn
            180                 185                 190

Pro Trp Cys Glu Asp Asp Ala Val Tyr Leu Asp Asn Glu Lys Glu Arg
        195                 200                 205

Glu Glu Tyr Val Leu Asn Asp Ile Gly Val Ile Phe Tyr Gly Glu Val
    210                 215                 220

Asn Asp Ile Lys Thr Arg Ser Trp Ser Tyr Gly Gln Phe Glu Asp Gly
225                 230                 235                 240

Ile Leu Asp Thr Cys Leu Tyr Val Met Asp Arg Ala Gln Met Asp Leu
                245                 250                 255

Ser Gly Arg Gly Asn Pro Ile Lys Val Ser Arg Val Gly Ser Ala Met
            260                 265                 270

Val Asn Ala Lys Asp Asp Glu Gly Val Leu Val Gly Ser Trp Asp Asn
        275                 280                 285

Ile Tyr Ala Tyr Gly Val Pro Pro Ser Ala Trp Thr Gly Ser Val Asp
    290                 295                 300

Ile Leu Leu Glu Tyr Arg Ser Ser Glu Asn Pro Val Arg Tyr Gly Gln
305                 310                 315                 320

Cys Trp Val Phe Ala Gly Val Phe Asn Thr Phe Leu Arg Cys Leu Gly
                325                 330                 335

Ile Pro Ala Arg Ile Val Thr Asn Tyr Phe Ser Ala His Asp Asn Asp
            340                 345                 350

Ala Asn Leu Gln Met Asp Ile Phe Leu Glu Glu Asp Gly Asn Val Asn
        355                 360                 365

Ser Lys Leu Thr Lys Asp Ser Val Trp Asn Tyr His Cys Trp Asn Glu
    370                 375                 380

Ala Trp Met Thr Arg Pro Asp Leu Pro Val Gly Phe Gly Gly Trp Gln
385                 390                 395                 400

Ala Val Asp Ser Thr Pro Gln Glu Asn Ser Asp Gly Met Tyr Arg Cys
                405                 410                 415

Gly Pro Ala Ser Val Gln Ala Ile Lys His Gly His Val Cys Phe Gln
            420                 425                 430

Phe Asp Ala Pro Phe Val Phe Ala Glu Val Asn Ser Asp Leu Ile Tyr
        435                 440                 445

Ile Thr Ala Lys Lys Asp Gly Thr His Val Val Glu Asn Val Asp Ala
    450                 455                 460

Thr His Ile Gly Lys Leu Ile Val Thr Lys Gln Ile Gly Gly Asp Gly
465                 470                 475                 480

Met Met Asp Ile Thr Asp Thr Tyr Lys Phe Gln Glu Gly Gln Glu Glu
                485                 490                 495

Glu Arg Leu Ala Leu Glu Thr Ala Leu Met Tyr Gly Ala Lys Lys Pro
            500                 505                 510

Leu Asn Thr Glu Gly Val Met Lys Ser Arg Ser Asn Val Asp Met Asp
        515                 520                 525

Phe Glu Val Glu Asn Ala Val Leu Gly Lys Asp Phe Lys Leu Ser Ile
    530                 535                 540

Thr Phe Arg Asn Asn Ser His Asn Arg Tyr Thr Ile Thr Ala Tyr Leu
545                 550                 555                 560

Ser Ala Asn Ile Thr Phe Tyr Thr Gly Val Pro Lys Ala Glu Phe Lys
                565                 570                 575

Lys Glu Thr Phe Asp Val Thr Leu Glu Pro Leu Ser Phe Lys Lys Glu
            580                 585                 590

Ala Val Leu Ile Gln Ala Gly Glu Tyr Met Gly Gln Leu Leu Glu Gln
        595                 600                 605

Ala Ser Leu His Phe Phe Val Thr Ala Arg Ile Asn Glu Thr Arg Asp
    610                 615                 620

Val Leu Ala Lys Gln Lys Ser Thr Val Leu Thr Ile Pro Glu Ile Ile
625                 630                 635                 640

Ile Lys Val Arg Gly Thr Gln Val Val Gly Ser Asp Met Thr Val Ile
                645                 650                 655

Val Glu Phe Thr Asn Pro Leu Lys Glu Thr Leu Arg Asn Val Trp Val
            660                 665                 670

His Leu Asp Gly Pro Gly Val Thr Arg Pro Met Lys Lys Met Phe Arg
        675                 680                 685

Glu Ile Arg Pro Asn Ser Thr Val Gln Trp Glu Glu Val Cys Arg Pro
    690                 695                 700

Trp Val Ser Gly His Arg Lys Leu Ile Ala Ser Met Ser Ser Asp Ser
705                 710                 715                 720

Leu Arg His Val Tyr Gly Glu Leu Asp Val Gln Ile Gln Arg Arg Pro
                725                 730                 735

Ser Met